# EUROPEAN PATENT SPECIFICATION

(11) **EP 4 420 265 B1**
(45) Date of publication and mention of the grant of the patent: **02.12.2026**
(21) Application number: 22882115.3
(22) Date of filing: 21.10.2022
(51) Int. Cl.: H04B 10/071, A61B 5/07, A61B 5/145, A61B 5/1491, A61B 5/00, A61B 5/06, A61B 5/11

(54) **METHOD, PROGRAM, AND APPARATUS FOR DETERMINING LOCATION OF INGESTIBLE CAPSULE**
VERFAHREN, PROGRAMM UND VORRICHTUNG ZUR BESTIMMUNG DER POSITION EINER EINNEHMBAREN KAPSEL
PROCÉDÉ, PROGRAMME ET APPAREIL POUR DÉTERMINER L'EMPLACEMENT D'UNE CAPSULE INGÉRABLE

(30) Priority: 21.10.2021 AU 2021903378; 04.04.2022 AU 2022900873
(43) Date of publication of application: 28.08.2024
(73) Proprietor: Atmo Biosciences Limited, Box Hill, Victoria 3128 (AU)
(72) Inventor: JOHN, James, Box Hill, Victoria 3128 (AU); BEREAN, Kyle, Box Hill, Victoria 3128 (AU); HEBBLEWHITE, Malcolm, Box Hill, Victoria 3128 (AU); CHRIMES, Adam, Box Hill, Victoria 3128 (AU); ROHR, Eduardo Rath, Box Hill, Victoria 3128 (AU); WOOD, Spencer Terry, Shanklin Hampshire PO37 6NJ (GB)
(74) Representative: Kennedy, Richard E.
(86) International application number: PCT/AU2022/051270
(87) International publication number: WO 2023/064996

(56) References cited:
- WO-A1-2014/122655
- WO-A1-2018/032032
- WO-A1-2018/032032
- US-A1- 2008 058 597
- US-A1- 2009 318 783
- US-A1- 2018 279 908
- KOUROSH KALANTAR-ZADEH ET AL: "Ingestible Sensors", ACS SENSORS, vol. 2, no. 4, 14 March 2017 (2017-03-14), US, pages 468 - 483, XP055612460, ISSN: 2379-3694, DOI: 10.1021/acssensors.7b00045
- KOUROSH KALANTAR-ZADEH ET AL: "A human pilot trial of ingestible electronic capsules capable of sensing different gases in the gut", NATURE ELECTRONICS, vol. 1, no. 1, 1 January 2018 (2018-01-01), pages 79 - 87, XP055647114, DOI: 10.1038/s41928-017-0004-x
- KALANTAR-ZADEH, K. ET AL.: "A human pilot trial of ingestible electronic capsules capable of sensing different gases in the gut", NATURE ELECTRONICS, vol. 1, January 2018 (2018-01-01), pages 79 - 87, XP055647114, DOI: 10.1038/s41928-017-0004-x
- KOUROSH KALANTAR-ZADEH, NAM HA, JIAN ZHEN OU, KYLE J. BEREAN: "Ingestible Sensors", ACS SENSORS, AMERICAN CHEMICAL SOCIETY, US, vol. 2, no. 4, 28 April 2017 (2017-04-28), US, pages 468 - 483, XP055612460, ISSN: 2379-3694, DOI: 10.1021/acssensors.7b00045
- JIAN ZHEN OU, JEREMY J. COTTRELL, NAM HA, NARESH PILLAI, CHU K. YAO, KYLE J. BEREAN, STEPHANIE A. WARD, DANILLA GRANDO, JANE G. MU: "Potential of in vivo real-time gastric gas profiling: a pilot evaluation of heat-stress and modulating dietary cinnamon effect in an animal model", SCIENTIFIC REPORTS, vol. 6, no. 1, 1 December 2016 (2016-12-01), XP055467486, DOI: 10.1038/srep33387

## Description

### Technical Field

This invention relates to sensors useful in an ingestible sensor capsule for medical and health applications in the gastrointestinal (GI) tract of mammals including humans, and specifically relates to recording sensor readings from an ingestible capsule and determining a location within the GI tract at the time of the reading.

### Background

Gas sensor capsules such as that disclosed in EP3497437A1 house gas sensors within an ingestible capsule so that readings may be made from within the gastrointestinal (GI) tract of a mammal, from which readings concentrations of analyte gases may be determined.

A process for determining type and concentration of particular gases in a multigas mixture based on readings taken from within the GI tract by gas sensors on-board an ingestible capsule is disclosed in EP3619526A1.

There are many reports on the strong likelihood of the association of these gas constituents to different illnesses, conditions, etc, and to the association of gases with gut health and therefore overall wellbeing.

However, in order to associate the gas constituents to different illnesses, conditions, etc, it is desirable to know or to be able to accurately predict a location of the capsule within the GI tract at the instant a gas sensor reading is obtained. Certain gases or concentrations of gases that would be expected in one part of the GI tract may be an indicator of an illness or condition in another location within the GI tract.

Furthermore, gut health is increasingly identified as a contributor to overall health and wellness. Motility of an ingestible capsule (with or without associated gas constituent measurements) provides important information in the assessment of gut health. WO2018/032032A1 discloses a capsule adapted to be introduced into the digestive system and gastrointestinal (Gl) tract of a mammal which consists of a capsule shaped container consisting of a wall material capable of being bio compatible with the digestive system and being adapted to protect the electronic and sensor devices contained in the capsule. The capsule contains gas composition sensors operable at several temperature points for a short duration, a temperature sensor, a micro controller, a power source and a wireless transmission device. The capsule wall incorporates gas permeable membranes adjacent said gas sensors. The microprocessor is programmed to receive data signals from the sensors and convert the signals into gas composition and concentration data and temperature data suitable for transmission to an external computing device.

### Summary

The invention is set out in the claims, to which reference should now be made.

Furthermore, data gathered by on-capsule sensors for specific medical or more general gut health data gathering purposes is of enhanced informational value if associated with an indication of capsule location within the gut at the instance of data gathering.

Gut health is a major component of wellbeing of humans and other mammals. Gut health is very difficult to analyse owing to difficulty in taking readings from within the gut, or in accurately assessing gut health parameters based on readings from outside the gut.

Embodiments provide a mechanism to take sensor readings from within the GI tract and to determine a location of the sensors at the instance of said readings.

Embodiments provide a mechanism to obtain timing information for the passage of an article through the GI tract and through specific regions of the GI tract. Such timing information is in itself a useful artefact in gut health and gut-related medical applications.

Embodiments provide a mechanism to take and record readings from sensors on-board an ingestible capsule, and to process the readings to determine a location of the capsule within the GI tract at the time of the reading. Said processing may be performed on-board the capsule or at a remote apparatus receiving data (directly or indirectly) from the capsule.

Embodiments leverage sensor readings to determine capsule location within the GI tract. However, embodiments are not reliant upon translating said readings to measurements of constituent gas concentrations. Patterns and features in the sensor output itself (i.e. the raw readings) may be utilised as markers/indicators for determining which physical events have occurred (i.e. through which gut sections or junctions the capsule has passed). Therefore issues relating to accuracy of calibration and being able to derive accurate and precise measurements of gas concentrations may be avoided in the process of locating the capsule. Of course, it may be that gas concentrations are of interest as companion information, but the gas concentrations themselves are required in determination of motility information. Whilst for example, gastric emptying is definitely associated with a change in CO2 concentration, it is not necessary to know the actual CO2 concentration around the capsule to determine whether or not gastric emptying has occurred (at the instance of readings being analysed). One or more indicators in the sensor readings which may be caused by the change in CO2 or may be caused by other physical changes associated with gastric emptying are detected and combined to determine whether or not gastric emptying has occurred (at the instance of readings being analysed).

Optionally, the ingestible capsule includes an environmental sensor, and the readings include environmental sensor readings; and wherein either: determining the first transition event timing further comprises comparing one or more of the environmental temperature sensor readings at or around a timing of a detected gastric-duodenal transition indicator with a baseline environmental temperature value, and based on a result of the comparison, determining whether or not the timing of the detected gastric-duodenal transition indicator is associated with the first transition event timing; or detecting the gastric-duodenal transition indicator includes moderating the TCD gas sensor readings according to the respective contemporaneous environmental temperature sensor readings, and detecting the gastric-duodenal transition indicator in the moderated TCD gas sensor readings.

Optionally, the gastric-duodenal transition indicator is a spike, step change, or inflection, in the TCD gas sensor readings from the recorded readings, and wherein it is determined that the timing of the gastric-duodenal transition indicator is associated with the first transition event timing if the environmental temperature value was within a predefined threshold distance of a baseline environmental temperature value for a predetermined period preceding the timing of the gastric-duodenal transition indicator.

Optionally, the VOC gas sensor comprises a heating element and is configured to drive the heating element in pulses, and wherein the VOC gas sensor readings are each taken at the same point in the phase of a respective pulse of the heating element.

Optionally, the ingestible capsule further comprises a primary transceiver comprising an antenna, the method further comprises: transmitting, by the antenna, the readings of the ingestible capsule to a receiver apparatus external to the subject mammal configured to record the readings.

Optionally, the ingestible capsule further comprises a directional coupler in series with the antenna to form a reflectometer, and the readings of the ingestible capsule include readings of the reflectometer, the first subset including readings of the reflectometer.

Optionally, the ingestible capsule further comprises, at a primary transceiver, a directional coupler in series with the antenna to form a reflectometer.

Optionally, the ingestible capsule further comprises a diode detector and the diode detector forms a part of the reflectometer, the diode detector being configured to receive the reflected signal from the antenna via the directional coupler and to measure an amplitude of the reflected signal.

Optionally, the ingestible capsule further comprises a quadrature demodulator and the quadrature demodulator forms a part of the reflectometer, the quadrature demodulator being configured to receive the reflected signal from the antenna via the directional coupler and to extract phase information of the reflected signal relative to a carrier signal, the carrier signal being the carrier signal for transmitting data away from the ingestible capsule to the receiver apparatus.

Optionally, the ingestible capsule further comprises an antenna impedance control mechanism comprising a variable capacitor configured to vary impedance of the antenna, and a controller, wherein the reflectometer and the antenna impedance control mechanism form a closed loop or feedback loop, and wherein the controller is configured to receive the measurements of the amplitude of the reflected signal from the diode detector and to execute a control algorithm to use the amplitude measurements to generate an antenna impedance control signal setting a capacitance of the variable capacitor to vary impedance of the antenna to reduce amplitude of the reflected signal.

Optionally, the closed loop or feedback loop further comprises the quadrature demodulator, and wherein the phase information extracted by the quadrature demodulator is output to the controller, and wherein the controller is configured to use the amplitude information and the phase information to generate the antenna impedance control signal.

Optionally, the recorded readings of the gas sensor capsule comprise one or more antenna reflectance related readings from among:
readings of the amplitude of the reflected signal measured by the diode detector;
readings of the phase information of the reflected signal extracted by the quadrature demodulator; and
the antenna impedance control signal;
and wherein the first subset of recorded readings, from which the first transition event timing is determined, comprises the antenna reflectance related readings, and/or the second subset of recorded readings, form which the second transition event timing is determined, comprises the antenna reflectance related readings.

Optionally, the reflectometer further comprises a diode detector, t antenna reflectance related readings being measurements by the diode detector of an amplitude of reflected signals from the antenna.

Optionally, determining the first transition event timing further comprises: detecting a gastric-duodenal transition indicator in the antenna reflectance related readings from the first subset; and determining that the first transition event has occurred and a timing thereof based on the a gastric-duodenal junction indicator detected in the antenna reflectance related readings from the first subset.

Optionally, method further comprises: detecting, as a first a gastric-duodenal transition indicator, a gastric-duodenal transition indicator in the TCD gas sensor readings from the first subset; and detecting, as a second gastric-duodenal transition indicator, a gastric-duodenal transition indicator in the antenna reflectance related readings from the first subset within a predefined temporal range of the detected first gastric-duodenal transition indicator; and based on detecting the first and second gastric-duodenal transition indicators, determining that the first transition event has occurred and a timing thereof.

Optionally, the ingestible capsule further comprises an accelerometer, and the readings of the ingestible capsule include accelerometer readings, the first subset including accelerometer readings.

Optionally, determining the first transition event timing further comprises: detecting a gastric-duodenal transition indicator in the accelerometer readings from the first subset; and determining that the first transition event has occurred and a timing thereof based on the gastric-duodenal transition indicator detected in the accelerometer readings from the first subset.

Optionally, the first subset includes the antenna reflectance related readings, and determining the first transition event timing further comprises: detecting a gastric-duodenal transition indicator in the antenna reflectance related readings from the first subset; and determining that the first transition event has occurred and a timing thereof based on the gastric-duodenal transition indicator detected in the antenna reflectance related readings from the first subset.

Optionally, the method comprises detecting, as a first a gastric-duodenal transition indicator, a gastric-duodenal transition indicator in the TCD gas sensor readings from the first subset; and detecting, as a second gastric-duodenal transition indicator, a gastric-duodenal transition indicator in the antenna reflectance related readings from the first subset within a predefined temporal range of the detected first gastric-duodenal transition indicator; and based on detecting the first and second gastric-duodenal transition indicators, determining that the first transition event has occurred and a timing thereof.

Optionally, method further comprises: detecting, as a first gastric-duodenal junction indicator, a gastric-duodenal junction indicator in the TCD gas sensor readings from the first subset; and detecting, as a second gastric-duodenal transition indicator, a gastric-duodenal junction indicator in the accelerometer readings from the first subset within a predefined temporal range of the detected first gastric-duodenal transition indicator; and based on detecting the first and second gastric-duodenal transition indicators, determining that the first transition event has occurred and a timing thereof.

Optionally, the method includes: detecting, as a first gastric-duodenal transition indicator, a gastric-duodenal transition indicator in the TCD gas sensor readings from the first subset; and calculating a confidence score representing a likelihood that the detected gastric-duodenal transition indicator in the TCD gas sensor readings is caused by the ingestible capsule traversing the gastric-duodenal junction; comparing the calculated confidence score with a threshold, and if the confidence score meets the threshold, determining that the first transition event has occurred and a timing thereof based on a timing of the detected gastric-duodenal transition indicator, and if the confidence score does not meet the threshold, assigning the detected gastric-duodenal transition indicator from the TCD gas sensor readings as a first gastric-duodenal transition indicator, and detecting whether or not a second gastric-duodenal transition indicator is present in readings from the first subset other than the TCD gas sensor readings and within a predefined temporal range of the first gastric-duodenal transition indicator, and if the second gastric-duodenal transition indicator is detected, determining that the first transition event has occurred and a timing thereof based on a timing of the first gastric-duodenal transition indicator.

Optionally, detecting whether or not a second gastric-duodenal transition indicator is present in readings from the first subset other than the TCD gas sensor readings and within a predefined temporal range of the first gastric-duodenal transition indicator, comprises: first detecting whether or not a second gastric-duodenal transition indicator is present in the antenna reflectance related readings from the first subset within a predefined temporal range of the first gastric-duodenal transition indicator, and if the second gastric-duodenal transition indicator is detected in the antenna reflectance related readings from the first subset, determining that the first transition event has occurred and a timing thereof based on a timing of the first gastric-duodenal transition indicator; and if the second gastric-duodenal transition indicator is not present in the antenna reflectance related readings from the first subset, second detecting whether or not a second gastric-duodenal transition indicator is present in the accelerometer readings from the first subset within a predefined temporal range of the first gastric-duodenal transition indicator, and if the second gastric-duodenal transition indicator is detected in the accelerometer readings from the first subset within a predefined temporal range of the first gastric-duodenal transition indicator, determining that the first transition event has occurred and a timing thereof based on a timing of the first gastric-duodenal transition indicator, and if the second gastric-duodenal transition indicator is not detected in the accelerometer readings from the first subset within a predefined temporal range of the first gastric-duodenal transition indicator, issuing a notification to request a manual review and continuing to monitor the first subset for another instance of a first gastric-duodenal transition indicator.

Optionally, the accelerometer readings provide a reading of an orientation of the ingestible capsule relative to a frame of reference in fixed relation to a gravitational vector, and wherein detecting a gastric-duodenal transition indicator in the accelerometer readings comprises: recording an orientation of the ingestible capsule given by a first accelerometer reading as a reference orientation, and repetitively in respect of each successive accelerometer reading chronologically: determining whether the orientation of the ingestible capsule given by the respective accelerometer reading is more than a threshold angular displacement from the reference orientation, and if the threshold angular displacement is not met, progressing to the next accelerometer reading without changing the reference orientation, and if the threshold angular displacement is met, changing the reference orientation to align with the orientation of the ingestible capsule given by the respective accelerometer reading; the gastric-duodenal transition indicator in the accelerometer readings being an increase in the rate of change of the reference orientation.

Optionally, the accelerometer readings provide a reading of an orientation of the ingestible capsule relative to a frame of reference in fixed relation to a gravitational vector, and wherein detecting a gastric-duodenal transition indicator in the accelerometer readings comprises: for each of three orthogonal axes in fixed spatial relation to the ingestible capsule derivable from the reading of the orientation, repetitively in respect of each successive accelerometer reading chronologically: calculating, as a scalar value, a change in the orthogonal axis relative to the gravitational vector from the preceding accelerometer reading; applying a low pass filter to the calculated changes; recording the cumulative filtered calculated changes; the gastric-duodenal transition indicator in the accelerometer readings being a step change in the rate of increase of the cumulative filtered calculated changes.

Optionally, the ingestible capsule includes an environmental sensor, and the readings include readings of the environmental sensor, the environmental sensor being an environmental temperature sensor, an environmental relative humidity sensor, or an environmental temperature sensor and an environmental humidity sensor; the processing the recorded readings including determining an excretion event timing by detecting a change in the environmental sensor readings between an internal environmental condition of the subject mammal and an external environmental condition at a location of the subject mammal, the excretion event timing being a timing of excretion of the ingestible capsule by the subject mammal.

Optionally, the ingestible capsule includes an environmental sensor, and the readings include readings of the environmental sensor, the environmental sensor being an environmental temperature sensor, an environmental relative humidity sensor, or an environmental temperature sensor and an environmental humidity sensor; the processing the recorded readings including determining an ingestion event timing by detecting a change in the environmental sensor readings between an internal environmental condition of the subject mammal and an external environmental condition at a location of the subject mammal, the ingestion event timing being a timing of ingestion of the ingestible capsule by the subject mammal.

Embodiments include apparatus for determining a location of an ingestible capsule within a gastrointestinal, GI, tract of a subject mammal, comprising: an ingestible capsule, being ingestible by a subject mammal, the ingestible capsule comprising a housing, a power source, a TCD gas sensor, and a VOC gas sensor; a receiver apparatus, the receiver apparatus being configured to receive, at a location external to the subject mammal, readings from the ingestible capsule at a location internal to the subject mammal, and to record the readings of the ingestible capsule as a function of time including a period during which the ingestible capsule is within the gastrointestinal, GI, tract of the subject mammal, the readings including TCD gas sensor readings and VOC gas sensor readings; a processing apparatus, the processing apparatus being configured to process the recorded readings by a process including: determining a first transition event timing, the first transition event timing being a timing of a gastric-duodenal transition by the ingestible capsule, wherein determining the first transition event timing comprises detecting a gastric-duodenal transition indicator in a first subset of the recorded readings, the first subset including the recorded TCD gas sensor readings; and determining a second transition event timing, the second transition event timing being a timing of a transition across an ileocecal junction by the ingestible capsule, wherein determining the second transition event timing comprises detecting an ileocecal junction transition indicator in a second subset of the recorded readings, the second subset including the VOC gas sensor readings.

Optionally, the receiver apparatus and the processing apparatus is the same computing device. Alternatively, the receiver apparatus is a first device and the processing apparatus is a second device, the second device being a computing device distinct from the first device and configured to access the readings recorded by the first device. The processing apparatus may be referred to as a remote computer elsewhere in this disclosure. The receiver apparatus may be a smartphone running an application for one or more from among storing, processing, and transmitting; data received from the capsule 10. The receiver apparatus may be a dedicated device configured to one or more from among store, process, and transmit; data received from the capsule 10.

Optionally, the ingestible capsule further comprises a secondary transceiver, the secondary transceiver being operable in a listening phase of the ingestible capsule during which the primary transceiver, sensors, and on-board processor of ingestible capsule are powered down, the secondary transceiver being configured during the listening mode to receive an encoded activation control signal from an encoded activation control signal transmitting device, and to respond by ending the listening phase and initiating a live phase of the ingestible capsule during which the primary transceiver, sensors, and on-board processor and memory, are powered-on and the readings are being recorded and optionally transmitted to a receiver apparatus.

Optionally the secondary transceiver is an NFC transceiver and wherein the encoded activation control signal is an NFC signal.

Optionally the receiver apparatus is the encoded activation control signal transmitting device, and wherein the receiver apparatus is a smartphone or tablet computer running an application causing the smartphone or tablet computer to generate and transmit the encoded activation control signal and to receive, and optionally to process and transmit, the recorded readings transmitted away from the capsule by the primary transceiver.

Embodiments may include a computer program for determining a location of an ingestible capsule within a gastrointestinal, GI, tract of a subject mammal, the ingestible capsule being ingestible by a subject mammal and comprising a housing, a power source, a TCD gas sensor, and a VOC gas sensor; the computer program being executable by a computing apparatus comprising a processor and a memory, and upon execution to cause the computing apparatus to perform a process comprising: accessing recorded readings of the ingestible capsule as a function of time including a period during which the ingestible capsule is within the GI tract of the subject mammal, the readings including TCD gas sensor readings, and VOC gas sensor readings, processing the recorded readings, including: determining a first transition event timing, the first transition event timing being a timing of a gastric-duodenal transition by the ingestible capsule, wherein determining the first transition event timing comprises detecting a gastric-duodenal transition indicator in a first subset of the recorded readings, the first subset including the recorded TCD gas sensor readings; and determining a second transition event timing, the second transition event timing being a timing of a transition across an ileocecal junction by the ingestible capsule, wherein determining the second transition event timing comprises detecting an ileocecal junction indicator in a second subset of the recorded readings, the second subset including the VOC gas sensor readings.

Embodiments of another aspect include a method of determining a location of an ingestible capsule within a gastrointestinal, GI, tract of a subject mammal, the method comprising: providing the ingestible capsule to the subject mammal for ingestion, the ingestible capsule comprising a housing, a power source, a TCD gas sensor, and a VOC gas sensor; recording readings of the ingestible capsule as a function of time, the readings including TCD gas sensor readings and VOC gas sensor readings; processing the recorded readings, comprising: determining a first transition event timing, the first transition event timing being a timing of a gastric-duodenal transition by the ingestible capsule, wherein determining the first transition event timing comprises detecting a gastric-duodenal transition indicator in a first subset of the recorded readings, the first subset including the recorded TCD gas sensor readings. Such embodiments may also include one or more of the optional features set out above. Such embodiments may further comprises determining a second transition event timing, the second transition event timing being a timing of a transition across an ileocecal junction by the ingestible capsule, wherein determining the second transition event timing comprises detecting an ileocecal junction indicator in a second subset of the recorded readings, the second subset including the VOC gas sensor readings.

Embodiments of another aspect include a method of determining a location of an ingestible capsule within a gastrointestinal, GI, tract of a subject mammal, the method comprising: providing the ingestible capsule to the subject mammal for ingestion, the ingestible capsule comprising a housing, a power source, a TCD gas sensor, and a VOC gas sensor; recording readings of the ingestible capsule as a function of time, the readings including TCD gas sensor readings and VOC gas sensor readings; processing the recorded readings, comprising: determining a transition event timing, the transition event timing being a timing of a transition across an ileocecal junction by the ingestible capsule, wherein determining the transition event timing comprises detecting an ileocecal junction indicator in a subset of the recorded readings, the subset including the VOC gas sensor readings. Such embodiments may also include one or more of the optional features set out above. Such embodiments may further comprise determining a first transition event timing, the first transition event timing being a timing of a gastric-duodenal transition by the ingestible capsule, wherein determining the first transition event timing comprises detecting a gastric-duodenal transition indicator in a first subset of the recorded readings, the first subset including the recorded TCD gas sensor readings.

Embodiments may comprise an ingestible capsule, being ingestible by a subject mammal, the ingestible capsule comprising: a housing; a power source; a primary transceiver comprising an antenna; a processor and a memory, the processor and the memory being configured to record readings from one or more sensors of the ingestible capsule and to store the recorded readings for transmission by the primary transceiver to a receiver apparatus; and a directional coupler in series with the antenna to form a reflectometer; wherein, the one or more sensors includes the reflectometer.

Optionally the ingestible capsule further comprises a diode detector, the diode detector forming a part of the reflectometer, the diode detector being configured to receive a reflected signal from the antenna via the directional coupler and to measure an amplitude of the reflected signal, wherein the recorded readings include the measurements of the amplitude of the reflected signal.

Optionally the ingestible capsule further comprises a quadrature demodulator and the quadrature demodulator forms a part of the reflectometer, the quadrature demodulator being configured to receive the reflected signal from the antenna via the directional coupler and to extract phase information of the reflected signal relative to a carrier signal, the carrier signal being the carrier signal for transmitting data away from the ingestible capsule to the receiver apparatus.

Optionally the ingestible capsule further comprises an antenna impedance control mechanism comprising a variable capacitor configured to vary impedance of the antenna, and a controller, wherein the reflectometer and the antenna impedance control mechanism form a closed loop or feedback loop, and wherein the controller is configured to receive the measurements of the amplitude of the reflected signal from the diode detector and to execute a control algorithm to use the amplitude measurements to generate an antenna impedance control signal setting a capacitance of the variable capacitor to vary impedance of the antenna to reduce amplitude of the reflected signal.

Optionally, the closed loop or feedback loop further comprises the quadrature demodulator, and wherein the phase information extracted by the quadrature demodulator is output to the controller, and wherein the controller is configured to use the amplitude information and the phase information to generate the antenna impedance control signal.

Optionally the recorded readings of the gas sensor capsule comprise one or more antenna reflectance related readings from among: readings of the amplitude of the reflected signal measured by the diode detector; readings of the phase information of the reflected signal extracted by the quadrature demodulator; and the antenna impedance control signal.

Optionally, the processor is configured, based on a spike or step change in the antenna reflectance related readings, and/or based on a level of a physical quantity represented by the antenna reflectance related readings, to detect that the ingestible capsule is located in one of the stomach, the small intestine, and the large intestine, of the subject mammal.

Optionally the ingestible capsule further comprises one or more gas sensors, wherein the one or more sensors includes the one or more gas sensors.

Embodiments include a system comprising the ingestible capsule and a receiver apparatus configured to receive the recorded readings transmitted by the antenna of the primary receiver, and to process the recorded readings to identify a spike or step change in the antenna reflectance related readings, and/or based on a level of a physical quantity represented by particular antenna reflectance related readings, to detect that, at the timing of the recorded readings of the identified spike or step change or the particular recorded readings, the ingestible capsule is located in one of the stomach, the small intestine, and the large intestine, of the subject mammal.

### Brief Description of Drawings

Embodiments will be described with reference to the accompanying drawings, in which:
Figure 1A is a schematic of an ingestible capsule;
Figure 1B is a cross sectional view of the ingestible capsule;
Figure 1C is a schematic of a system;
Figure 2A illustrates a system during a live phase of an ingestible capsule;
Figure 2B illustrates a system comprising an ingestible capsule;
Figure 2C illustrates a receiver apparatus and a relay apparatus;
Figure 2D illustrates a user interface of a receiver apparatus;
Figure 3A is a schematic view of an electronics arrangement of an ingestible capsule;
Figure 3B is a schematic diagram of a primary transceiver;
Figure 4 is a flowchart of a method for determining a location of an ingestible capsule within a GI tract;
Figure 5 is a flowchart of a method for determining a location of an ingestible capsule within a GI tract;
Figure 6 is a plot of thermal conductivity against time and associated constituent gas concentrations;
Figure 7A is a plot of capsule readings against time;
Figure 7B is a plot of capsule readings against time;
Figure 7C is a plot of environmental temperature readings and environmental humidity readings against time;
Figures 8A to 8E are plots of sensor readings and determined gas concentrations against time;
Figures 9A to 9D are plots of recorded capsule readings against time;
Figure 10 is a flowchart of a method for determining a timing of a gastric-duodenal transition event;
Figure 11A illustrates the relationships between sensors, algorithms, and processing results;
Figure 11B shows an exemplary data visualisation marked with transit time metrics; and
Figures 12A to 12D illustrate processing algorithms.

### Description of Embodiments

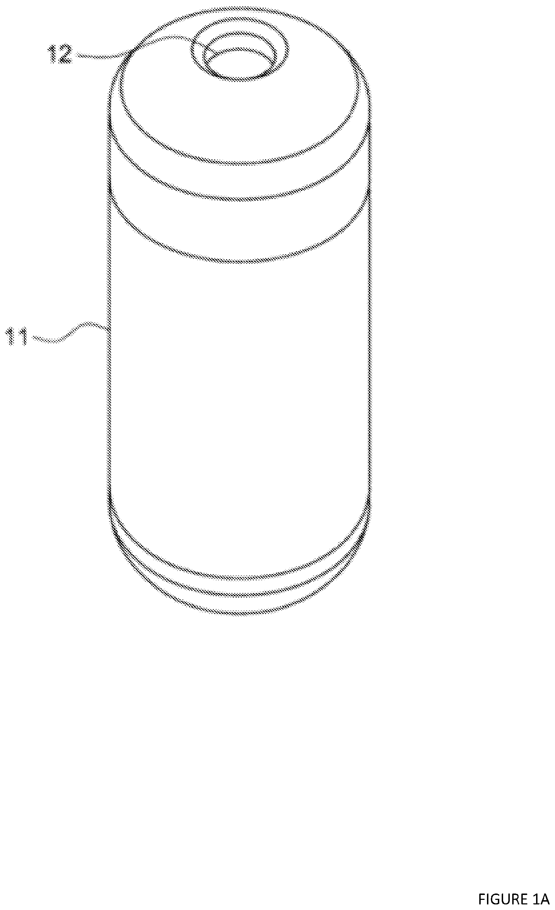

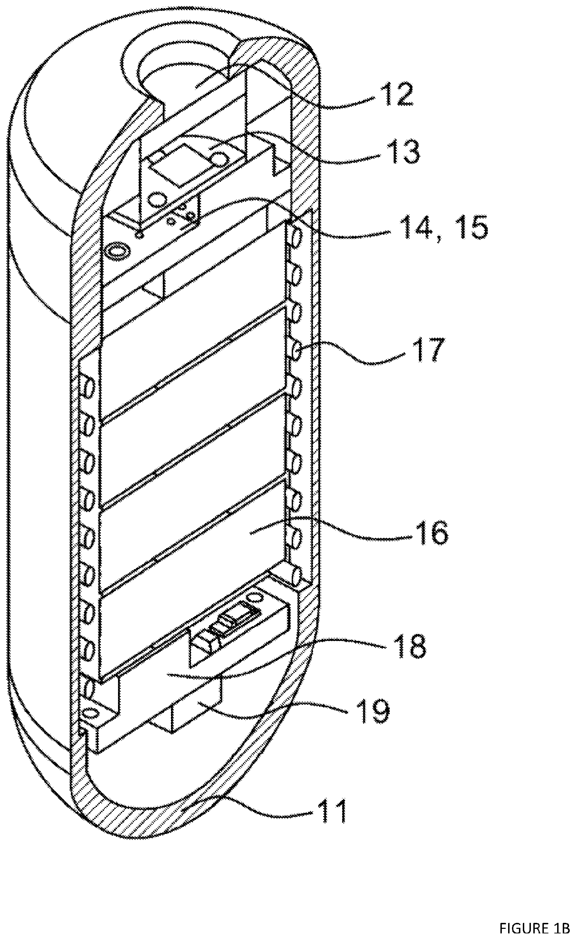

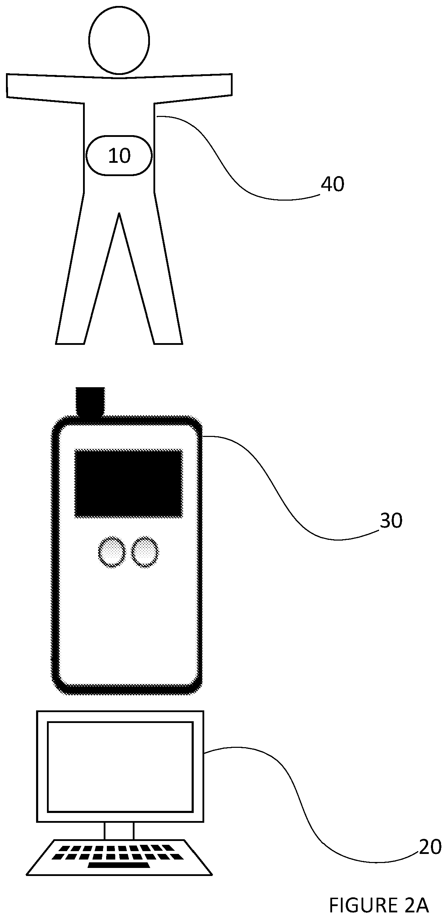

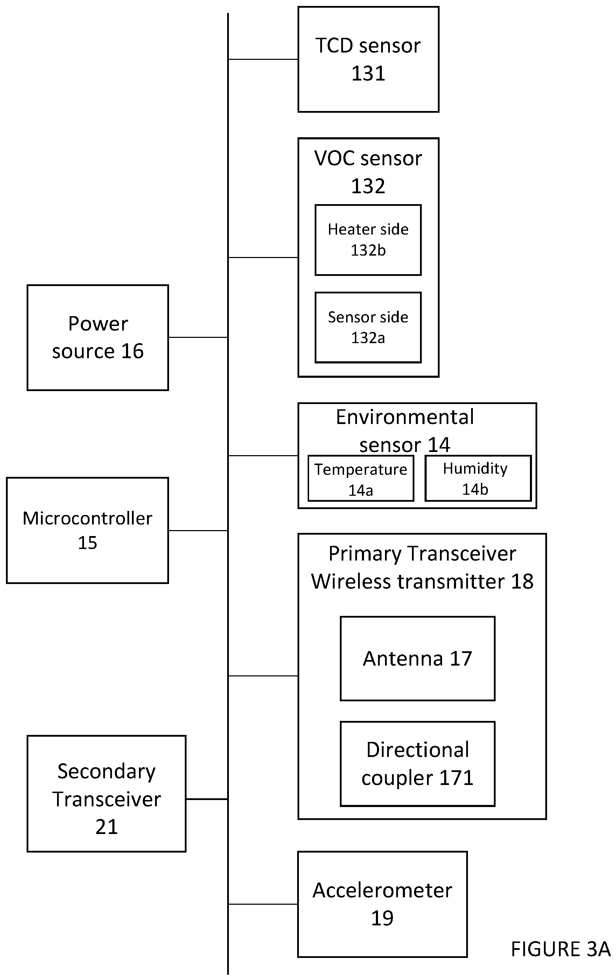

Figures 1A and 1B illustrate an ingestible capsule 10. A system including the ingestible capsule 10 of Figures 1A and 1B is illustrated in Figure 2A, during a live phase of the ingestible capsule 10 (i.e. while the ingestible capsule 10 is obtaining readings from within the GI tract of a subject mammal 40). A schematic of an exemplary arrangement of the electronic components within an ingestible capsule 10 such as that illustrated in Figures 1A and 1B is shown in Figure 3A.

As shown in Figures 1 A and B the capsule 10 consists of a housing such as a gas impermeable shell 11 which has an opening covered by a gas permeable membrane 12.

### System Architecture

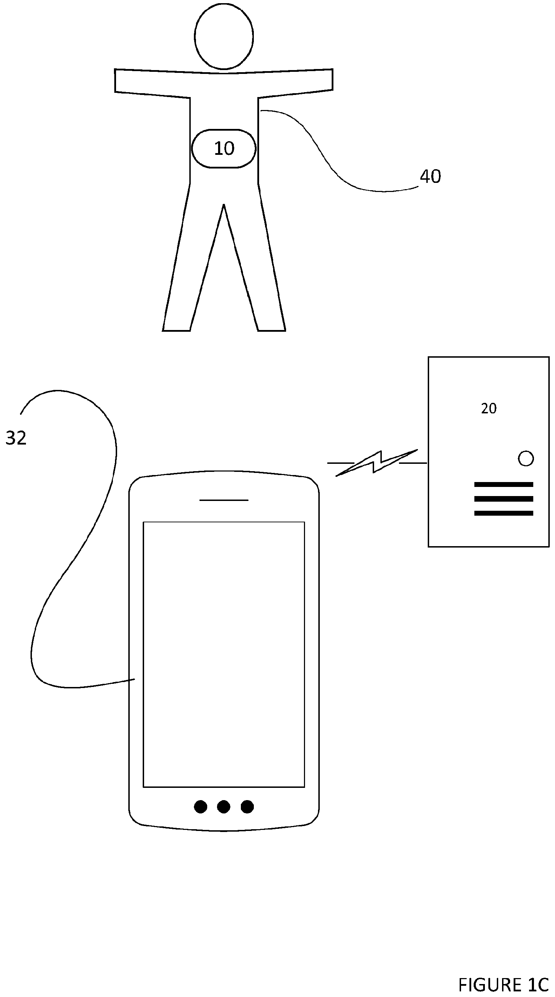

As shown in Figure 1C, a system, in addition to the capsule 10, further comprises a receiver apparatus 30 which receives data transmitted by the capsule from within the GI tract of the subject mammal during the live phase. Concurrently or subsequently, the receiver apparatus 30 processes the received data and may also upload some or all of the received data to a remote processing apparatus such as a cloud-based service for further processing. The remote computer may be a cloud resource, or may be a standalone computer at a clinician premise at which the subject is a patient, or may be a server (be it cloud-based or otherwise) at a service provider to which the clinician is a subscriber/customer/servicer user.

The receiver apparatus 30 may be a dedicated device (designed for storing and optionally processing data received from the capsule 10) or may be a general purpose device such as a smartphone. The smartphone may be running an app (downloaded thereto in advance of capsule ingestion) for storing and optionally processing data received from the capsule 10. In particular, the capsule 10 may comprise a Bluetooth transceiver and the receiver apparatus 30 may be a Bluetooth-enabled tablet, smartphone, or personal computer.

Optionally, a system may further comprise a remote processing apparatus 20 such as a server forming part of a cloud computing environment or some other distributed processing environment. The remote processing apparatus 20 may be a server provided by or on behalf of a clinical centre at which subject 40 is a patient and taking responsibility for interpreting the results generated by the capsule 10 (i.e. the data transmission payload) and reporting them to the subject 40.

### Capsule Components

By way of example, the capsule illustrated in Figure 1C houses, as sensor hardware, an environmental sensor 14 in the form of a temperature sensor 14a and/or a humidity sensor 14b, gas sensors in the form of a TCD gas sensor 131 and a VOC gas sensor 132, an accelerometer 19, and a reflectometer. Embodiments may include any single or combination of those individual sensors. Alternatively or additionally, embodiments may include one or more sensors not illustrated in Figure 1C such as a spectrophotometer, Surface Acoustic Wave sensor, and/or Bulk Acoustic Resonator Arrays. The reflectometer includes the primary transceiver and associated circuitry.

### Communication between capsule and receiver apparatus

During a live phase of the capsule connectivity between the capsule 10 and the receiver apparatus 30 is via the data transmitter on the capsule, which may be part of a wireless transceiver, for example a Bluetooth transceiver, which may operate according to a standard Bluetooth transmission protocol or according to Bluetooth Low Energy transmission protocol. Other operable communication technologies include LoRa, wifi and 433 MHz radio.

Optionally, there may be plural wireless transceivers on the capsule 10, such as a Bluetooth, or primary, transceiver and an NFC, or secondary, transceiver. A single integrated chip may provide both, so that certain of the circuitry need not be duplicated. However, the single integrated chip includes two separate antennae, one for Bluetooth communication (for example at 2.4Ghz) and one for NFC communication (for example at 13.6MHz). The capsule 10 may comprise two separate wireless communications mechanisms, each being configured to at least one of send and receive data, from a smartphone or tablet within communication range. The wireless communication mechanisms may be a primary, or Bluetooth, wireless communication mechanism and a secondary, or NFC, wireless communication mechanism.

For example, the smartphone or tablet may be running an application managing data communication with the capsule 10 and in particular configured to at least one of store, process, and transmit data from the capsule 10. The same application may facilitate communication with the communication mechanisms on the capsule 10, for example, by encoding one or more signals transmitted from the smartphone or tablet with a code which the capsule 10 has been preconfigured to accept as a key with which to unlock functionality. That is, the capsule 10 has been preconfigured only to respond to received signals encoded with the code. The code is unique to the specific capsule instance.

### Primary Transceiver

Where capsules 10 comprise two wireless transceivers, a first may be configured as a primary transceiver, which may use a Bluetooth, or a 433 MHz radio, communication protocol. The primary transceiver is configured, during a live phase of the capsule 10, to transmit the data transmission payload to the receiver apparatus 30. The data transmission payload is discussed elsewhere, but may comprise readings, metrics representing readings, timings or reports of motility event indicators, and/or reports of determinations that motility events have occurred and timings thereof. The primary transceiver is active during passage of the capsule 10 through the GI tract of the subject mammal 40 and therefore is configured to transmit signals comprising the data transmission payload from inside the GI tract of the subject mammal 40 to a receiver apparatus 30 external to the subject mammal. Thus, where the present disclosure makes reference to the data transceiver or the data transmitter, or to the transmitter, it is assumed, unless specifically referenced as the secondary or NFC wireless transceiver, that it is the primary wireless transceiver (i.e. the Bluetooth or 433MHz transceiver) being discussed.

### Secondary Transceiver

A second wireless transceiver is a secondary wireless transceiver, which may use an NFC communication protocol. The secondary transceiver is for specific activation control signalling only, such as for initiating an active mode of the capsule 10 at an unpackaging stage or otherwise prior to ingestion of the capsule 10. The secondary transceiver is not active during the live phase of the capsule 10, i.e. during passage through the GI tract of the subject mammal. The secondary transceiver does not contribute to transmission of the data transmission payload from the capsule 10 to the receiver apparatus 30. The secondary transceiver may not be required to perform any transmission whatsoever, that is, the secondary transceiver may only be required to receive an activation control signal from a smartphone or tablet running an application. However, the NFC protocol may require two-way exchange of signals such as a handshake or coupling process to enable said activation control signal to be transmitted from the smartphone or tablet and received by the capsule 10. Nonetheless, since the secondary transceiver is inactive while the capsule 10 passes through the GI tract of the subject mammal, unlike in the case of the primary transceiver, there is no requirement that the secondary transceiver be configured to transmit signals from inside the GI tract of the subject mammal 40 to a receiver apparatus 30 external to the subject mammal.

Optionally, the secondary transceiver may be configured to receive an encoded activation control signal from a smartphone or tablet running an application configured for managing interactions between the smartphone or tablet (tablet in this context meaning tablet computer) and the capsule 10, which encoded activation control signal initiates a live phase of the capsule 10 during which capsule sensors take readings and the readings themselves or metrics and/or reports based on the readings are transmitted from the capsule 10 to the smartphone or table via the primary wireless data transceiver. Thus, the secondary wireless data transceiver is active in a listening phase which precedes a live phase of the capsule. The primary wireless data transceiver is inactive (i.e. consuming no power whatsoever) during the listening phase. Once the encoded activation control signal is received (and the capsule 10 powered on in response) the listening phase ends and the secondary wireless data transceiver becomes inactive. The primary wireless data transceiver is active during the live phase.

In order to conserve battery power, capsule 10 operates in a standby or listening mode during the time between release from manufacturing and initiation of the live phase during which readings are recorded by the on-board sensors and transmitted away from the capsule. The standby or listening mode is an extremely low power mode. A live phase of the capsule is initiated prior to ingestion by the subject mammal. A mechanism for ending the standby or listening mode and entering a live phase include a reed switch coupled to a magnet on the packaging which is triggered by release of the capsule from the packaging and when triggered powers on the processor, sensors, and primary transceiver (i.e. initiates the live phase). An alternative mechanism is based on Near Field Communication, NFC. In the alternative mechanism, the capsule 10 is maintained in the standby or listening mode (which in the particular example of the NFC is a SENSE mode) prior to being issued to the subject. In the listening mode, when an electromagnetic field is detected with an appropriately encoded activation control message, the on-board microcontroller (i.e. processor) enters a live phase. A tablet computer or mobile phone running an application configured for the purpose of managing interactions with the capsule 10 and the processing of data received therefrom, and having NFC capability, can generate the appropriately encoded activation control message. In particular, a back-end server may link a user account to a particular capsule instance, so that when that user is logged in to the application and selects to activate a capsule, the application performs a lookup to the back-end server to determine how to encode the activation control message. In other words, optionally the encoding is unique per capsule. Alternatively, the encoding may be uniform across a batch of capsules or all capsules.

The NFC transceiver may be on the same integrated chip as the primary transceiver. The NFC transceiver may be positioned at an end of the capsule and close to the housing, to facilitate communication with the tablet computer or mobile phone.

### Further example system architectures

As shown in Figure 2A, the system, in addition to the capsule, further comprises a receiver apparatus 30 which receives data transmitted by the capsule from within the GI tract of the subject mammal during the live phase. Concurrently or subsequently, the receiver apparatus 30 uploads the recorded readings to a remote computer 20 for processing. The remote computer may be a cloud resource, or may be a standalone computer at a clinician premise at which the subject is a patient, or may be a server (be it cloud-based or otherwise) at a service provider to which the clinician is a subscriber/customer/servicer user.

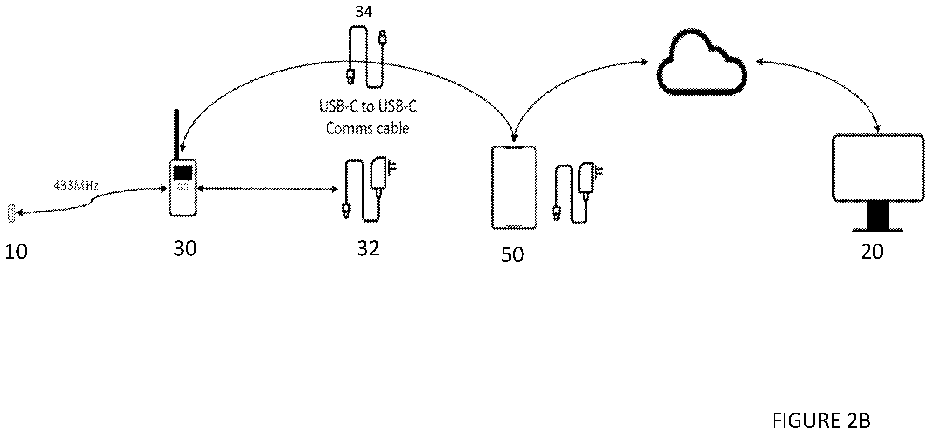

Figure 2B illustrates a further example of a system. The system comprises the ingestible capsule 10, a receiver apparatus 30, a charger 32 for the receiver apparatus, a comms cable 34 for connecting the receiver apparatus to a relay apparatus 50, and a remote computer 20 accessible via the cloud. The ingestible capsule 10 is provided in a sealed package to a patient. For example, during a live phase the ingestible capsule transmits sensor readings to a receiver apparatus 30 via a 433MHz transceiver. The receiver apparatus 30 is mounted on a belt to be worn by the patient and also comprises a 433MHz transceiver. The receiver apparatus 30 is rechargeable via the charger 32. A user interface comprises buttons and an LED display. The receiver apparatus 30 is connectable to a relay apparatus 50 via, for example, a USB-C connection. Firmware and midware on the receiver apparatus 30 is updateable via the relay apparatus 50. The relay apparatus 50 may be a tablet, loaded with an application, and also being rechargeable. The relay apparatus 50 is kept by a clinician and thereby is configured to associate data uploaded from the receiver apparatus to a patient ID before the data is sent for processing by the remote computer 20. Once the live phase of the capsule has been terminated, the receiver apparatus is returned to the clinician by the patient, and the readings transmitted by the capsule 10 during the live phase are uploaded to the relay apparatus 50 for association with patient data and forwarding to the remote computer 20 for processing. The remote computer 20 analyses the received data to detect the indicators and determine the timings of the motility events including gastric-duodenal transition, ileocecal junction transition, ingestion, and excretion. An external magnet clip may keep the capsule in a powered off state during storage and transportation (magnetically coupled to a reed switch in the capsule 10 prior to unpackaging). Alternatively, the capsule 10 may be in a very low power mode in which a secondary, NFC, transceiver is in a listening mode and is configured to receive an encoded NFC control signal from an NFC-enabled receiver apparatus 30 such as a smartphone or tablet running an application for managing data communication with the capsule 10, which signal once received powers-on the capsule 10 and initiates a live phase thereof.

As illustrated in Figure 1C, a Bluetooth enabled capsule may communicate directly with a smartphone or tablet running an application for managing data communication with the capsule 10 via which the smartphone is operable as a receiver apparatus as disclosed herein. As discussed elsewhere in the present disclosure, the capsule 10 may further comprise an NFC communication mechanism for communication with a smartphone or tablet as a secondary transceiver.

### Pairing or Coupling

The primary wireless data transmitter may be a Bluetooth transmitter, a wifi transmitter, a radio transmitter, or another form of wireless data transmitter. A primary radio transmitter may be configured to transmit in the 433 MHz band. In any case, the primary wireless data transmitter may be provided as part of a primary wireless data transceiver. For example, the primary wireless data transceiver may receive signals at least in performing pairing or any other form of coupling to a recipient device 30. The capsule 10 may be configured to enter into a wireless pairing or coupling mode immediately upon initiation of the live phase (i.e. first full power-on), wherein a subject or another user is instructed (via written instructions or via an application running on the recipient device itself) to pair or couple the capsule 10 and specifically the primary wireless transceiver thereof to the recipient device 30 prior to ingestion of the capsule 10. However, embodiments may be configured such that pairing or coupling is not necessary, for example the capsule 10 may be configured to broadcast data to a recipient device in a data transmission technique that is agnostic to pairing or coupling status. Pairing or coupling establishes a data communication connection or pathway for transmission of the data transmission payload from the capsule 10 to a receiver apparatus 30 by the primary data transceiver.

Data transmission payload is data to be transmitted away from the capsule 10 to the receiver apparatus 30, either in pre-excretion transmission routine, post-excretion transmission routine, or both. Data transmission payload may comprise one or more from among: raw readings from the sensors or quasi-sensors on board the capsule 10; a metric or metrics calculated by on-board processing of said raw readings; a motility event indicator identified by on-board processing of said raw readings.

### Primary Transceiver: Post-Excretion Data Transmission Routine

There are two principal data transmission routines, which ingestible capsules may be configured to use either or both of, depending on implementation details (i.e. use case). In a post-excretion data transmission model, signals from the sensors are received at the processor hardware 151 (utilising also the storage capabilities of the memory hardware 152) and processed on-board the capsule 10 in order to identify and record motility indicators (and optionally also other characteristics of the sensor output or sensor readings of interest or groups of sensor readings of interest) and the recorded motility indicators (and optionally also the other characteristics, metrics, and readings or groups of readings of interest, such as peak H2, area under a plot of H2 against time) are stored on the memory hardware 152 as a data transmission payload. Other characteristics and readings or groups of readings of interest may include, for example, maximum or minimum readings from specific sensors or from metrics calculated by combining sensors. The maximum or minimum readings may be local maximum or local minimum readings, wherein local is defined by, for example, predefined timings or motility events determined to have occurred by the capsule 10 itself. A specific example is maximum or minimum H2 concentration, which is a metric calculated from the gas sensor readings by an appropriately calibrated processor hardware.

In the post-excretion data transmission routine, the data transmission payload is transmitted by the wireless transceiver once excretion of the capsule 10 from the GI tract is detected (for example by the temperature sensor 14a signal and/or by the accelerometer 19 signal). Metrics further include peak H2 level or value, timing of peak H2, and total H2 (area under the curve). Such metrics may be calculated by the on-board processor hardware 151 during passage through the GI tract of the subject, and transmitted away from the capsule 10 to a receiver device in post-excretion transmission as part of a report or otherwise.

In the post-excretion data transmission routine, the transmission may be via a Bluetooth transmission mode that is not dependent upon pairing status. That is, for example, if the Bluetooth transceiver is paired to a receiver apparatus 30 then it transmits the data transmission payload to the paired receiver device, and if the Bluetooth transceiver is unpaired then it broadcasts the data transmission payload to a recipient device 30 in the absence of pairing in an inquiry mode (which may be referred to as discovery mode or beacon mode). Bluetooth protocol has an inquiry mode in which a device broadcasts a unique identifier, name and other information. The data transmission payload, or part thereof, may comprise or be included in the said other information. In particular, the data transmission payload may be prioritised or otherwise filtered by the processor hardware 151 so that information deemed particular important such as an indication that excretion has occurred (it is important for clinical reasons to know that the capsule 10 has been excreted) and potentially information such as timing of determined motility events, is transferred away from the capsule 10 in preference to other information.

Following the inquiry mode transmission, the transceiver may again attempt to pair, connect, or otherwise couple, with the recipient device, and if successful, to transmit the remainder of the data transmission payload. Of course, said pairing, connecting, or coupling, may have been performed initially pre-ingestion so that post-excretion the Bluetooth transceiver is attempting to re-pair, re-connect, or re-couple, with the receiver device 30. It is noted that the present discussion uses Bluetooth as an example of a transmission protocol, but that the same techniques could be applied to different transmission protocols.

In the event that there is data transmission payload pending transmission away from the capsule 10 after the broadcast of the unique identifier, name, and other information during the Bluetooth inquiry mode, then capsule 10 may be configured to initiate or reinitiate a data communication connection (i.e. a pairing or re-pairing) with a receiver device 30. Upon successful initiation or re-initiation of the communication connection, transmission of the said data transmission payload pending transmission away from the capsule 10 is performed whilst the data communication connection remains active.

The Bluetooth, primary, transceiver 18, or any other primary wireless data transceiver 18, may be configured to automatically re-connect following an initial (i.e. pre-ingestion) connection to a receiver device 30. The receiver device 30 may run an app or web app to guide the subject in terms of how to ingest the capsule 10, to notify the subject that the excretion event has been determined, and optionally also that the data transmission payload has been successfully transmitted to the receiver device 30 and so the capsule 10 may be flushed away. It is noted that the terms pair, connect, and couple, are interchangeable in the present document, each representing the establishment of a wireless connection between two devices for wireless data transfer.

It is noted that data transmission payload may be being transmitted throughout passage of the capsule 10 through the GI tract, dependent upon pairing, coupling, or connection to the receiver device 30. However, confirmation that occurrence of an excretion event has been determined by the capsule is information that is of particular importance since safety of capsule 10 is reliant on the capsule 10 being excreted. Therefore, information representing determination of occurrence of the excretion event (i.e. a report thereof) is prioritised and may be transmitted in a broadcast or inquiry mode, whereas the remaining data transmission payload is transmitted once connection between the wireless data transmitter 18 and the receiver device 30 is established.

In Bluetooth inquiry mode, data can be transmitted to the receiver apparatus 30, or to any Bluetooth receiver apparatus within range of the capsule 10, without pairing. The primary transceiver 18 is operable in a Bluetooth inquiry mode or a Bluetooth low energy mode. Capsules 10 may store and transmit among the data transmission payload readings from one or more sensors representing a predefined period either side of the identified motility indicators. For example, gas sensor signals only, or for all sensors. Such readings may be used to add confidence to the identified motility indicators in terms of determining whether or not a motility event has occurred, and/or may provide other information useful in a health or clinical context.

More generally, data transmitted according to the post-excretion data transmission routine may be any of the data transmission payload that has not already been transmitted. For example, the primary transceiver 18 may be configured to transmit the data transmission payload to a paired receiver apparatus while still in the GI tract (this element of the transmission is referred to herein as pre-excretion data transmission routine). However, owing to issues such as signal attenuation, noise, power supply issues, temporary pairing failure, or if pairing was never performed in the first place, or for any other reason, some or all of the data transmission payload may be pending transmission at the point of excretion. In that case, the remaining data transmission payload is transmitted according to the post-excretion data transmission routine once excretion is detected. It is noted that down-sampling of the data transmission payload may be performed prior to transmission via the post-excretion data transmission routine. Furthermore it is noted that some elements of the data transmission payload may be prevented from transmission via the post-excretion data transmission routine. For example, since bandwidth, and also time within which to transmit, may be limited, it may be that the motility event indicators and diagnostic indicators themselves are included, but that sensor readings are excluded from the data to be transmitted according to the post-excretion data transmission routine.

### Primary Transceiver: Post-Excretion Data Transmission Routine

In a pre-excretion data transmission technique, the sensor signals are transmitted continuously (errors, faults, and other unintentional interruptions notwithstanding) by the primary transceiver 18. In the pre-excretion data transmission routine, the process hardware 151 coordinates the receipt of the signals from the sensors and the storage at the memory hardware 152 for transmission by the wireless transceiver 18.

In the example of a Bluetooth primary transceiver 18, in the pre-excretion transmission routine the transceiver may be operated according to a long-range or codedphy Bluetooth transmission procedure, such as BTLE Coded PHY. A signal power enhancement of around 10dB is achievable via BTLE Coded PHY Bluetooth transmission procedure.

During a data transmission phase of the ingestible capsule 10 (i.e. which in the post-excretion data transmission routine is in a short burst post-excretion and in the pre-excretion data transmission routine is continuous while the ingestible capsule 10 is in use, that is, in the GI tract of a subject mammal 40 and obtaining and transmitting readings) the wireless transmitter 18 transmits the readings to a receiver apparatus 30, which may be a dedicated device for receiving and storing the readings (and optionally with a user interface) or may be a multi-function device such as a mobile phone (such as a smart phone). The mobile phone may be running an application which processes some or all of the data transmission payload to generate a motility report or diagnosis of a medical condition based on motility indicators and diagnostic indicators either included in the data transmission payload or derivable therefrom. Alternatively, the application may be configured to transmit the data transmission payload on to a server or another processing apparatus to generate the motility report or diagnosis based on the data transmission payload. The subject mammal need not remain within a specific range of the remote computer 20 during the live phase. Capsules 10 equipped with a Bluetooth transceiver 18 may communicate directly with a smartphone of a user, which obviates any need for a dedicated receiver apparatus (the smartphone taking on the role of receiver apparatus 30). The receiver apparatus 30 (whether a dedicated device or a mobile phone or tablet computer) may process the readings itself or may upload the readings to a remote computer 20 for processing (i.e. identifying motility indicators, determining motility event timings, resolving gas analytes). The upload may be continuous during a live phase of the capsule, or the upload may be after the live phase of the capsule is terminated. The receiver apparatus 30 may also store the readings, so that loss of connectivity between the receiver apparatus 30 and a remote processing apparatus is not critical.

### Pre-Processing

The on-board processor 151 may apply one or more processing or pre-processing steps, as discussed in more detail below. Digitisation of the readings is performed either by the sensors themselves, by the processor 151 or by the wireless transceiver 18. The digitised readings are transmitted via the antenna 17. The readings of the capsule 10 are made at an instant in time and are associated with the instant in time at which they are made. For example, a time stamp may be associated with the readings by the microcontroller 15, the wireless transmitter 18, or at the receiver apparatus 30 or remote computer 20. For example, if readings are made and transmitted more-or-less instantaneously (i.e. within one second or a few seconds) by the wireless transmitter 18 then the time of receipt by the receiver apparatus may be associated with the readings as a time stamp. Processing of the readings discussed further below is somewhat dependent on the relative timings of the readings (i.e. so that contemporaneous readings from the different sensors can be identified as contemporaneous), however accuracy to the level of one second, a few seconds, or 10 seconds, is sufficient.

### Combining Data Transmission Routines

In a hybrid mode, capsules 10 may combine the two data transmission routines. For example, the capsule 10 may process sensor readings on-board to identify motility markers (and optionally also other readings or groups of readings of interest) for transmission in Bluetooth inquiry mode immediately post-excretion. In addition, the capsule 10 may continuously transmit sensor readings to a paired receiver apparatus. Optionally, the continuous transmission may be of the gas sensor signals only, or gas sensor signals and temperature sensor signals required to calibrate gas sensor signals. Gas sensor signals are of particular interest in providing health and clinical information, particularly once combined with motility indicators provided by the other sensors such as accelerometer, reflectometer. Gas sensor signals may be downsampled or subject to other compression techniques by the on-board processor prior to transmission. Optionally, the on-board processor hardware 151 may apply one or more filters, such as a high pass or low pass filter applied to the values themselves or to the derivative with respect to time, so that only gas sensor signals meeting particular thresholds are included in the data transmission payload. Metrics representing gas sensor signals, such as peak of a derived H2 value, or area under a plot of derived H2 value with respect to time, may be maintained and transmitted away from the capsule 10.

For capsules 10 configured to perform data transmission during passage through the GI tract (i.e. pre-excretion data transmission routine), commercial bands (such as 433 MHz, Bluetooth (2.4Ghz)) are used by the antenna 17 as electromagnetic waves in this frequency range can safely penetrate the mammalian tissues 40. Bluetooth may also be used in such capsules, wherein Bluetooth may be long-range Bluetooth, particularly when BMI of the subject (human) is above a threshold, or a high level of attenuation is expected for some other reason. Other commercial bands and protocols may be used in various applications, such as LoRa. Coding may be applied at the digitisation stage to assure that the data transmitted by the capsule 10 is distinguishable from data transmitted by other similar capsules 10. The transmission antenna 17 may be, for example, a pseudo patch type for transmitting data to the outside of the body data acquisition system.

### Power Source

Power source 16 is a battery or super capacitor that can supply the power for the sensors and electronic circuits including the processor hardware 151 and memory hardware 152. A life time of at least 48 hours may be set as a minimum requirement for digestive tract capsules. A number of silver oxide batteries in the power source 16 is configurable, depending on the needed life time and other specifications for the capsule. For example, long-range Bluetooth may consume more power than standard Bluetooth. Capsules may be configured to switch from long-range Bluetooth transmission to standard Bluetooth transmission once the stored energy in the battery (or batteries) drops below a predefined threshold, wherein the on-board processor is configured to monitor stored energy level.

### Relay Apparatus

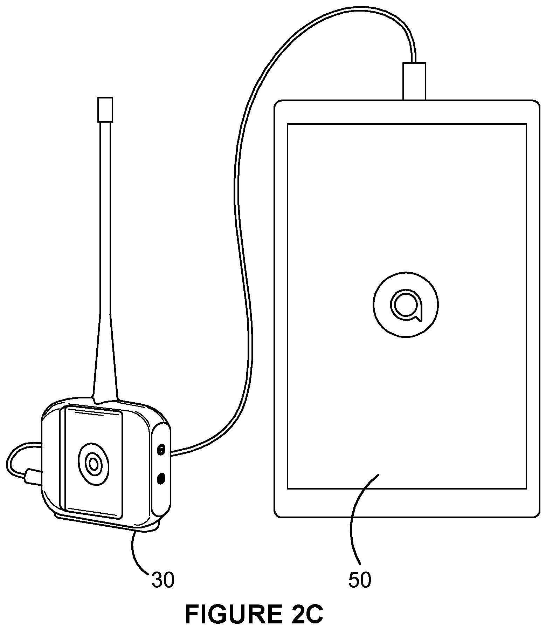

Figure 2C illustrates a receiver apparatus 30 connected to a relay apparatus 50. For example, the relay apparatus 50 may be an in-clinic Android tablet which is used to pair a capsule ID such as serial number to patent details that will appear on a generated report. The Android tablet is loaded with an application which features a series of screens that walk the clinician through the process of administering the capsule including instructing the patient to ingest the capsule. In some implementations, the timestamp of the instruction may be recorded as the ingestion event timing (for example if ingestion is witnessed by the clinician). Alternatively, (and as illustrated in Figs 2A to 2D) the ingestion event timing may be recorded via an interaction of the subject with a button on the receiver apparatus 30.

### On-Board Components

Internally the capsule 10 includes gas sensors 13, an environmental sensor 14, and a micro controller 15. The environmental sensor 14 may be a temperature sensor 14a or a humidity sensor 14b, or may be a temperature sensor 14a and a humidity sensor 14b. The internal electronics may also include a power source 16, for example, silver oxide batteries, an antenna 17, a wireless transmitter 18 and a reed switch. As illustrated in Figure 3A, the gas sensors 13 include a TCD gas sensor 131 and a VOC gas sensor 132.

The gas sensors 13 are less than several mm in dimension each and are sensitive to particular gas constituents including oxygen, hydrogen, carbon dioxide and methane. In fact, the VOC sensor 132 may be configured to give sensor side readings and driver or heater side readings. The heater side readings may be used to determine thermal conductivity of a surrounding gas and thereby the heater side readings of the VOC are TCD readings. The sensor side readings are used to determine concentrations of volatile organic compounds in the surrounding gases and are VOC readings. The TCD sensor 131 may be, for example, a heating element coupled to a thermopile output, with the thermopile temperature varying due to energy conducted into the gas at the location of the capsule 10. The TCD sensor 131 measures rate of heat diffusion away from the heating element.

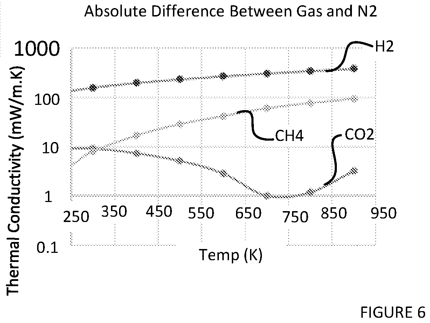

As illustrated in Figure 6, the heater side of the VOC sensor (operating as a TCD sensor) and the sensor side of the TCD sensor have different operating ranges, so TCD readings from the two sensors collectively span a wider range of operating temperatures than either of the sensors individually. Both sensors have heating elements. The TCD sensor has a low operating temperature but with a high precision. The heater side of the VOC increases the operating range but has a lower precision for TCD readings than the TCD sensor. The larger collective thermal range achieved by the two gas sensors 13 in concert enables better resolution of analytes in the second processing branch. The thermal conductivity of constituent gases in the gas mixture of the GI tract varies with temperature and so by obtaining TCD readings at different operating temperatures the different gases can be resolved from each other. This is leveraged in a second processing branch, which is to determine identity and concentrations of constituent gases in the gas mixture surrounding the capsule 10.

The gas sensors 13 are contained in a portion of the capsule 10 sealed from the power source 16 and other electronic components. The outer surface of this portion of the capsule is composed of a selectively permeable membrane. For example, the gas sensors 13 include respective heaters which are driven to heat sensing portions of the respective gas sensors 13 to temperatures at which sensor readings are obtained (i.e. a measurement temperature). The heaters may be driven in pulses so that there is temporal variation in the sensing portion temperature and so that measurement temperatures are obtained for periods sufficient to take readings but without consuming the power that would be required to sustain the measurement temperature continuously.

The gas sensors 13 are calibrated, so that a gas sensor reading can be used to identify the composition and concentration of a particular gas. Calibration coefficients are gathered in manufacturing and applied to the recorded readings at the processing stage (i.e. by a server such as on the cloud). Otherwise, this calibration could be performed on the capsule 10, at the receiver apparatus 30, or on any device having access to the calibration coefficients and the recorded readings from the gas sensors 13. Such calibration relates to a branch of processing concerned with measuring the concentration of constituent gases in the gas mixture at the capsule 10. Context for the outputs of that branch of processing is provided by another branch of processing, which determines (or predicts to within predefined confidence level) a location of the capsule 10 within the GI tract at which said gas mixture is found. In the location determination processing branch, some calibration may also be required in seeking to find gastric-duodenal transition indicators, since ingested foodstuffs at different temperatures change the environmental temperature in the stomach, which influences rate of heat diffusion. In the case of gas sensor readings taken after ingestion and before the gastric-duodenal transition (i.e. whilst the capsule 10 is in the stomach), processing of readings may include applying a moderation to TCD readings, from either gas sensor, in order to correct for variations in environmental temperature, based on environmental temperature readings by the environmental temperature sensor 14a. TCD readings are effectively measuring rate of heat loss to surroundings, and so accuracy is improved by measuring the temperature of the surroundings rather than by relying on assumption (i.e. prior knowledge of internal temperature of the subject mammal). However, the processing may rely on assumption, for example, if the capsule 10 does not include an environmental temperature sensor 14a or if there is some issue with the environmental temperature sensor readings, or, for example, if the level of accuracy provided by assumption is acceptable in a particular implementation. Gastric temperature may vary based on, for example, ingestion of liquids or foodstuffs by the subject mammal, or physical activity undertaken by the subject mammal 40. Environmental temperature is a term used in this document to refer to the temperature of the environment in which the capsule 10 is located, as distinct from operational temperatures of the gas sensors 13. The sensitivity of the gas sensors 13 to different constituent gases vary according to the operating temperature of the sensors and the processing of the readings includes calibrating (also referred to as moderating or correcting) readings from the gas sensors according to contemporaneous operating temperature and optionally also according to contemporaneous environmental temperature.

In addition to the gas sensors 13 and the environmental sensor 14, the capsule electronics further include a microcontroller 15, a power source 16, an antenna 17 or plural antennae, a wireless transceiver 18 or plural wireless transceivers, and optionally a reed switch (though in the case of there being two wireless transceivers the reed switch may be omitted). The wireless transmitter 18 operates in concert with the antenna 17 of the primary transceiver to transmit the data transmission payload including readings from the sensors (collectively referring to the gas sensors 13 and the environmental sensor 14) to a receiver apparatus 30 and/or a remote computer 20 for processing.

During a live phase of the ingestible capsule 10 (i.e. while the ingestible capsule 10 is in use, that is, in the GI tract of a subject mammal 40 and obtaining and transmitting readings), and assuming a pre-excretion data transmission routine, the wireless transmitter 18 of the primary transceiver transmits the readings to a receiver apparatus 30, which may be a dedicated device for receiving and storing the readings (and optionally with a user interface) or may be a multi-function device such as a mobile phone (such as a smart phone) running an application managing the role of the smartphone in receiving, processing, and/or transmitting data from the capsule 10. The subject mammal 40 need not remain within a specific range of the remote computer 20 during the live phase. The receiver apparatus 30 uploads the readings to the remote computer 20. The upload may be continuous during a live phase of the capsule, or the upload may be after the live phase of the capsule is terminated. In case of continuous transmission, the receiver apparatus 30 may also store the readings, so that loss of connectivity between the receiver apparatus 30 and the network is not critical. The receiver apparatus 30 may apply one or more pre-processing steps. Digitisation of the readings is performed either by the sensors themselves, by the microcontroller 15 or by the wireless transmitter 18. The digitised readings are transmitted via the antenna 17. The readings of the capsule 10 are made at an instant in time and are associated with the instant in time at which they are made. For example, a time stamp may be associated with the readings by the microcontroller 15, the wireless transmitter 18, or at the receiver apparatus 30 or remote computer 20. For example, if readings are made and transmitted more-or-less instantaneously (i.e. within one second or a few seconds) by the wireless transmitter 18 then the time of receipt by the receiver apparatus may be associated with the readings as a time stamp. Processing of the readings discussed further below is somewhat dependent on the relative timings of the readings (i.e. so that contemporaneous readings from the different sensors can be identified as contemporaneous), however accuracy to the level of one second, a few seconds, or 10 seconds, is sufficient.

Figure 3A illustrates the primary transceiver antenna 17 and directional coupler 171 as elements of the wireless transmitter 18, since the antenna is the physical means by which the wireless transmitter 18 transmits data to the receiver apparatus 30. The wireless transmitter 18 is also configured to buffer data for transmission. The wireless transmitter 18 may also be configured to encode the data with a code unique to the capsule 10 among a population of like capsules 10.

Interconnections between electronic components in Figure 3A are illustrated as being via a central bus. This is one example of how power and data may be distributed between components. Other circuitry architecture may be implemented, for example, all connections may be via the microcontroller 15 which coordinates distribution of data and power between components. The sensors (the TCD sensor 131, the VOC sensor 132, the environmental sensor 14, the accelerometer 19, and the directional coupler 171) take readings under the instruction of the microcontroller 15, powered by the power source 16, and transfer the readings to the wireless transmitter 18 for transmission to the receiver apparatus via the antenna 17.

The dimension of the capsule may be less than 11.2 mm in diameter and 27.8 mm in length. The housing of the capsule 10 may be made of indigestible polymer, which is biocompatible. The housing may be smooth and non-sticky to allow its passage in the shortest possible time and to minimise risk of any capsule retention.

### Data Processing

Processing may be performed in more-or-less real time, allowing for latency caused by transmission and processing. Alternatively, the readings may be received by a receiver apparatus 30 and stored for upload and processing retrospectively. Such retrospective processing may be performed by analysing the most recent readings first (i.e. in reverse chronological order), so that the first event timing to be determined is egestion, followed by ICJ, then GET, then ingestion. Or the analysis may be of the readings in chronological order.

The remote computer 20 may process the readings, or processing may be performed on-board the capsule 10, or by the receiver apparatus 30. Some combination of those devices may perform the processing. The processing may be considered to include two branches: a first (motility) branch to determine a location of the ingestible capsule 10 within the GI tract based on the readings; and an optional second (gas composition) branch to determine constituent gases and the concentrations thereof in the gas mixture at the location of the ingestible capsule 10. The embodiments discussed herein are primarily concerned with the first (motility) branch, noting that a particular benefit of accurately determining the location of the ingestible capsule 10 in the GI tract is to provide context to the determinations of the second (gas composition) branch. However, it is noted that the outcomes of the first (motility) branch of processing provide useful information in the assessment of gut health even in the absence of the second (gas composition) branch of processing, and may have other utility beyond the second (gas composition) branch of processing. Optionally, determinations of the second (gas composition) branch of processing may be utilised to add confidence to determinations in the first (motility) branch of processing.

Readings from different sensors or pseudo sensors will be used in the first (motility) branch and/or the second (gas composition) branch as appropriate. For example, the TCD gas sensor readings are utilised for detecting a gastric duodenal transition indicator in the first (motility) branch, and in the second (gas composition) branch for, for example, determining concentration of H2 at the location of the capsule 10. The readings from the VOC heater side are used in the second (gas composition) branch as a hotter TCD sensor, to increase the temperature range at which TCD readings are obtained and thus to increase the range of H2 concentrations that are detectable. The VOC sensor side is sensitive to both O2 and H2 as well as other gases and so these readings may be utilised in the second (gas composition) branch. Other gases include CH4 and SCFAs. Optionally, the VOC sensor side readings are not used in the second (gas composition) branch and the VOC sensor side readings are only used to detect an ileocecal junction indicator. Optionally, the VOC sensor side (i.e. the VOC sensing element) forms a resistor in a voltage divider network, the output of which is measured as the VOC sensor side live reading. A transform may be applied at the capsule 10 and/or as part of the processing to transform the output of the voltage divider network into a resistance measurement from the sensing element. The VOC sensor side may be driven with a consistent (i.e. repeated) voltage pulse profile. VOC sensor side readings may be taken in sync with the voltage pulse profile so that there is no phase shift between the voltage pulse and the timing of the readings. CH4 concentration is determined from the TCD gas sensor readings and/or from the VOC heater side readings.

### Primary Transceiver Configuration and Antenna Reflectance Related Readings

Commercial bands (such as 433 MHz and Bluetooth 2.4GHz) are used by the antenna 17 of the primary transceiver as electromagnetic waves in this frequency range can safely penetrate the mammalian tissues 40. Other commercial bands may be used. Coding may be applied at the digitisation stage to ensure that the data transmitted by the capsule 10 is distinguishable from data transmitted by other similar capsules 10. The transmission antenna 17 may be, for example, a pseudo patch type for transmitting data to the outside of the body data acquisition system. Power source 16 is a battery or super capacitor that can supply the power for the sensors and electronic circuits. A life time of at least 48 hours is required for digestive tract capsules. A number of silver oxide batteries in the power source 16 is configurable, depending on the needed life time and other specifications for the capsule.

The antenna 17 may be in series with a directional coupler 171. The directional coupler 171 and the antenna 17 are configured as a reflectometer. The reflectometer measures the amplitude of reflected signals by means of a diode detector. The amplitude measurements of the reflectometer are readings that represent electromagnetic properties of material in the vicinity of the capsule. For example, good impedance matching between the antenna and the environment surrounding the capsule 10 will result in a low amplitude reflectance signal and therefore low amplitude measurement. Poor impedance matching between the antenna and the environment surrounding the capsule 10 will result in a high amplitude reflectance signal and therefore high amplitude measurement.

In the example above, the reflectometer measures amplitude of reflected signal at the antenna 17 of the primary transceiver. Optionally, the reflectometer may be configured to measure phase of the reflected signal. For example, the capsule 10 may comprise a quadrature demodulator to extract phase information from the reflected signal. Phase information provides a dimension in addition to the amplitude information with which to represent the reflected signal. In a first example, the phase information from the reflected signal may exhibit a step change at a change in environment surrounding the capsule so that analysis of the phase information provides a motility event indicator. **In** a second example, discussed below in more detail, the phase information enables a determination to be made of how to modify an antenna control signal to better match the antenna impedance to the impedance of the environment.

Quadrature demodulation converts modulation of the reflectance signal into imaginary and real baseband signals. The quadrature demodulators are driven by carrier frequency (carrier frequency being frequency of transmission by primary transceiver) sinusoids with a 90 degree phase difference to generate two baseband signals that can be compared to generate phase information. Low pass filtering may be applied (to each of the imaginary and the baseband signals) to filter out high frequency content at around double the original baseband frequency.

The reflectometer readings (being one or both of the amplitude and phase readings) provide a basis for differentiating between gaseous, liquid, and solid matter at the location of the capsule in the GI tract. The reflectometer readings (being one or both of the amplitude and phase readings) provide a basis for differentiating between different physical environments surrounding the capsule 10. Readings of the reflectometer enable the antenna 17 and directional coupler 171 to operate in cooperation as an environmental dielectric and impedance sensor.

### Reflectometer: Tuneable Antenna

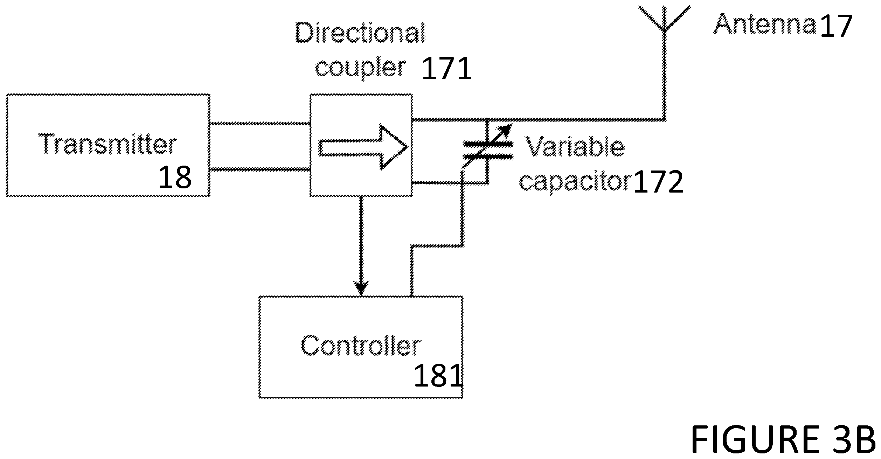

Figure 3B illustrates a particular example of a reflectometer. The capsule 10 is configured to transmit its data transmission payload captured via the on-board sensors and quasi-sensors via radio signals to a receiver apparatus 30. Since available energy is limited within the capsule 10, the capsule 10 may be configured to transmit radio signals in an energy-efficient manner. The constrained volume and shape of the capsule 10 in combination with the varying electromagnetic properties of the surrounding environment during transit through the GI tract of the subject mammal mean that impedance matching between the antenna 17 and the surrounding environment is difficult to achieve. Transmission efficiency is improved with better impedance matching. The transmitter 18 may be, for example, control circuitry of the transceiver including a buffer buffering data for transmission.

The transceiver illustrated in Figure 3B comprises a tuneable antenna 17. A reflected signal from the antenna 17 is generated during transmission, received at the directional coupler, and processed at the controller 181 to extract one or both of amplitude and phase information from the reflected signal.

Amplitude provides a measure of amount of reflected energy. Phase information provides information about how the phase shifts between the transmitted and reflected signals. Step changes in either or both may be caused by a change in electromagnetic properties of a transmission environment, that is, an environment in which the capsule 10 is located. Therefore, the reflectometer measurements (being a collective term applied to either or both of the amplitude information and the phase information), either by virtue of their absolute values (and via reference to calibration information such as a lookup table), and/or by virtue of the presence of a step change in their values (in which case calibration information is not required), provide an indicator of an environment surrounding the capsule or of a change in environment surrounding the capsule.

The antenna 17, directional coupler 171, controller 181, variable capacitor 172, form a closed loop mechanism to measure the efficiency of the antenna (wherein amplitude of reflected signal measures efficiency, low amplitude indicating efficiency and high amplitude indicating inefficiency), and to generate a control signal by the controller 181 to a variable capacitor 172 to minimise the antenna reflectance. Depending on how the reflectometer is configured, it may be that the controller 181 is configured to incrementally change the control signal to the variable capacitor 172, to compare amplitude reading with an amplitude reading before the incremental change, and based on the comparison, to determine whether to reverse the direction of the incremental changes or not. Otherwise, in reflectometers that extract the phase information, the phase information itself may inform the controller 181 in which direction the control signal should be varied to reduce the amplitude readings.

The controller 181 is configured, based on the antenna reflectance related readings, to generate a control signal to vary the capacitance of the variable capacitor 172 which varies the impedance of the antenna 17. A control algorithm is responsible for determining the control signal output by the controller 181 to vary the capacitance of the variable capacitor 172 to vary the impedance of the antenna 17 to reduce amplitude of reflected signal from the antenna 17. The controller 181, may generate the control signal empirically by periodically adjusting the control signal in a given direction, comparing a reflectometer amplitude reading pre- and post-adjustment, and changing the direction of adjustment for the next periodical adjustment if the reflectometer amplitude reading increase from pre- to post-, and maintaining the direction of adjustment for the next periodical adjustment if the antenna reflectance related readings decreases from pre- to post. The controller may generate the control signal determinatively based on the reflectometer phase information wherein a particular phase reading value range indicates the controller is to increase the control signal and a particular phase reading value range indicates the controller is to decrease the control signal, and optionally a particular phase reading value range indicates the controller is to maintain the control signal. The level of the control signal generated by the controller 181 is proportional or directly proportional to the capacitance of the variable capacitor 172 and therefore to the impedance of the antenna 17. Since, as detailed above, the antenna 17, controller 181, and variable capacitor 172 form a closed loop or feedback loop mechanism to impedance match (i.e. to reduce reflected signal amplitude) the antenna 17 to the surrounding environment, it follows logically that the control signal generated by the controller 181 to set the capacitance of the variable capacitor is proportional to the impedance of the environment surrounding the capsule 10. Therefore, the control signal may itself be recorded as an antenna reflectance related reading representative or indicative of the environment surrounding the capsule 10.

The reflectometer, configured with the directional coupler 171, controller 181, variable capacitor 172, and antenna 17, forming a closed loop (that is, a feedback loop), provides automatic tuning of the antenna 17 to increase transmission efficiency. Furthermore, the control signal from the controller 181 to the variable capacitor 172, as detailed above, is indicative of impedance of the antenna 17 and therefore also of the environment surrounding the capsule 10, and therefore the control signal may itself be sampled as an antenna reflectance related readings for use in motility processing. Changes in the control signal or even absolute values of the control signal itself (combined with a calibrated lookup table) provide indicators of capsule 10 location within the GI tract of the subject mammal.

The transmitter 18 in the context of Figure 3B is the circuitry providing the transmission signal (that is, the carrier wave with the encoded data transmission payload, and any metadata etc required by the transmission protocol). The transmitter 18 may be a Bluetooth transmitter.

The readings of the ingestible capsule 10, which include one or more from among readings from: the environmental sensor 14, the heater side 132b of the VOC gas sensor 132, the sensor side 132a of the VOC gas sensor 132, and the TCD gas sensor 131, may also include readings of the reflectometer. Hence, change in capsule location within the GI tract causes a change in antenna reflectance related readings, and therefore provide an indicator that a transition event between two sections of the GI tract has occurred.

### Ingestible capsule: accelerometer

The ingestible capsule 10 may further comprise an accelerometer 19. The accelerometer 19 may be a tri-axial accelerometer. A rate of change of angular position or orientation of the capsule 10 is somewhat dependent upon location within the GI tract, and therefore accelerometer readings provide an indicator that a transition event between two sections of the GI tract has occurred. The accelerometer readings may measure angular acceleration about three axes of rotation, wherein the three axes of rotation may be mutually orthogonal.

### Off-board processing

The ingestible capsule 10 of Figures 1A to 3 is a data collection and data transmission device. As mentioned elsewhere in this disclosure, the collected data (i.e. the readings) are uploaded to a remote computer 20 for processing, via a receiver apparatus 30, which may be a relay apparatus for transmitting the readings to the remote computer or may be directly connectable to the remote computer for uploading the readings thereto. For example, the receiver apparatus 30 may comprise a memory readable by the remote computer 20. The receiver apparatus 30 provides a data connection, such as a wired connection, a network connection, or a plug-socket connection, to a remote computer 20, either directly or via a network such as the internet via which the readings are relayed to the remote computer 20. In this manner, the capsule 10 need only be configured to establish a data connection with a receiver apparatus 30, which may be a dedicated device for receiving and storing the readings (and optionally with a user interface) or may be a multi-function device such as a mobile phone (such as a smart phone) in order that the subject mammal need not remain within a specific range of the remote computer 20 during the live phase. The receiver apparatus 30 uploads the readings to the remote computer 20. The upload may be continuous during a live phase of the capsule, or the upload may be after the live phase of the capsule is terminated. In case of continuous transmission, the receiver apparatus 30 may also store the readings, so that loss of connectivity between the receiver apparatus 30 and the network is not critical.

### Processing Recorded Readings

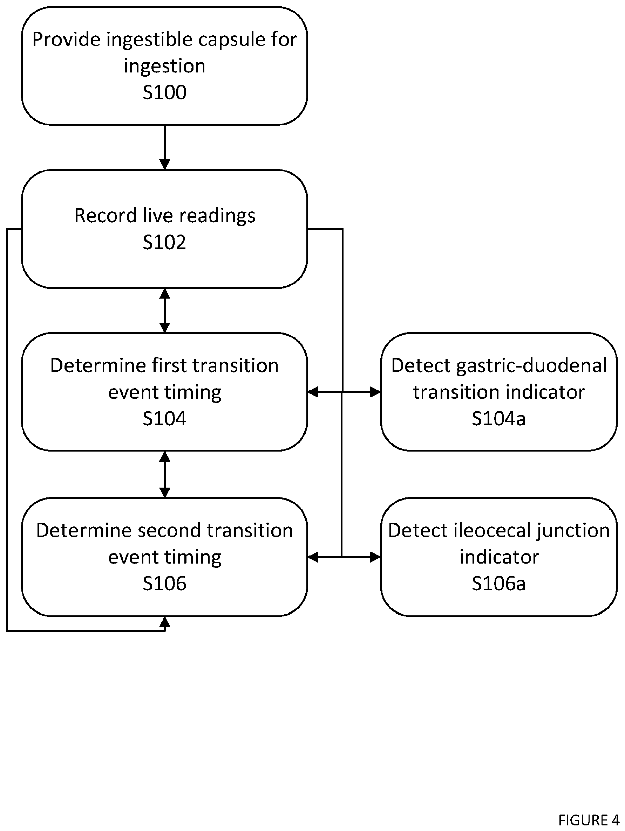

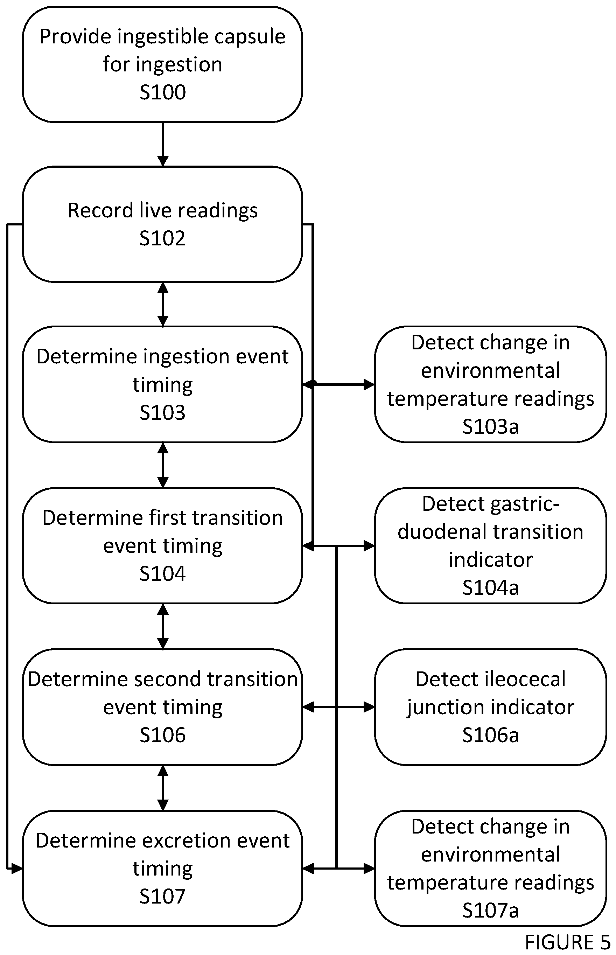

Methods including obtaining the readings and processing the readings are illustrated in Figures 4 & 5. The processing can be considered to include two branches: a first (motility) branch to determine a location of the ingestible capsule 10 within the GI tract based on the readings; and a second (gas composition) branch to determine constituent gases and the concentrations thereof in the gas mixture at the location of the ingestible capsule 10. The second (gas composition) branch is optional and is an example of utility of the outcomes of the first (motility) branch of processing. This disclosure is principally concerned with the first (motility) branch. The second (gas composition) branch is conducted in parallel with the first (motility) branch, or may be delayed with respect to the first (motility) branch, since the second (gas composition) branch leverages the determinations of the first (motility) branch to detect illnesses or conditions. For example, the second (gas composition) branch comprises processing readings of the TCD gas sensor 131, the VOC gas sensor 132 to determine constituent gases and the concentrations thereof in the gas mixture at the location of the ingestible capsule 10, and to use the determinations of the first (motility) branch of processing to determine the location of the ingestible capsule 10. In this way, it can be determined that the determined gases and concentrations are post-ingestion-pre-gastric-duodenal-transition, or post-gastric-duodenal-transition-pre-ileocecal-junction, or post-ileocecal-junction-pre-excretion. However, it is noted that the outcomes of the first (motility) branch of processing provide useful information in the assessment of gut health even in the absence of the second (gas composition) branch of processing, and may have other utility beyond the second (gas composition) branch of processing. Optionally, determinations of the second (gas composition) branch of processing may be utilised to add confidence to determinations in the first (motility) branch of processing.

Methods may include storing and/or transmitting the TCD gas sensor readings and the VOC gas sensor readings from among the readings, recorded as a function of time, along with the determined timings of the first transition event and the second transition event (and optionally also of the ingestion event and the excretion event), for analysis. For example, the analysis may comprise diagnosis of one or more conditions or illnesses associated with production of particular constituent gases or concentrations thereof at particular locations or sections of the GI tract.

Figure 4 illustrates a method for providing S100 an ingestible capsule 10 to a mammal for ingestion, recording S102 readings from the capsule 10, and determining a location of the capsule 10 in terms of sections of the GI tract by determining a first transition event timing S104 and a second transition event timing S106.

Figure 5 illustrates a specific example of the method of Figure 4, which further includes determining an ingestion event timing S103 and an excretion event timing S107.

As stated elsewhere, the readings may be processed chronologically or reversechronologically. So, the order in which steps are performed in Figure 4 may be:
- chronological processing: S100; S102; S104 & S104a; S106 & S106a; or
- reverse chronological processing: S100; S102; S106 & S106a; S104 & S104a.
Or in the case of Figure 5:
- chronological processing: S100; S102; S103 & S103a; S104 & S104a; S106 & S106a; S107 & S107a; or
- reverse chronological processing: S100; S102; S107 & S107a; S106 & S106a; S104 & S104a; S103 & S103a.

Bounds on the readings analysed for detecting indicators of an event are set by the already determined events. So, in the case of chronological processing, in detecting event indicators, readings are analysed on a rolling basis forwards from a determined timing of the preceding event (in the case of detecting ingestion the preceding event is initiation). In the case of reverse chronological processing, in detecting event indicators, readings are analysed on a rolling basis backwards from a determining timing of the processing event (in the case of detecting excretion the proceeding event is termination which may be set by there being no further readings or by a subject on a user interface). So, the processing may be:
- chronological processing: S100; S102; S104 & S104a; S106 & S106a (with earlybound provided by S104);

- -reverse chronological processing: S100; S102; S106 & S106a; S104 & S104a (with late bound provided by S106).
Or in the case of Figure 5:
- - chronological processing: S 100; S102; S103 & S 103a (with early bounds provided by initiation event); S104 & S104a (with early bounds provided by S103); S106 & S106a (with early bound provided by S104); S107 & S107a (with early bounds provided by S106); or
- -reverse chronological processing: S100; S102; S107 & S107a (with late bound provided by termination event); S106 & S106a (with late bound provided by S107); S104 & S104a (with late bound provided by S106); S103 & S103a (with later bound provided by S104).

For the sake of brevity and ease of comprehension, discussion of Figures 4 & 5 relates to the chronological processing example with rolling averages being forward from an early bound starting point. However, the above provides instructions enabling the disclosure to be applied to reverse-chronological processing with rolling averages being backwards from a late bound starting point.

### Initiation

References to an initiation event refer to: a power on event of the capsule initiating a live phase during which the capsule is active and readings are generated by the sensors and received by the receiver apparatus; or an initiation of recording by a button press on a user interface of the receiver apparatus 30 (so that it is possible that the capsule is already powered). The live phase refers to the time during which the capsule is powered on and readings are being recorded (i.e. stored or relayed) by the receiver apparatus 30.

Initiation event may be an encoded activation control signal received by a secondary transceiver, being an NFC transceiver, at the capsule. The encoded activation control signal being transmitted by an NFC transceiver of the receiver apparatus 30.

### Termination

References to a termination event refer to an end of the live phase, which termination event may be: a power off event of the capsule ending the live phase; or a termination of the live phase by a button press on a user interface of the receiver apparatus 30.

### Ingestion

At S100 the ingestible capsule 10 is provided to the subject mammal 40 for ingestion. The ingestible capsule 10 is as illustrated in any of Figures 1A to 3 and comprises, inter alia, a housing 11, a power source 16, an environmental sensor 14, a TCD gas sensor 131, and a VOC gas sensor 132. The ingestible capsule 10 may be stored in a powered down state in contact with packaging, wherein separating the ingestible capsule 10 from the packaging causes the powered down state to end and the capsule 10 to enter a powered state. Entering of the powered state may be the initiation event, or the initiation event may require the capsule to enter the powered state and a button press (or other interaction) with a user interface on a receiver apparatus 30. It may be that separation of the capsule 10 from the packaging is the event that causes the capsule to be powered on and the obtaining and transmission of readings by the capsule 10 to begin.

It is foreseen that ingestion will take place soon after entering the powered state, wherein soon is taken to mean within 15 minutes, within half an hour, or within one hour. Subjects may be instructed, via the application and/or via a note on the packaging of the capsule 10, to activate the capsule (i.e. NFC activation via application) only when ready to ingest, so that time between activation and ingestion can be kept to a minute or less.

### Recording Readings

At S102 recording of the readings begins. The readings are recorded by the receiver apparatus 30, which either relays them immediately to a remote computer 20 for processing or stores them for later upload to the remote computer 20. The readings include readings of the TCD gas sensor 131, readings of the sensor-side of the VOC gas sensor 132a, and may also include one or more from among environmental sensor readings, readings from the heater side of the VOC sensor 132b, antenna reflectance related readings (i.e. from the antenna 17 and directional coupler 171), and readings from the accelerometer 19. The readings are recorded as a function of time. The temporal value assigned to each live reading may be assigned at the capsule 10, for example by the microcontroller 15 and/or the wireless transmitter 18, may be assigned by the receiver apparatus 30 based on a time of receipt of the respective readings from the capsule 10, and/or may be assigned by the remote computer 20 based on time of receipt from the capsule 10 or from the receiver apparatus 30. Alternatively or additionally a temporal value assigned to each live reading may be based on order of arrival. For example, if it is known that TCD gas sensor readings are obtained every n seconds, then the mth reading is timed at m x n seconds (or m-1 x n, depending on implementation) after the initiation event starting the live phase. It is noted that temporal values may be relative to a baseline such as the capsule 10 entering a powered state rather than being absolute values of time based on a calendar and time of day value.

The steps are illustrated in a serial manner in Figures 4 & 5, but in practice the obtaining and recording the readings S102 may be performed whilst the processing steps S103 to S107 are being performed (in the case of chronological processing, evidently for reverse chronological processing the live phase is terminated before the processing steps are performed). Optionally, the processing may be performed after the recording of the readings S102 has been completed and the capsule excreted. The processing may be performed on the cloud. The processing may be performed on server computing apparatus connectable to the capsule 10 via an internet connection to a receiver apparatus 30. The receiver apparatus itself may perform some or all of the processing steps S103 to S107.

### Processing to determine motility event timing

Steps S103 to S107 are processing steps and relate to analysing the recorded readings in order to determine whether or not specific events have occurred that enable the location of the capsule 10 within the GI tract to be determined. The processing is not necessarily intended to determine a live or contemporaneous location of the capsule 10, but to determine a location of the capsule 10 at the timing of recorded readings. The location at the timing of subsets of the readings is the intended output, rather than the location at the timing of the processing. For example, to determine that the capsule was in a particular section of the GI tract at a timing at which a series of readings were obtained from the VOC gas sensor 132 or the TCD gas sensor 131. Or, for example, to determine a timing of passage through sections of the GI tract which are themselves an indicator of gut health.

Each determination step: determining ingestion event timing S103; determining first transition event timing S104; determining second transition event timing S106; determining excretion event timing S107; has a respective associated detection step. In general, the detecting steps comprise processing and analysing the recorded readings to identify indicators (i.e. markers) that indicate an event associated with the motility of the capsule 10 may have occurred. The respective determining step, in addition to the detecting, includes applying a condition or some other logic to the detected indicator to determine (to within a confidence level) that the indicator was caused by a motility event, and thus the motility event can be determined to have occurred at (or around) the timing of the detected indicator. Motility events include one or more from among the ingestion event, the gastric-duodenal transition, the ileocecal junction transition, and the excretion event. Gastrointestinal motility is defined by the movements of the digestive system, and the transit of the contents within it. The indicator is a feature in a plot of recorded readings vs time from the relevant sensor or pseudo sensor. The feature is a step, bump, inflexion point, or gradient change. Particular indicators may be more specific, for example the condition may be more specific than the indicator simply being a step, bump, inflexion point or gradient change.

Indicators may be detected in readings from a first sensor. An indicator is associated with a hypothesis that the indicator was caused by an event associated with the motility of the capsule. Confidence may be added to the hypothesis by obtaining readings from other sensors at the timing of the indicator (and around said timing) and detecting confirmatory indicators in those readings. For example, hydrogen (H2) levels vary through the GI tract and so readings of H2 levels may be used to add confidence to readings from other sensors. Readings of H2 levels may be used as a basis for an ileocecal junction transition indicator. In particular, an ileocecal junction transition indicator may be detected by identifying an increase in (sensor side) VOC gas sensor output exceeding a predefined threshold with a contemporaneous, or temporally adjacent to within a predefined temporal distance either side, increase in H2 levels exceeding a predefined threshold. Noting that H2 levels are determined from the TCD gas sensor output and/or heater-side VOC sensor output.

Similarly, readings of CH4 levels may be used as a basis for an ileocecal junction transition indicator. In particular, an ileocecal junction transition indicator may be detected by identifying an increase in (sensor side) VOC gas sensor output exceeding a predefined threshold with a contemporaneous, or temporally adjacent to within a predefined temporal distance either side, increase in CH4 levels exceeding a predefined threshold. Noting that CH4 levels may be determined from the TCD gas sensor output and/or heater-side VOC sensor output.

Different subsets of the recorded readings may be analysed in order to detect different indicators. The subsets may be partitioned according to timing and according to the sensor from which they were obtained. Partitioning according to timing is discussed above with respect to early bounds and late bounds for chronological processing and reverse chronological processing.

It is noted that the term sensor is used broadly to encompass not only the sensors per se (i.e. the TCD gas sensor 131, the sensor side of the VOC gas sensor 132a, and optionally the environmental sensor 14 and/or the accelerometer 19), but also the components that provide readings and are not sensors per se, such as the directional coupler 171 and the heater side of the VOC sensor 132b (which components may be referred to as pseudo sensors). The term sensor encompasses the sensors per se and the pseudo sensors.

At S103a the recorded readings from the environmental sensor 14 are analysed to detect a change in the environment that would indicate an ingestion event. In this context, the change may be a change in environmental temperature indicated by the readings of the environmental temperature sensor 14a, or the change may be a change in environmental humidity indicated by the readings of the environmental humidity sensor 14b. The detection may be based on readings from both the environmental temperature sensor 14a and the environmental humidity sensor 14b, either to add confidence to one another, or to account for unusual ambient humidity or temperature conditions which could reduce the change in one condition of the other upon ingestion (i.e. ingestion on a hot day may not register a significant temperature change, but would, in many circumstances, register a significant humidity change). In chronological processing, the analysis may be of environmental sensor readings from an initiation event (such as power on of the capsule 10) forwards, with a temporal upper bound being set by determination of the ingestion event timing. That is, once it is determined that a detected change in the environmental sensor readings is caused by an ingestion event, no further analysis to detect an ingestion event is performed. In reverse-chronological processing, the analysis may be of environmental sensor readings from a gastric-duodenal transition event backwards, with a temporal lower bound being set by determination of the ingestion event timing. That is, once it is determined that a detected change in the environmental sensor readings is caused by an ingestion event, no further analysis to detect an ingestion event is performed. Regardless of processing direction (wherein processing direction refers to chronological or reverse-chronological), detecting the change at S103a may be on a rolling basis by comparing a subject one or more readings with a predetermined number of preceding readings, with a difference of more than a threshold (i.e. one or two degrees centigrade or one or two % relative humidity) being a detected change. Determining the ingestion event timing may include comparing the temperature or humidity of the subject reading with an expected temperature or humidity for the environment at the start of the GI tract of the subject mammal 40, wherein being within a threshold is a determination that the capsule 10 has been ingested. Alternatively the condition may be that a predefined number or more consecutive readings are within a threshold of the expected temperature or humidity for the environment at the start of the GI tract of the subject mammal.

### Determining Ingestion Timing

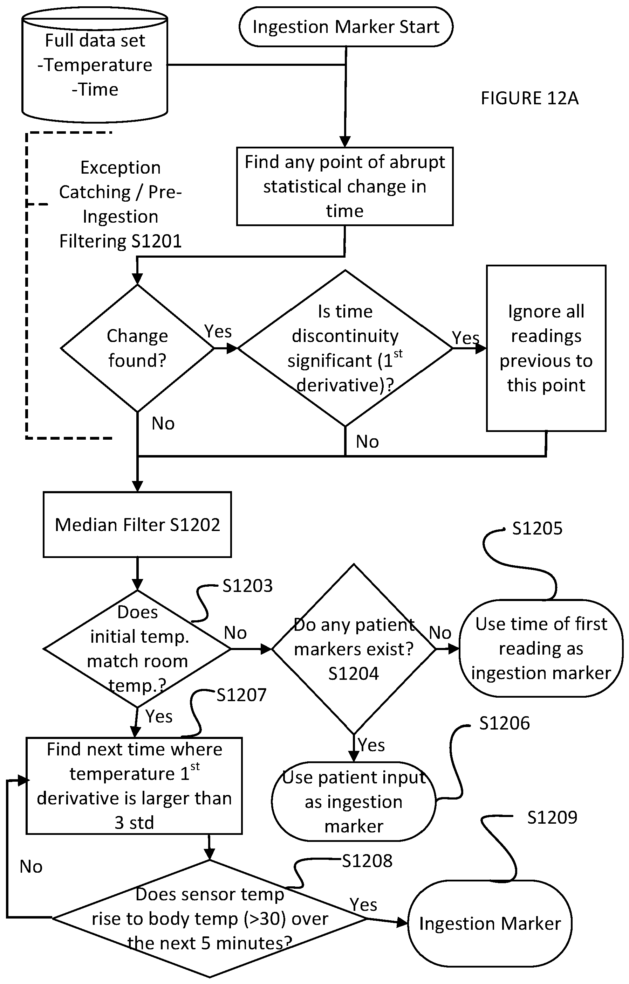

Figure 12a illustrates an exemplary algorithm for determining ingestion timing. At S1201 abrupt changes in the environmental sensor readings are detected as potential ingestion indicators, and exception catching and pre-ingestion filtering is applied and at S1202 median filtering is applied. The algorithm preferentially determines ingestion automatically by, at S1203, checking whether temperature at the start of the abrupt change is within a predefined range for room temperature. If not, then at S1204 a check is performed for a patient marker (i.e. a patient marker is an ingestion indicator provided by the patient via a user interface on the receiver apparatus 30 such as a button push, or an ingestion indicator provided via an application running on a device at a clinic at which the subject is a patient). So, if yes at S1204, the existence of the patient marker is sufficient to be used as the ingestion marker and the ingestion timing is taken to be the timing of the patient marker at S1206. If no patient marker exists at S1204 then at S1205 the timing of the first reading of the abrupt change is taken as ingestion timing. If the temperature at the start of the detected abrupt change is within predefined room temperature range at S1203, then processing proceeds to S1207 and the next time where temperature 1^{st} derivative is larger than 3 standard deviations is found at S1207, and at S1208 a check is performed on whether the environmental temperature sensor readings show a rise to within a predefined range for body temperature over the proceeding 5 minutes. If not, the flow returns to S1207. If yes, the flow proceeds to S1209 and the timing of the temperature rise is the determined ingestion timing.

### Sensors, Algorithms, Processing

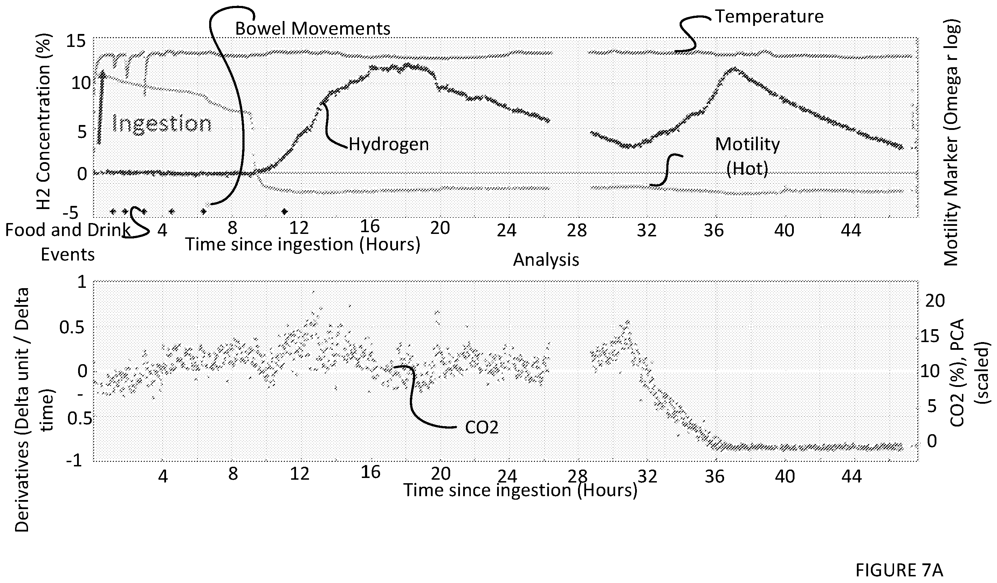

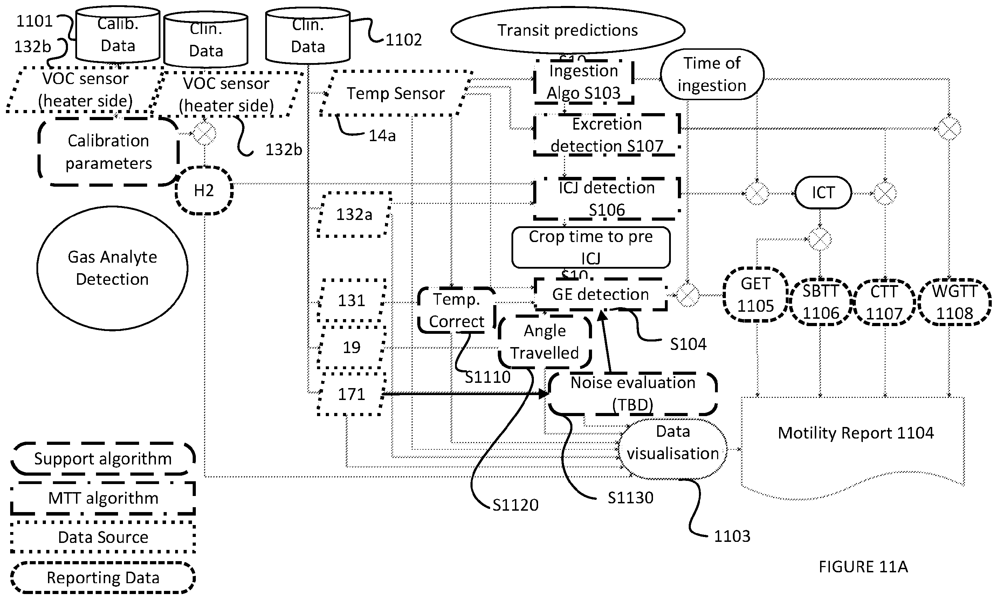

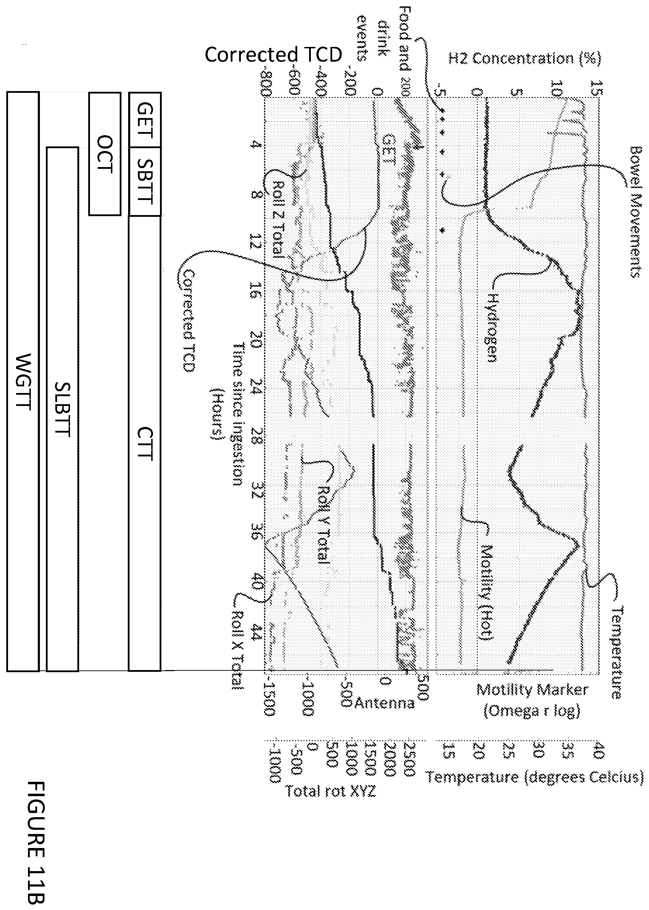

Figure 11a illustrates the relationships between sensors, algorithms, and processing results in an embodiment. Calibration data 1101 is lookup tables etc for calibrating the VOC sensor for operating as a TCD sensor in different environmental temperatures, which combines with the heater side of the VOC sensor 132b to provide calibration parameters. Clinical data 1102 is the knowledge that changes in VOC sensor heater side readings are associated with a change in H2 concentration in subject gas mixture, which feeds into ICJ detection at S106 & S106a, and is in itself an output data entity at 1103. Like reference numerals are used for equivalent features in other Figures, and so a full description of the features of Figure 11a is disclosed herein by reference to the other Figures. Noting that the ingestion algorithm executes steps S103 & S103a, the excretion detection algorithm executes steps S107 & S107a, the ICJ detection algorithm executes steps S106 & S106a, and the gastric emptying algorithm executes steps S104 & S104a. Step S1110 is the correction of the TCD sensor readings to account for changes in environmental temperature. Step S1120 is applying an algorithm to process the accelerometer data as described below in relation to the first, or angle travelled, technique. S1130 is an exemplary processing algorithm for the antenna reflectance related readings and is determining changes in noise in the output signal thereof. Other processing algorithms may be applied to the accelerometer and readings. Algorithms S1110, S1120, & S1130, may be considered to be pre-processing algorithms. The event timings determined by the algorithms are combined with one another to determine event timings of ingestion, gastric emptying, ileocecal junction transition, and excretion. The event timings are in turn combined to determine transit time metrics including gastric emptying timing 1105, small bowel transit time 1106, colon transit time 1107, and whole gut transit time 1108. These are included in an output motility report 1104. A data visualisation 1103 is such as illustrated in Figures 7A, 7B, 7C, 8, 9A, & 9B, for example. Figure 11B shows an exemplary data visualisation marked with transit time metrics.

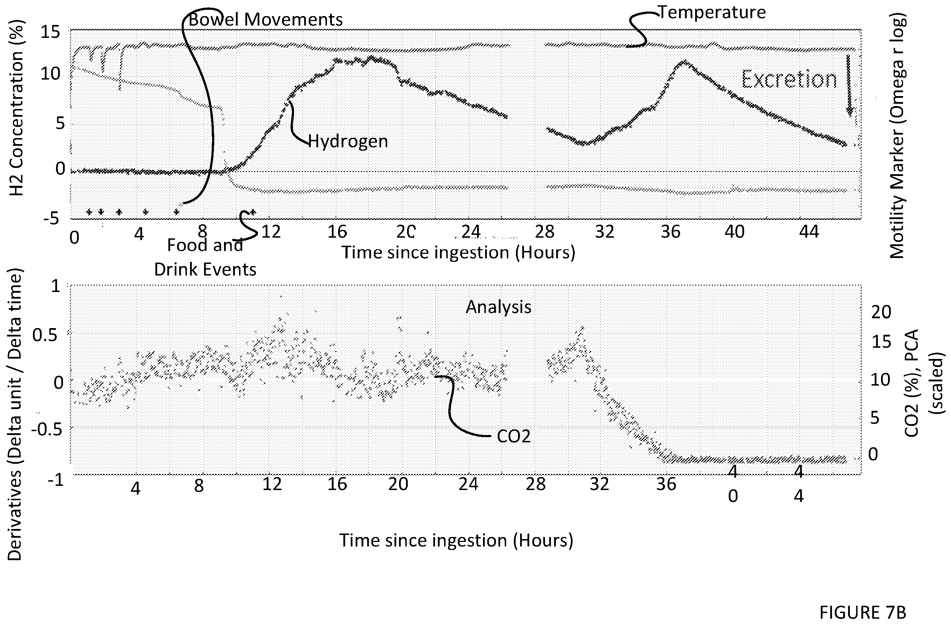

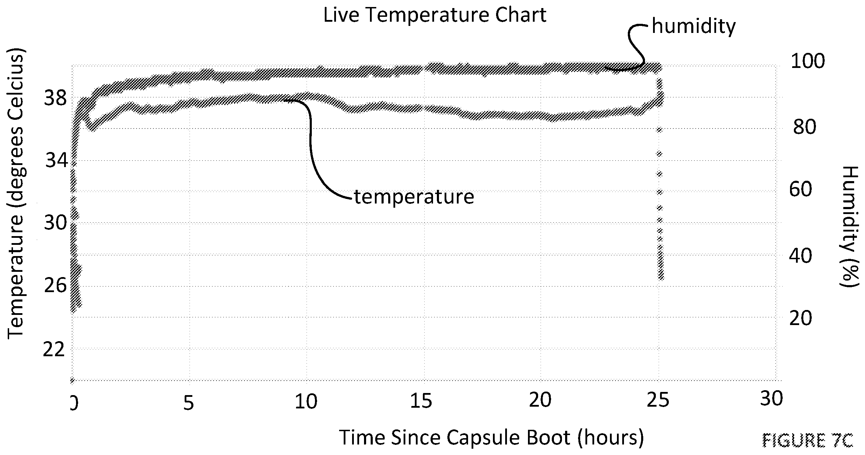

Figures 7A & 7B illustrate plots of capsule readings vs time since initiation event (boot) for an ingestible capsule 10 which is ingested by a subject human, makes its way through the GI tract, and is then excreted. The ingestion events and excretion events are marked. In the example, the external temperature is well below the internal temperature of the subject human. The plots also show hydrogen readings, motility readings, and CO2 readings, and are marked with food and drink events and bowel movement events (which events may be detected automatically or manually reported). The specific timing assigned to the ingestion event and the excretion event can be determined in a number of ways. Figure 7C shows environmental temperature sensor readings and environmental humidity sensor readings vs time since initiation event (boot) for an ingestible capsule 10 which is ingested by a subject human, makes its way through the GI tract, and is then excreted. The events are not marked since it is evident from Figure 7A where the ingestion and excretion indicators are detectable in Figure 7C. The specific timing assigned to the ingestion event and the excretion event can be determined in a number of ways, noting that processing of readings may be performed chronologically or reverse-chronologically. An example for the ingestion event: on a progressive (i.e. rolling) basis from a starting point being initiation event (in chronological case) or gastric-duodenal transition timing (in reverse-chronological case), determine mean value of three adjacent environmental sensor readings, determine a timing at which the mean value either began or ceased to be within a threshold distance of the expected environmental value (i.e. within 1 degree centigrade of expected temperature or within 1, 2, 5, or 10% of expected humidity) post-ingestion (i.e. average internal environment for subject), then determine that the ingestion event timing is the during the three readings (for example, the mid-point, the earliest point, or the latest point). The number three is exemplary and different numbers for the number of samples in the rolling average could be selected, such as five, ten, twelve, or twenty. Furthermore, the tolerance of 1 degree centigrade is configurable and could be, for example, 2 degrees, 3 degrees etc.

In the above example, ingestion event timing is determined by detection of an ingestion indicator (rise in environmental temperature readings) in the readings of the temperature sensor. The ingestion event timing is contemporaneous with the ingestion indicator. An ingestion indicator (i.e. marker) may be detected in the antenna reflectance signal from the directional coupler, the indicator being a step change in the readings (this is specific to embodiments in which the antenna 17 and directional coupler 19 operate as a reflectometer from which readings are taken). The ingestion event timing is contemporaneous with the ingestion indicator in the antenna reflectance related readings. As a further example, the capsule may include a relative humidity sensor as a form of environmental sensor, wherein an ingestion indicator may be detected by processing readings from said relative humidity sensor. The indicator is the earliest (post-initiation event) rise of relative humidity to within a predefined threshold of 100%, for example, plus minus 5%, or plus minus 1%. A further ingestion indicator is a button press of an ingestion confirmation button on a user interface of a user device. Embodiments may combine one or more of the disclosed ingestion indicators to determine ingestion event timing. For example, more than one of the disclosed ingestion indicators being detected at timings within a predefined timing window of one another, for example, one minute of each other, results in determination of ingestion event timing.

An example for the excretion event: on a progressive (i.e. rolling) basis from a starting point being ICJ event (in chronological case) or a termination event (in reverse-chronological case), determine mean value of three adjacent environmental sensor readings, determine a timing at which the mean value either ceased to be or began to be within a threshold distance of the expected environmental value (i.e. within 1 degree centigrade of expected temperature or within 1, 2, 5, or 10% of expected humidity) pre-excretion, then determine that the excretion event timing is the during the three readings (for example, the mid-point, the earliest point, or the latest point). The number three is exemplary and different numbers for the number of samples in the rolling average could be selected, such as five, ten, twelve, or twenty. Furthermore, the tolerance of 1 degree centigrade is configurable and could be, for example, 2 degrees, 3 degrees etc. Excretion event may be confirmed or detected by accelerometer readings indicating a freefall event.

It is noted that, on occasion, there may be no change in temperature at ingestion or excretion. The processing may include a backup algorithm which is performed in the event the earliest environmental temperature readings at initiation (it being assumed that the capsule 10 has not yet been ingested) are within the threshold range of the expected temperature for the environment at the start of the GI tract of the subject mammal. The backup algorithm looks for other ingestion indicators or excretion indicators in recorded readings from other sensors (such as the accelerometer, and/or the other ingestion indicators or excretion indicators discussed above) that may indicate an excretion or ingestion event. Alternatively, in instances in which the environmental sensor 14 further comprises an environmental humidity sensor 14b, the relative humidity readings may be used as a fallback for temperature. A further example is a manual button press on a user interface of a device (such as the receiver apparatus 30). Embodiments may combine indicators in a hierarchical manner (i.e. look for indicator in temperature readings first, and look for indicators in readings from other sensors only if indicator in temperature readings cannot be found), or may treat indicators equally (i.e. look for any two contemporaneous indicators). Other algorithms for determining timing may be implemented, for example, a confidence level may be attributed to a detected indicator and then only if the confidence level does not satisfy a threshold are readings from other sensors processed to find a contemporaneous indicator to improve confidence. It is noted in this document that humidity refers to relative humidity.

A specific excretion indicator used to add confidence to an excretion indicator (start of decrease from body temperature) in environmental temperature readings is a button press on a bowel movement button on the user interface of the receiver apparatus, and a further excretion indicator is communication loss at the receiver. For example, communication loss at the timing (i.e. within a predefined timing window) of a recorded button press of a bowel movement button is an excretion indicator, which may be used instead of, or to add confidence to, an excretion indicator in the environmental temperature readings, in order to determine excretion event timing.

### Excretion Timing

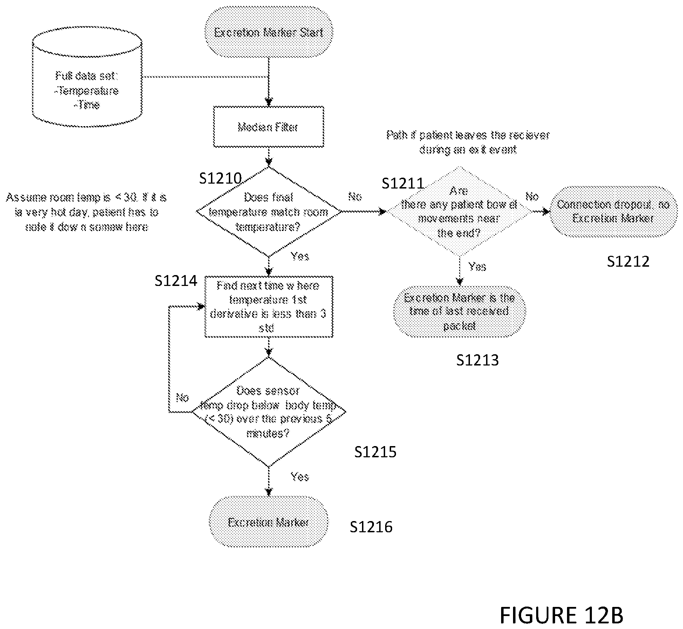

Figure 12b illustrates an exemplary algorithm for determining excretion timing. At S1210 a check is performed whether the temperature at timing of the termination event (which may be powering down of the capsule or may be a patient provided marker such as pushing of a button on a user interface on the receiver apparatus 30) the environmental temperature sensor reading is within a range for room temperature. If not at S1211 a check is performed for bowel movements at around the timing of the termination event. If not, then it is determined that excretion has not yet occurred and the termination event is determined to be a connection dropout rather than an excretion event at S1212. At S1213, if there have been bowel movements (wherein a bowel movement may be recorded by a patient for example by pushing a button on the user interface of the receiver apparatus) then the excretion event timing is timing of the last received data packet. At S1214, the algorithm identifies the next time where temperature 1^{st} derivative is less than 3 standard deviations, and at S1215 a check is performed on whether the environmental temperature sensor readings show a drop to below a predefined range for body temperature over the preceding 5 minutes. If not, the flow returns to S1214. If yes, the flow proceeds to S1216 and the timing of the temperature drop is the determined excretion timing.

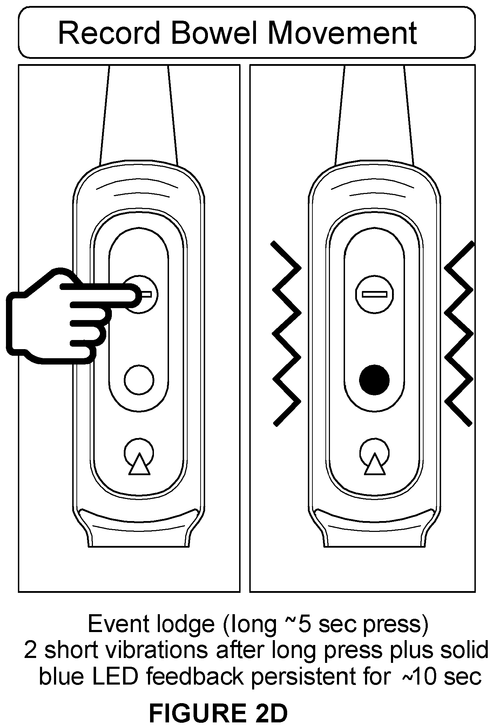

Figure 2D illustrates a user interface on a receiver apparatus 30 which comprises a button for recording a bowel movement event by a manual press of the button. In Figure 2D, an example of 5 seconds is provided as a length of time for which the button is manually depressed to record the bowel movement. An LED display provides feedback to the patient that the bowel movement event is recorded.

Layout of Figure 4 illustrates a chronological processing direction in which gastric duodenal event timing is determined first and used as an early bound (i.e. lower bound) for the earliest readings to be analysed in detecting the ileocecal junction indicator. Processing may be performed in a reverse chronological direction, as indicated by arrows in Figure 4, in which the ileocecal junction event timing is determined and used as a late bound (i.e. upper bound) for the readings to be analysed in detecting the gastric-duodenal transition indicator.

### Gastric Emptying

At S104a recorded readings later than the determined ingestion event timing are analysed for a gastric-duodenal transition indicator, or in the reverse chronological processing case readings earlier than the determined ileocecal junction transition event timing. So, recorded readings are analysed by iteratively or repetitively progressing in a respective processing direction until a timing of a determined first transition event.

The first transition event is gastric emptying or crossing the interface between the stomach and the duodenum. Gastric duodenal indicator or indicators may be detected in a first subset of recorded readings, the first subset being defined temporally as explained above. Furthermore, the first subset may be constrained by sensor, comprising readings from the TCD gas sensor 131. The first subset may further comprise antenna reflectance related readings (i.e. from the antenna 17 and directional coupler 171) and/or the accelerometer 19.

The gastric-duodenal transition indicator in the TCD gas sensor readings may be a, spike, step change or an inflection point in the TCD gas sensor readings. A correction may be applied to the TCD gas sensor readings to account for changes in environmental temperature, based on recorded readings from the environmental temperature sensor 14a. The correction may be applied at the detecting stage S104a so that the recorded readings themselves are corrected to account from changes in environmental temperature, and a gastric-duodenal transition indicator is detected in the corrected readings. Alternatively, the gastric-duodenal transition indicator may be detected in the raw readings (i.e. the uncorrected readings) and then at the determining step S104 a check performed to determine whether or not the indicator is attributable to a change in the environmental temperature or not, and if not, then it is either determined that the gastric-duodenal transition indicator is caused by a gastric-duodenal transition by the capsule 10, or a further condition is applied in the determination (for example, recorded readings from another sensor are checked for a contemporaneous indicator). Alternatively, the further condition may be a threshold or some other condition applied to the detected spike, step change, or inflection point itself.

The primary physical mechanism being sensed in the TCD gas sensor readings in detecting the gastric-duodenal transition indicator is as follows: Hydrochloric acid in the gastric juices leaving the stomach mixes with bicarbonate within the bile acids that is released by the pancreas. This bile acid works to neutralize the pH of the liquid and a byproduct of this reaction is CO2. In this area of the GI tract the surrounding gases are primarily N2 and O2 with some trace amounts of CO2. The amount of CO2 created in this reaction are significantly higher than the trace amounts that are around due to swallowing of exhaled breath. Therefore, simply using the TCD sensor output without calculating CO2 is appropriate. In other words, the TCD gas sensor readings, once corrected for environmental temperature variations, themselves provide the gastric-duodenal transition indicator, owing to a change in heat conductivity caused by variation in CO2 concentration across the two sides of the gastric-duodenal transition. For motility purposes (i.e. for determining the location of the ingestible capsule 10) there is no particular need to calculate the actual CO2 concentration.

As the TCD sensor 131 is affected by the temperature of the gas mixture at the location of the capsule, a temperature correction process is required to account for changes in the external environmental temperature changes i.e. drinking cold water, exercise, eating etc. Starting from the determined ingestion event timing, the first bump, step change or large inflection in the readings of the TCD gas sensor 131 plotted against time, that is not associated with an environmental temperature change, identifies the gastric-duodenal transition.

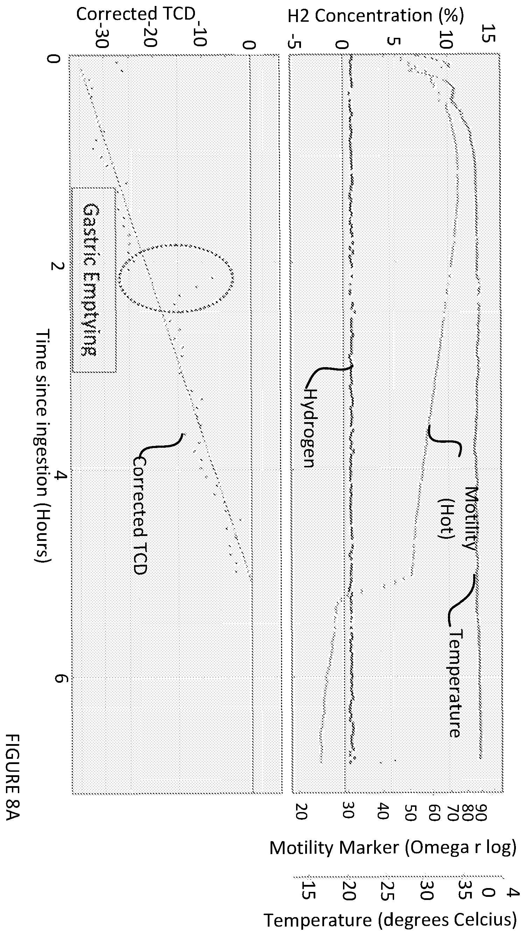

Figure 8a illustrates recorded readings of an environmental temperature sensor 14a (top line of readings on the top graph) against time, and corrected TCD gas sensor readings against time for an instance of capsule ingestion and progression through a GI tract. The gastric-duodenal transition indicator, which may be labelled gastric emptying, is indicated by a spike above a threshold height in the corrected TCD gas sensor readings. Spike height may be measured, for example, by distance (e.g. as a proportion, as an absolute value, or as a number of standard deviations) from a trend line fitted against the readings up to that point.

Gas concentrations are examples of metrics that may be calculated by the on-board processor or remote processing apparatus based on raw data obtained from the on-board sensors. For example, capsule 10 may be configured to calculate and translate one or more such metrics for transmission away from the capsule 10. Raw data may be discarded or stored for transmission away from the capsule 10. Optionally, metrics may be calculated by the on-board or off-board processor based on the calculated gas concentrations. An example of such a metric is peak H2, which may be finalised at timing of excretion event and transmitted away from the capsule 10 in data transmission payload from capsule 10 to receiver apparatus 30.

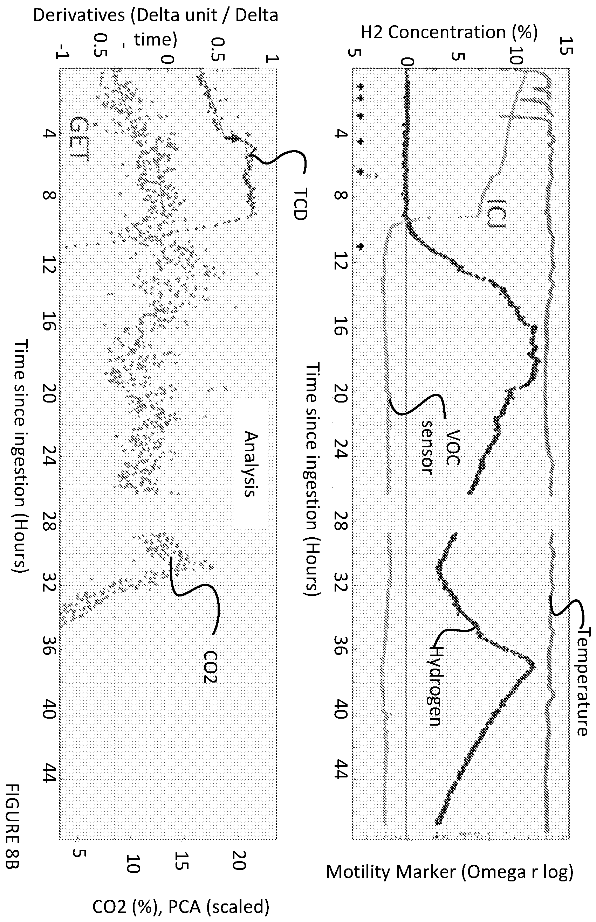

Figure 8B shows gastric emptying as visible in TCD sensor output and CO2 readings. CO2 is produced when the hydrochloric acid in the gastric juices leave the stomach and mix with bicarbonate in the bile acids released by the pancreas. This reaction also neutralizes the pH of the liquid. Embodiments use the temperature compensated raw TCD sensor output to detect this event, rather than the calculated CO2, since it contains much less noise. The TCD sensor output is adjusted to compensate for the temperature fluctuations measured by the environmental temperature sensor 14a. An algorithm is used to find the moment CO2 increases by removing drinking events and searching for a distinct discontinuity in the TCD output between ingestion and ICJ transition.

The processing may include detecting, as a first gastric-duodenal transition indicator, a gastric-duodenal transition indicator in the TCD gas sensor readings from the first subset of recorded readings; and calculating a confidence score representing a likelihood that the detected gastric-duodenal transition indicator in the TCD gas sensor readings is caused by the ingestible capsule 10 traversing the gastric-duodenal junction. The confidence score may be based, for example, on the height of the spike relative to the trend line, wherein more standard deviations above the trend line gives higher confidence level. A probability distribution lookup table may be utilised to transform spike height to confidence score. The confidence score may be a percentage likelihood of the spike in corrected TCD readings being caused by a first transition event rather than being caused by noise or other random variation in the corrected TCD readings.

The processing may include comparing the calculated confidence score with a threshold, and if the confidence score meets the threshold, determining that the first transition event has occurred and a timing thereof based on a timing of the detected gastric-duodenal transition indicator, and if the confidence score does not meet the threshold, assigning the detected gastric-duodenal transition indicator from the TCD gas sensor readings as a first gastric-duodenal transition indicator, and detecting whether or not a second gastric-duodenal transition indicator is present in readings from the first subset other than the TCD gas sensor readings and contemporaneous with the first gastric-duodenal transition indicator, and if the second gastric-duodenal transition indicator is detected, determining that the first transition event has occurred and a timing thereof based on a timing of the first gastric-duodenal transition indicator.

In effect, the first gastric-duodenal transition indicator not meeting the confidence score threshold initiates a further processing thread for detecting a further gastric-duodenal transition indicator to add confidence to the first. Recorded readings contemporaneous with the first gastric duodenal transition indicator from other sensors or pseudo sensors are analysed to identify one or more second gastric-duodenal transition indicators. The temporal bounds of the readings included in the analysis may be, for example, a predefined temporal distance either side of the first gastric duodenal transition indicator, for example, one second, five seconds, ten seconds, twenty seconds, thirty seconds, one minute, two minute, or five minutes. Recorded readings from either or both of the antenna reflectance related readings (i.e. the antenna 17 and directional coupler 171 configured as a reflectometer sensing whether and how the dielectric of the environment surrounding the capsule 10 changes) and the accelerometer 19 (i.e. sensing whether and how the capsule rate of orientation change varies) may be analysed in seeking to identify the one or more second gastric-duodenal transition indicators.

As illustrated in Figure 3A, the circuitry includes a directional coupler 171 in series with the antenna 17, which operate as a reflectometer. A diode detector measures the amplitude of reflected signals from the antenna. The measurements of the diode detector are exemplary of antenna reflectance related readings, and measure the reflected energy from the antenna, i.e. energy that was not radiated from the antenna 17 due to impedance mismatches. The reflectometer readings measure the antenna's radiation efficiency which is affected by the dielectric of the material surrounding the capsule. In another example, antenna reflectance related readings are generated by a feedback loop including a controller controlling a variable capacitor impedance matching the antenna with the surrounding environment. The control signal from the controller to the variable capacitor acts to impedance match the antenna with the surrounding environment and therefore is representative or correlated to electromagnetic properties of the environment surrounding the capsule).

The readings may become noisy and/or a baseline shift occurs at the timing of the gastric-duodenal transition event. For example, the increase in noise and/or the baseline shift are detectable as transition indicators.

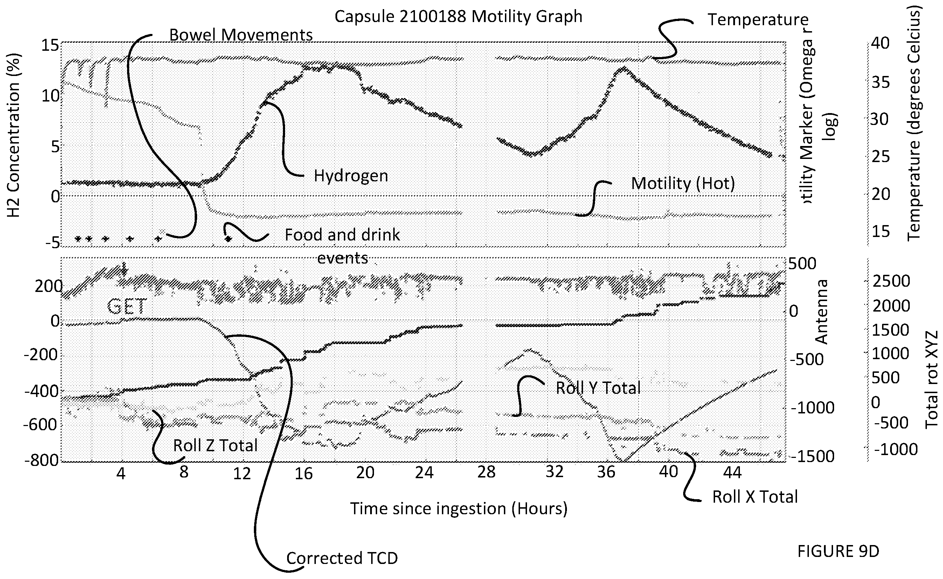

Figure 9D illustrates (on the uppermost plot on the lower of the two sets of axes) antenna reflectance related readings against time (labelled "Ant" for antenna), and is marked with the gastric emptying event. The antenna 17 and directional coupler 171 function as a reflectometer to measure the reflected energy from the antenna, i.e. energy that was not radiated out of the antenna. This signal varies as the surrounding dielectric properties change, most notably when the capsule leaves the cavernous fluid filled stomach and transitions to being surrounded by tubular tissue in the small intestine. A shift in the antenna reflectance related readings is observed to be coincident with the TCD marker, adding confidence, as a secondary measure.

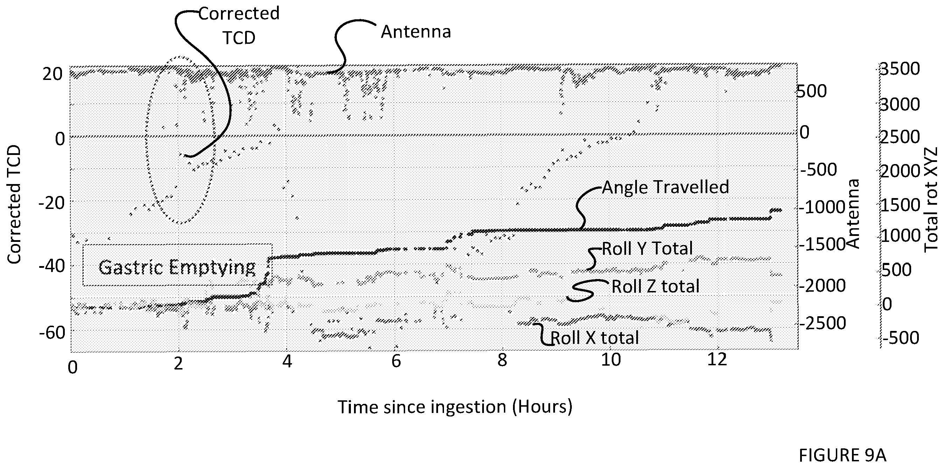

Figure 9A is a plot of recorded readings (or processed versions thereof) against time for a number of sensors and pseudo sensors in the capsule 10. A gastric emptying (gastric-duodenal transition) event is labelled. The top plot in the graph of Figure 9A is antenna reflectance related readings against time (labelled "Ant" for antenna). It can be seen that a baseline shift occurs at a time coincident with the spike in corrected TCD gas sensor readings. So, if, for example, a confidence score representing likelihood of the spike being caused by gastric-duodenal transition did not meet a threshold, then the antenna reflectance related readings are analysed to detect a baseline shift coincident with the spike. For example, a baseline shift may be detected by, on a progressive/rolling basis, comparing a mean value of a latest number (e.g. five, ten, or twenty) of consecutive readings, with a mean value of a number of readings preceding (or proceeding in the case of reverse chronological processing) the latest number of consecutive readings. A baseline shift may be indicated by a difference more than a threshold, wherein the threshold may be an absolute value, a proportion, or determined relative to a standard deviation in the readings. Detecting a coincidental gastric-duodenal indicator in the output of the reflectometer may be sufficient to confirm that the first gastric duodenal transition indicator is caused by gastric-duodenal transition of the capsule 10 and thus to determine the timing of the gastric-duodenal transition. Alternatively, the combination of the two indicators may be assessed via a probability model to revise the confidence score and compare the revised confidence score with a threshold, wherein meeting the threshold is to determine that the first gastric duodenal transition indicator is caused by gastric-duodenal transition of the capsule 10 and thus to determine the timing of the gastric-duodenal transition.

### Accelerometer and Processing of Accelerometer Data

An exemplary accelerometer 19 measures roll about three mutually orthogonal axes. The readings from the accelerometer 19 may be vectors with a component per axis, with each component indicating an instantaneous angular acceleration about the corresponding axis, or an average acceleration about the corresponding axis over the time period since the preceding live reading. Alternatively, the readings may give a three dimensional orientation of the capsule. At the receiver apparatus 30 or at the remote computer 20, processing of the readings from the accelerometer may be performed to generate a representation (such as a plot vs time) of aggregated (i.e. all three axes) accelerometer readings from which a marker (i.e. a gastro-duodenal transition indicator) is identifiable. Such a plot or representation may also be used to identify markers for other events including excretion event. In Figure 9A, an "angle travelled" plot is generated. It is an accumulation of scalar angular displacement about all three axes cumulatively over time, wherein a low pass filter is applied to filter out small angular displacements.

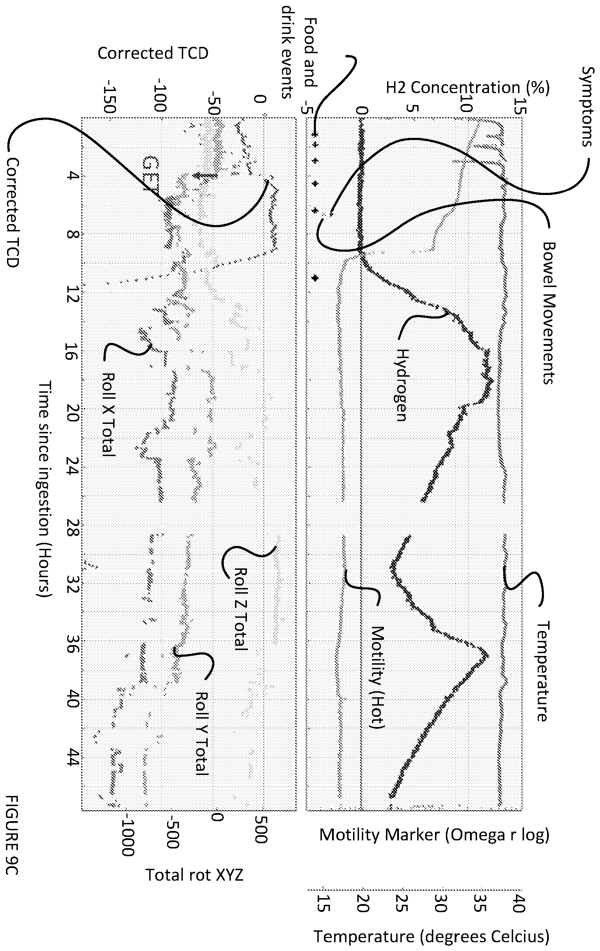

Figure 9C shows roll in each of three mutually orthogonal dimensions and is marked with gastric emptying event, from which it can be seen that the change in accelerometer readings correlates temporally with the change in corrected TCD readings (i.e. can be used to add confidence to a detection of gastric-duodenal transition indicator in the temperature corrected TCD readings). The capsule orientation is measured using a triaxial accelerometer and tracking the gravity vector with respect the capsule frame of reference. The capsule orientation is measured using a triaxial accelerometer and tracking the gravity vector with respect the capsule frame of reference. When the capsule leaves the stomach it tends to experience rapid changes in its orientation as it transits through the duodenum and small intestine. "Angle Travelled", simply accumulates the orientation change in excess of a 90 degree hysteresis angle. This algorithm tends to be robust to small changes in orientation experienced in the stomach and avoids some of the complexities of other approaches.

A first technique for processing accelerometer data may be referred to as angle travelled. Angle travelled uses vector mathematics to calculate the angle between the gravity vector and a temporary vector. The temporary vector is pulled in the direction of the change in angle, only when this angle exceeds a given threshold (currently 90 Deg). It is then the accumulation of the change in the temporary vector that is visualized in the representation from which markers are identifiable. What is generally seen is that this measure does not change much in the stomach since the angle between the gravity and temporary vectors rarely exceed the threshold in any one direction, (small back and forth orientation changes in the stomach are effectively ignored by the inherent hysteresis of this algorithm) and that once in the tortuous lumen of the small intestine, this measure accumulates significantly due to the larger, more continuous orientation changes of the capsule. Thus, a step change in the cumulative angle travelled measure is a gastric-duodenal transition indicator.

In an exemplary implementation of angle travelled: the accelerometer readings may provide a reading of an orientation of the ingestible capsule relative to a frame of reference in fixed relation to a gravitational vector. Processing of the readings from the accelerometer may comprise recording an orientation of the ingestible capsule given by a first accelerometer reading as a reference orientation, and repetitively in respect of each successive accelerometer reading chronologically: determining whether the orientation of the ingestible capsule given by the respective accelerometer reading is more than a threshold angular displacement from the reference orientation, and if the threshold angular displacement is not met, progressing to the next accelerometer reading without changing the reference orientation, and if the threshold angular displacement is met, changing the reference orientation to align with the orientation of the ingestible capsule given by the respective accelerometer reading. An indicator, such as the gastric-duodenal transition indicator, may be a step change in the rate of change of the reference orientation.

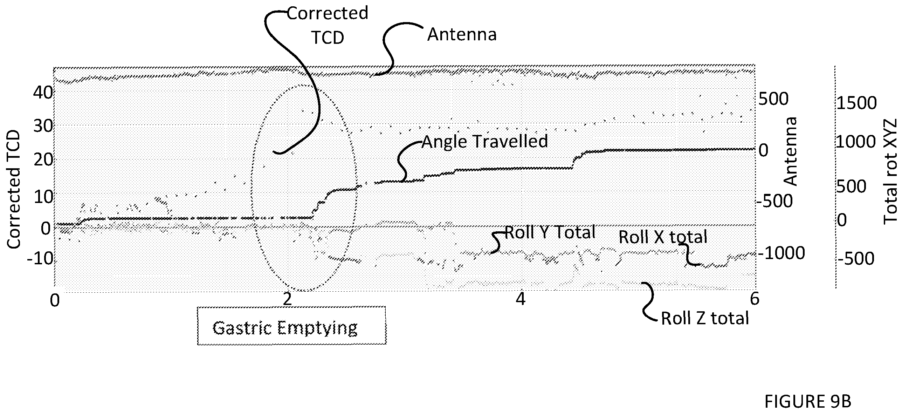

Figure 9B indicates that a step change in a plot of angle travelled is identifiable within a threshold time period of the detected spike in the TCD gas sensor readings. Therefore, the step change in the plot of angle travelled increases confidence in the hypothesis that the detected spike in the TCD gas sensor readings is caused by gastric-duodenal transition. There are two approximately contemporaneous gastric-duodenal transition indicators, which enables the timing of one of the indicators (which one may be pre-selected, for example, the TCD gas sensor readings) to be determined as the timing of the transition event.

A second technique for processing accelerometer data may be referred to as total roll. Total roll calculates the angle between the gravity vector and each of the capsule X, Y and Z axes and expresses this as a continuous measure that can accumulate beyond 360 Deg. For example, if the capsule x axis is at an angle of 350 Deg and rotates by a further 20 Deg, the resulting angle is expressed as 370 Deg rather than 10 Deg. This helps when representing the readings as a plot from which markers are identified since it avoids the sudden angle changes associated with crossing the zero line. In the example a real change of 20 Deg would be visualized instead of an artificial change of 340 Deg. In addition to this basic approach, low pass filtering may be applied to filter the raw data to remove sensor noise. Additionally, angles are only calculated when the raw accelerometer data provide sufficient data to calculate a meaningful angle. An example of where this is not the case is when the two accelerometer axis values used to calculate the orientation angle around the third axis both approach zero. In this case the calculation will be dominated by sensor noise and so a meaningful angle cannot be determined.

The accelerometer readings provide a reading of an orientation of the ingestible capsule relative to a frame of reference in fixed relation to a gravitational vector. Exemplary processing of the readings from the accelerometer may comprise for each of three orthogonal axes in fixed spatial relation to the ingestible capsule derivable from the reading of the orientation, repetitively in respect of each successive accelerometer reading chronologically: calculating, as a scalar value, a change in the orthogonal axis relative to the gravitational vector from the preceding accelerometer reading; applying a low pass filter to the calculated changes; recording the cumulative filtered calculated changes. A marker serving as a gastric-duodenal transition indicator may be, for example, an increase (such as a spike or step change) in the rate of increase in the cumulative filtered calculated changes.

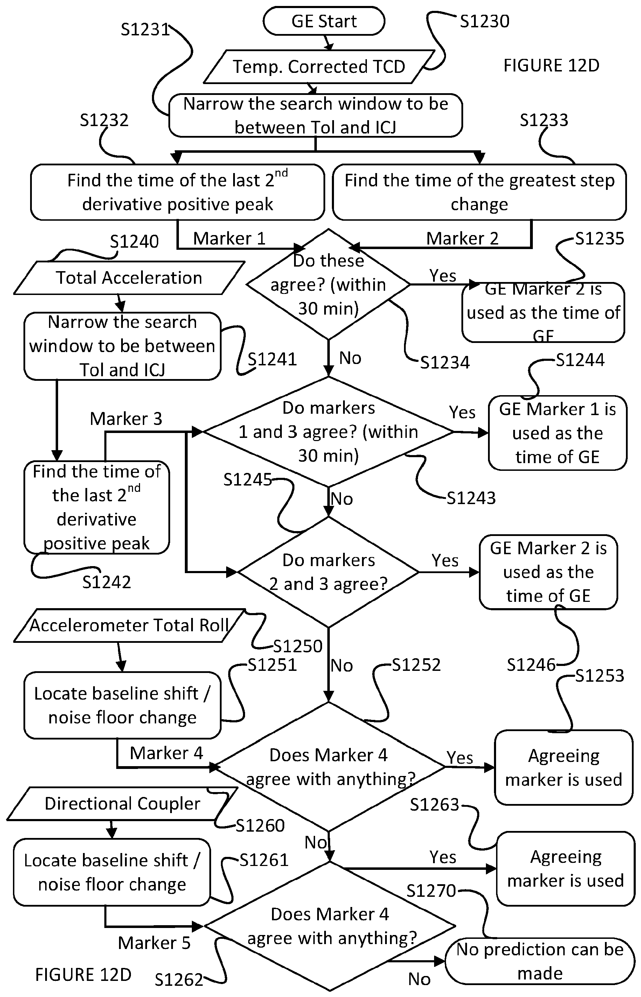

Figure 12D illustrates an exemplary algorithm for determining timing of gastric emptying (i.e. gastric duodenal transition event timing). At S1230 the temperature corrected TCD readings are obtained and at S1231 the search window narrowed by using the determined ICJ event timing as a late bound (in the reverse chronological processing example). Two indicators are detected in the readings: at S1232 the timing of the latest positive peak in the second derivative of the temperature corrected TCD readings is detected as marker 1; and at S1233 the timing of the greatest step change in the readings is detected as marker 2. At S1234 the timing of the two indicators is compared, and if they are within a predefined threshold distance such as 30 minutes of one another then at S1235 the timing of marker 2 is determined to be the timing of the gastric-duodenal transition of the capsule.

If the two do not agree, then accelerometer data is processed. At S1240 the angle travelled data is obtained and at S1241 the search window narrowed as in S1231. At S1242 marker 3 is detected as the timing of the latest positive peak in the second derivative of the angle travelled data. At S1243 a check is performed on whether timing of markers 1 and 3 agree to within a predefined threshold distance such as 30 minutes of one another, and if they do, the flow proceeds to S1244 and the timing of marker 1 is determined to be the timing of the gastric-duodenal transition of the capsule. If markers 1 and 3 do not agree then the flow proceeds to S1245 and a check is performed on whether timing of markers 2 and 3 agree to within a predefined threshold distance such as 30 minutes of one another. If yes, the flow proceeds to S1246 and the timing of marker 2 is determined to be the timing of the gastric-duodenal transition of the capsule. If not, then the total roll data is obtained from the accelerometer readings at S1250. At S1251 a gastric duodenal transition indicator is detected as marker 4 by detecting a baseline shift or noise floor change in the accelerometer total roll data. At S1252 timing of marker 4 is compared with timing of markers 1, 2, and 3. If timing of marker 4 is within a predefined threshold distance such as 30 minutes of any of the other markers then the timing of the other marker is determined to be the timing of the gastric-duodenal transition of the capsule at S1253. If marker 4 agrees with >1 of markers 1, 2, 3, then, for example, the timing of the marker from among 1, 2, and 3 most closely matching that of marker 4 may be determined to be the timing of the gastric-duodenal transition of the capsule. Otherwise, a predetermined hierarchy may be programmed into the algorithm so that, for example, marker 1 takes precedence over marker 2, which in turn takes precedence over marker 3.

If no agreement is found at S1252, then the reflectometer readings are obtained at S1260 (marked "Directional Coupler" owing to the directional coupler 171 in series with the antenna 17 forming the reflectometer). At S1261 processing is performed to detect, as marker 5, a timing of a baseline shift or noise floor change in the reflectometer readings. At S1262 timing of marker 5 is compared with timing of markers 1, 2, 3, and 4. If timing of marker 5 is within a predefined threshold distance such as 30 minutes of any of the other markers then the timing of the other marker is determined to be the timing of the gastric-duodenal transition of the capsule at S1253. If marker 5 agrees with >1 of markers 1, 2, 3, 4, then, for example, the timing of the marker from among 1, 2, 3, and 4 most closely matching that of marker 4 may be determined to be the timing of the gastric-duodenal transition of the capsule. Otherwise, a predetermined hierarchy may be programmed into the algorithm so that, for example, marker 1 takes precedence over marker 2, which in turn takes precedence over marker 3, which in turn takes precedence over marker 4.

In the case of chronological processing, at S106a recorded readings later than the determined first transition event timing are analysed for an ileocecal junction indicator. Or in the case of reverse-chronological processing, readings earlier than the determined excretion event timing are analysed for an ileocecal junction indicator.. So, recorded readings from the respective determined event timing and later or earlier are analysed by iteratively or repetitively progressing the readings being analysed until a timing of a determined second transition event.

### Determining Ileocecal Junction Transition Timing

The second transition event is passage of the capsule 10 through the ileocecal junction. Ileocecal junction indicator or indicators may be detected in a second subset of recorded readings, the second subset being defined temporally as explained above dependent upon chronological or reverse chronological processing. Furthermore, the second subset may be constrained by sensor, comprising readings from the sensor side of the VOC gas sensor 132a.

The ileocecal junction indicator in the VOC gas sensor readings may be a spike, step change or an inflection point in the VOC gas sensor readings.

The determining second transition event timing S106 is an application of one or more conditions to the detected ileocecal junction indicator to determine whether or not it can be attributed to (i.e. to predict to within a predefined confidence level) passage of the capsule 10 across the ileocecal junction.

The transit prediction of the transition from small intestine to large intestine is the determined second transition event timing. The gas environment change between the small and large intestine is significant due to the large intestine's bacterial population occurring in significantly higher prevalence, driving the creation, or increase, in volatiles and a reduction on O2 through fermentation of carbohydrates and proteins by the microbiota.

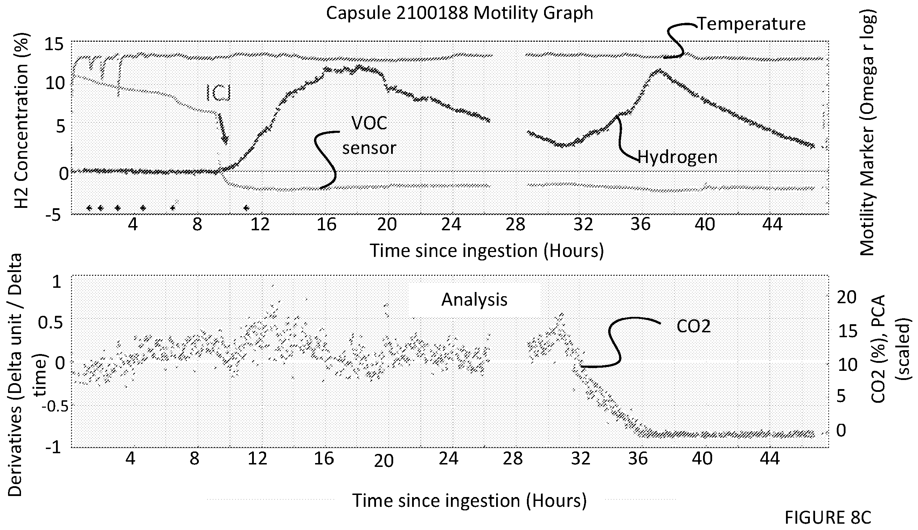

The VOC gas sensor output 132 from the sensor side 132a is sensitive to many different volatile analytes with the largest response being due to H2, and O2. At the time of transition through the ileocecal valve a large reduction on the VOC sensor is observed. As the capsule transits the GI tract the environment is increasingly anaerobic as the O2 is consumed by bacteria. Figure 8C illustrates indicators of ICJ on plots of VOC sensor output and determined H2 concentration. The indicator in the VOC sensor output may be identified at S106a through plotting the differential of the VOC sensor side readings vs time whilst the sensor is heated and finding the tallest negative peak. This differential locates the point of greatest change which is associated with the transition but does not occur at the start of the transition event. The start of the transition event may be found by the initial inflection point from the baseline in the first derivative. Thus, the indicator may detected by the tallest negative peak, and the event timing determined by the inflection point. The tallest negative peak may be found retrospectively by analyzing VOC gas sensor readings from a predefined temporal period (e.g. one hour, two hours, four hours etc) following determined gastric-duodenal transition event timing, or preceding the determined excretion event timing (in the case of reverse-chronological processing). Alternatively, a threshold negative peak size may be determined, with the first peak exceeding the threshold size being detected as the ileocecal junction transition indicator.

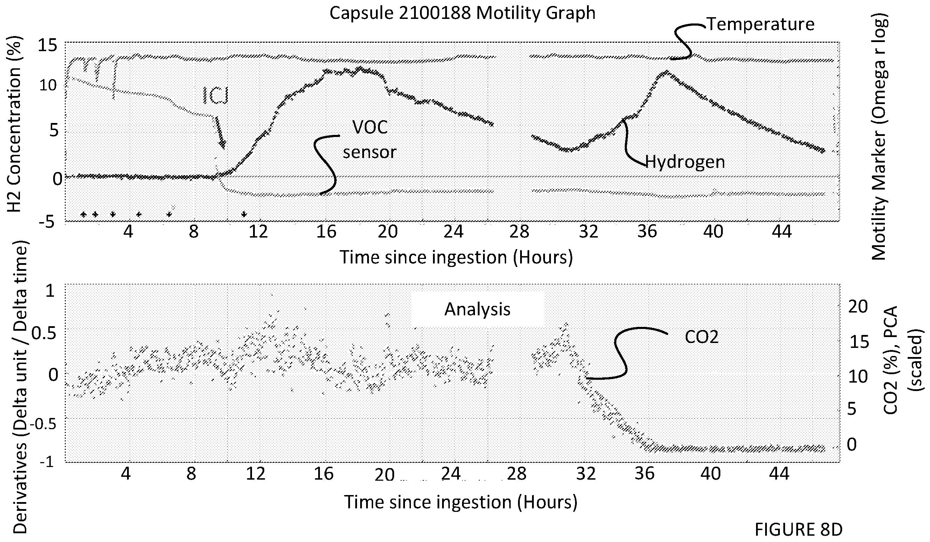

As illustrated in Figure 8D, an ICJ indicator is also present in the determined H2 concentration percentage, as a sharp increase in H2 when the capsule reaches the colon. The H2 produced in the GI tract is a byproduct of fermentation. The colonies of bacteria are orders of magnitude larger in the colon than in the small bowel. Therefore, determined H2 concentration may be used to add confidence to the ileocecal junction indicator in the VOC sensor output.

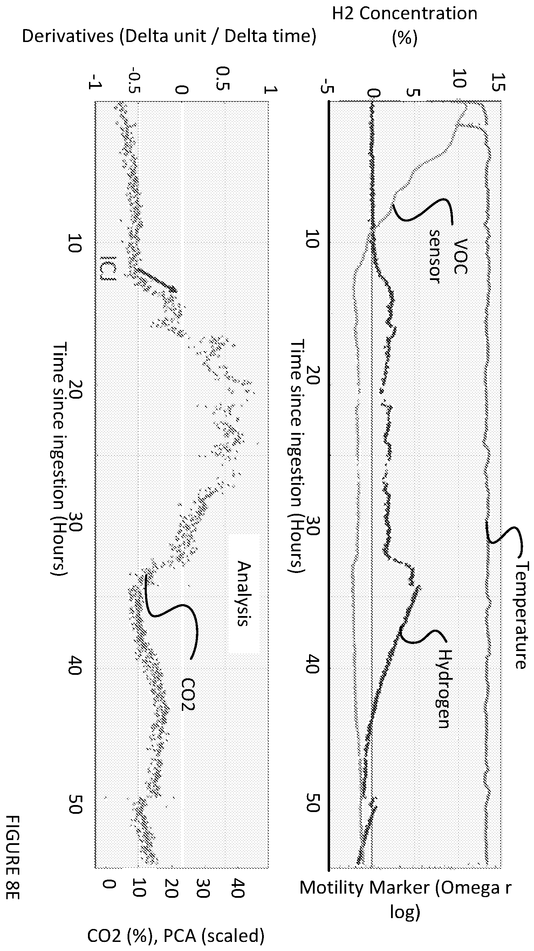

Figure 8E illustrates a further fallback marker for ileocecal junction transition in the form of the detected CO2 concentration. CO2 in the GI tract is produced as a byproduct of fermentation. The colonies of bacteria are orders of magnitude larger in the colon than in the small bowel. Therefore, determined CO2 concentration may be used to add confidence to the ileocecal junction indicator in the VOC sensor output.

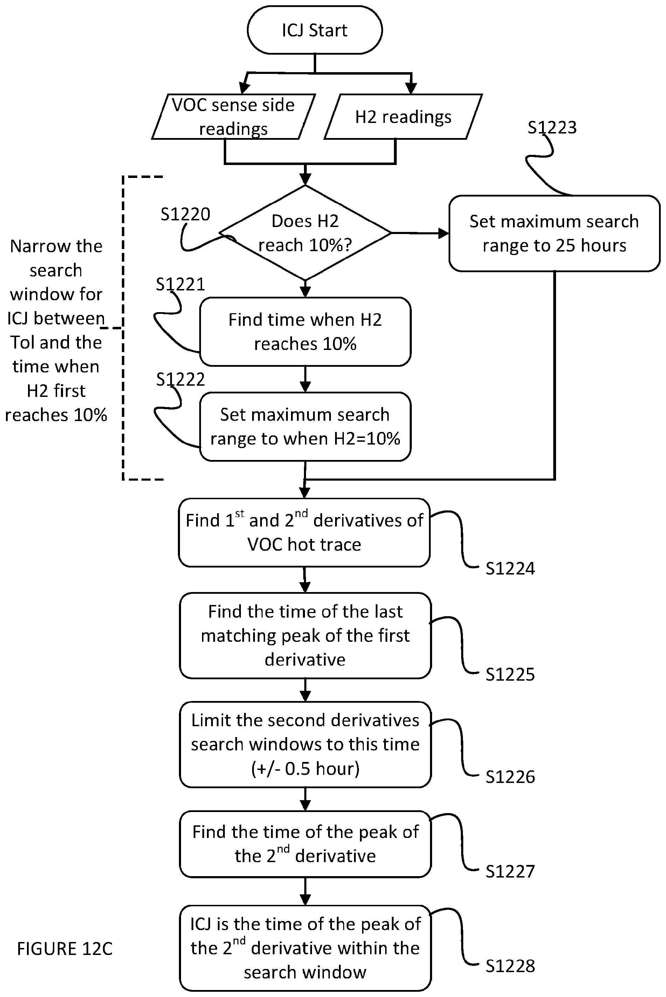

Figure 12C illustrates an exemplary algorithm for processing readings to determine ileocecal junction transition event timing. The input is the readings from the VOC sensor sense side and H2 readings (determined by preprocessing output of VOC sensor heater side). Steps S1220 to S1223 narrow the search window for ICJ indicators/markers to between the determined time of the transition event and when the determined concentration of H2 in the gas mixture detected by the capsule gas sensors first reaches 10%. At S1224 the 1^{st} and second derivatives of the VOC hot trace (being the hottest point of the VOC sensor sense side from a pulsed drive signal) are found and at S1225 the timing of the last matching peak of the first derivative (i.e. the largest negative peak from the first derivative) is found. At S1226 the timing of the last matching peak of the first derivative is used to delimit a search window for the second derivatives to 30 minutes either side of the last matching peak time. At S1227 the timing of the peak of the 2^{nd} derivative of the VOC hot trace is found, and at S1228 the ileocecal junction transition event timing is determined to be the timing of said 2^{nd} derivative peak.At S107a the recorded readings from the environmental sensor 14 are analysed to detect a change in an environmental condition (being one or both of environmental temperature and environmental relative humidity) that would indicate an excretion event. In the case of chronological processing the analysis is of environmental sensor readings from the determined timing of the second transition event and later. Once it is determined that a detected change in the environmental temperature is caused by an excretion event, no further analysis to detect an excretion event is performed. In the case of reverse chronological processing, the analysis at S107a is of readings from the termination event and earlier. For example, detecting the change at S107a may be on a rolling basis by comparing one or more readings with a predetermined number of preceding or processing readings, with a difference of more than a threshold (i.e. one or two degrees centigrade) being a detected change. The detected change may be a change in temperature, a change in relative humidity, or both, depending on geographic location and climactic considerations. Determining the excretion event timing S 107 may include comparing the temperature of one or more readings with an expected temperature for the environment at the end of the GI tract of the subject mammal 40, wherein a change of more than a threshold higher (in the case of reverse chronological processing) or lower (in the case of chronological processing) is a determination that the capsule 10 has been excreted. Alternatively the condition may be that a predefined number or more consecutive readings are outside of a threshold of the expected temperature for the environment at the end of the GI tract of the subject mammal.

### Determining Excretion Event Timing

In general the temperature drop at excretion is a reliable signal. However, there are cases when the temperature drop is not observed in the data. Bowel movement button press such as illustrated in Figure 2D may be used as a fallback, or further sensor readings may be processed as a fallback. Determining the excretion event timing S 107 may include comparing the relative humidity of one or more readings with an expected relative humidity for the environment at the end of the GI tract of the subject mammal 40, wherein a change of more than a threshold higher (in the case of reverse chronological processing) or lower (in the case of chronological processing) is a determination that the capsule 10 has been excreted. Alternatively the condition may be that a predefined number or more consecutive readings are outside of a threshold of the expected relative humidity for the environment at the end of the GI tract of the subject mammal.

It is noted that, on occasion, there may be no change in temperature at excretion. The processing may include a backup algorithm which is performed in the event the earliest environmental temperature readings at initiation (it being assumed that the capsule 10 has not yet been ingested) are within the threshold range of the expected temperature for the environment at the end of the GI tract of the subject mammal (which would be an indication that the subject is in an environment with a temperature at or around the expected GI tract temperature). The backup algorithm looks for markers in recorded readings from other sensors that may indicate an excretion event. Since excretion is generally associated with a physical fall, the marker may be an indicator in the accelerometer readings. Alternatively or additionally, a change in relative humidity may be detected by the backup algorithm.

### Gastric Emptying

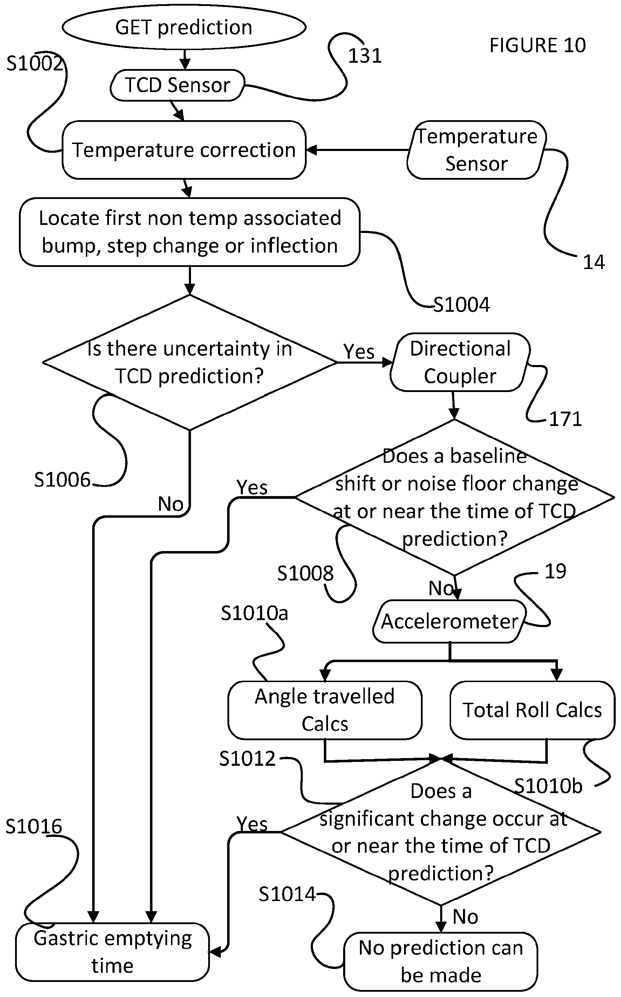

Figure 10 is a flowchart of a method, apparatus, and processing, for determining a gastric-duodenal transition event timing. It is noted that the gastric-duodenal transition event may be referred to as gastric emptying. Determining an event timing may also be referred to as predicting timing of the event.

At S1002, recorded readings from the environmental temperature sensor 14a and the TCD gas sensor 131 are combined to correct (i.e. to moderate or calibrate) the TCD gas sensor readings to correct for variations in temperature at the location of the capsule 10. The effect of temperature variations at the location of the capsule 10 is thereby compensated in corrected TCD gas sensor readings, from which a first gastric-duodenal indicator may be identified at S1004, which is exemplary of S104a discussed above. Assuming the processing steps are being performed whilst the capsule 10 is in the subject mammal, the recorded readings are monitored continuously for a gastric-duodenal transition indicator. For example, monitoring for a gastric-duodenal transition indicator may commence once an ingestion event timing has been determined. Should the processing be applied retrospectively, then the recorded readings are processed in a rolling manner from the ingestion event timing forward in seeking to identify a gastric-duodenal transition indicator. The gastric-duodenal transition indicator may be a bump, step change, or inflection, in the plot of corrected TCD gas sensor readings vs time. A detection algorithm may apply low pass filtering to prevent bump, step change, or inflection points below respective magnitude thresholds being detected as gastric-duodenal transition indicators.

Alternatively, as mentioned above, retrospective reverse-chronological processing may be performed to detect the first gastric-duodenal indicator at S1004.

At S1006, as discussed above, a confidence score is calculated for the detected indicator. For example, the hypothesis is that the detected indicator (in the corrected recorded TCD gas sensor readings) was caused by the gastric-duodenal transition event of the capsule 10, the null hypothesis being that the detected indicator was a random variation in readings caused by noise or by physical effects within the stomach. The confidence score may be generated by a probability distribution (e.g normal distribution by knowing standard distribution of TCD gas sensor readings when within the stomach) and knowing the size of the bump in standard deviations. Similarly, magnitude of step change or inflection point vs standard deviations can be used to generate a probability score from a probability distribution. A threshold minimum confidence score may be applied at S 1006, and if the calculated confidence score meets the threshold (e.g. 0.9, 0.92, 0.95, or 0.99), the flow proceeds to S 1016 and the timing of the detected transition indicator is determined to be the timing of the gastric-duodenal transition event.

If the calculated confidence score does not meet the threshold, then a second gastric-duodenal transition indicator is sought. Alternatively, it may be that plural transition indicators are detected at S 1004, for example, a first non-temperature associated bump, step-change, or inflection point is detected as a first transition indicator, and further non-temperature associated bumps, step-changes, or inflection points, are detected as alternative first transition indicators. Wherein the alternative first transition indicators are at different respective timings than the first transition indicator, and so a determination must be made of which is caused by the gastric-duodenal transition event of the capsule, and which are not. Noting that the change in the TCD gas sensor readings at gastric-duodenal transition are caused by an increase in CO2 in the gas mixture surrounding the capsule. Embodiments may process TCD gas sensor readings in raw form to detect the first transition indicator and alternative first transition indicators, or embodiments may process a preprocessed version of the TCD gas sensor readings preprocessed to represent CO2 concentration in the gas mixture, for example, based on lookups to calibration tables stored at the device executing the detecting. For example, if none of the first transition indicator or the alternative first transition indicators (the first transition indicator being the earliest of the indicators, the alternative transition indicators being later and thus being based on the assumption that the first transition indicator is false i.e. not caused by gastric-duodenal transition event) satisfies a confidence threshold, readings from one or more other sensors may be processed to detect indicators contemporaneous with either the first transition indicator or an alternative first transition indicator, wherein a contemporaneous indicator (i.e. a second gastric-duodenal transition indicator) in readings from another sensor adds confidence to the respective transition detector. The another sensor may be, for example, the accelerometer, wherein an increase in rate of orientation change is the second transition indicator. Alternatively, the another sensor may be the directional coupler (i.e. the reflectometer), wherein a step change in the antenna reflectance readings is the second indicator. High variance in the antenna reflectance signal (i.e. the reflectometer readings or variable capacitor control signal readings) is associated with presence in the intestines, therefore a step change or spike is an indicator that the capsule has undergone the gastric-duodenal transition. As a further example, the another sensor may be the temperature sensor, wherein large temperature changes are associated with presence in the stomach, whereas temperature in the small bowel is much more consistent. Therefore, cessation of large temperature changes may be considered a second gastric-duodenal transition indicator. Using the same logic, rapid temperature change at the timing of a first transition indicator may reduce confidence in said transition indicator. As a metric to represent temperature change, a quantity such as average rate of change in a rolling time window, for example, 30 seconds, or 1 minute, may be used to represent amount of temperature change. So that the average rate of change in the preceding time window being below a threshold is an indicator that the gastric-duodenal transition has occurred and thus adds confidence to contemporaneous or recent first transition indicators.

In Figure 10 a hierarchical procedure is illustrated in which the antenna reflectance related readings (i.e. the antenna 17 and directional coupler 171 configured as a reflectometer or the control signal of the variable capacitor 172) are analysed first for a second gastric-duodenal transition indicator, and then the recorded readings from the accelerometer 19 are analysed if no second gastric-duodenal indicator is identifiable in the recorded readings from the reflectometer. However, methods may be reversed, so that the accelerometer readings are analysed first, and then the recorded readings from the reflectometer are analysed if no second gastric-duodenal indicator is identifiable in the recorded readings from the accelerometer 19.

At S1008, recorded readings from the reflectometer (composed of the antenna 17 and directional coupler 171) within a predefined temporal range of the detected first gastric-duodenal transition indicator are analysed to detect or identify a second gastric-duodenal transition indicator. Specifically, the second gastric duodenal transition indicator in the recorded readings from the reflectometer may be a baseline shift and/or a noise floor change. If either or both are detected, then the first gastric-duodenal indicator detected in the recorded readings of the TCD gas sensor is confirmed as being caused by the capsule 10 undergoing a gastric-duodenal transition event at S1016.

At S1010a and S1010b, the readings of the accelerometer 19 within a predefined temporal range of the detected first gastric-duodenal transition indicator are analysed to detect or identify a second gastric-duodenal transition indicator. Methods may utilise one or both of step S1010a and S1010b. At S1010a, the angle travelled representation (detailed above) of the recorded readings of the accelerometer readings is analysed to detect or identify a second gastric-duodenal transition indicator. Specifically the second gastric-duodenal transition indicator may be a step change in the cumulative angle travelled. At S1010b, the total roll representation (detailed above) of the recorded readings of the accelerometer readings is analysed to detect or identify a second gastric-duodenal transition indicator. Specifically the second gastric-duodenal transition indicator may be a step change in the rate of increase of total roll.

At S1012 a check is performed for whether a second gastric-duodenal transition indicator is detected in the accelerometer readings. If not, then the flow proceeds to S1014 and the detected first gastric-duodenal indicator is not determined to be caused by gastric-duodenal transition event of the capsule (though it may be flagged for manual checking in the sensor outputs). Monitoring of the TCD gas sensor readings continues from the timing of the detected first gastric-duodenal transition indicator onward to detect a new first gastric-duodenal transition indicator at S 1004, or in the case of reverse chronological processing from the timing of the detected first gastric-duodenal transition indicator backward.

At S 1016 a label may be applied to the recorded readings at the determined timing of the gastric-duodenal transition event to add context to readings from, for example, the gas sensors in further analysis by medical professionals. Alternatively or additionally, an alert or notification may be generated and transmitted to a recipient notifying the timing of the gastric-duodenal transition event.

It will be appreciated by persons skilled in the art that numerous variations and/or modifications may be made to the above-described embodiments, without departing from the broad general scope of the present disclosure. The present embodiments are, therefore, to be considered in all respects as illustrative and not restrictive.

## Claims

1. Apparatus for determining a location of an ingestible capsule within a gastrointestinal, GI, tract of a subject mammal, comprising:
an ingestible capsule (10), being ingestible by a subject mammal (40), the ingestible capsule (10) comprising a housing, a power source (16), a TCD gas sensor (131), and a VOC gas sensor (132);
means to record the readings of the ingestible capsule as a function of time including a period during which the ingestible capsule (10) is within the gastrointestinal, GI, tract of the subject mammal (40), the readings including TCD gas sensor readings and VOC gas sensor readings, the said means being the ingestible capsule (10) or a receiver apparatus (30) configured to receive, at a location external to the subject mammal (40), readings from the ingestible capsule (10) at a location internal to the subject mammal (40);
the apparatus being **characterised by** a processing apparatus, the processing apparatus being configured to process the recorded readings by a process including:
determining (S104) a first transition event timing, the first transition event timing being a timing of a gastric-duodenal transition by the ingestible capsule (10), wherein determining the first transition event timing comprises detecting a gastric-duodenal transition indicator in a first subset of the recorded readings, the first subset including the recorded TCD gas sensor readings; and
determining (S106) a second transition event timing, the second transition event timing being a timing of a transition across an ileocecal junction by the ingestible capsule (10), wherein determining the second transition event timing comprises detecting an ileocecal junction indicator in a second subset of the recorded readings, the second subset including the VOC gas sensor readings.

2. The apparatus of claim 1, wherein the ingestible capsule (10) includes an environmental temperature sensor (14), and the readings include environmental temperature sensor readings;
and wherein either:
determining (S104) the first transition event timing further comprises comparing one or more of the environmental temperature sensor readings at or around a timing of a detected gastric-duodenal transition indicator with a baseline environmental temperature value, and based on a result of the comparison, determining whether or not the timing of the detected gastric-duodenal transition indicator is associated with the first transition event timing; or
detecting the gastric-duodenal transition indicator includes moderating the TCD gas sensor readings according to the respective contemporaneous environmental temperature sensor readings, and detecting the gastric-duodenal transition indicator in the moderated TCD gas sensor readings.

3. The apparatus of claim 2, wherein
the gastric-duodenal transition indicator is a spike, step change, or inflection, in the TCD gas sensor readings from the recorded readings, and wherein it is determined that the timing of the gastric-duodenal transition indicator is associated with the first transition event timing if the environmental temperature value was within a predefined threshold distance of a baseline environmental temperature value for a predetermined period preceding the timing of the gastric-duodenal transition indicator.

4. The apparatus of any of the preceding claims, wherein
the ingestible capsule (10) further comprises a primary transceiver (18) comprising an antenna (17), a directional coupler (171) in series with the antenna to form a reflectometer, and a diode detector forming part of the reflectometer;
the ingestible capsule (10) being configured to transmit, by the antenna (17), the readings of the ingestible capsule (10) to the receiver apparatus (30);
the diode detector being configured to receive the reflected signal from the antenna (17) via the directional coupler (171) and to measure an amplitude of the reflected signal;
wherein the recorded readings of the gas sensor capsule comprise one or more antenna reflectance related readings from among:
readings of the amplitude of the reflected signal measured by the diode detector;
readings of the phase information of the reflected signal extracted by the quadrature demodulator; and
the antenna impedance control signal;
and wherein the first subset of recorded readings, from which the first transition event timing is determined, comprises the antenna reflectance related readings, and/or the second subset of recorded readings, from which the second transition event timing is determined, comprises the antenna reflectance related readings.

5. The apparatus of claim 4,
wherein the first subset includes the antenna reflectance related readings, and determining (S104) the first transition event timing further comprises:
detecting a gastric-duodenal transition indicator in the antenna reflectance related readings from the first subset; and
determining that the first transition event has occurred and a timing thereof based on the gastric-duodenal transition indicator detected in the antenna reflectance related readings from the first subset;
and wherein the process further comprises:
detecting, as a first a gastric-duodenal transition indicator, a gastric-duodenal transition indicator in the TCD gas sensor readings from the first subset; and
detecting, as a second gastric-duodenal transition indicator, a gastric-duodenal transition indicator in the antenna reflectance related readings from the first subset within a predefined temporal range of the detected first gastric-duodenal transition indicator; and
based on detecting the first and second gastric-duodenal transition indicators, determining that the first transition event has occurred and a timing thereof.

6. The apparatus of any of the preceding claims, wherein the ingestible capsule (10) further comprises an accelerometer (10), and the readings of the ingestible capsule include accelerometer readings, the first subset including accelerometer readings.

7. The apparatus of claim 6, wherein determining (S104) the first transition event timing further comprises:
detecting a gastric-duodenal transition indicator in the accelerometer readings from the first subset; and
determining (S104) that the first transition event has occurred and a timing thereof based on the gastric-duodenal transition indicator detected in the accelerometer readings from the first subset.

8. The apparatus of claim 7, the process further comprising:
detecting, as a first gastric-duodenal junction indicator, a gastric-duodenal junction indicator in the TCD gas sensor readings from the first subset; and
detecting, as a second gastric-duodenal transition indicator, a gastric-duodenal junction indicator in the accelerometer readings from the first subset within a predefined temporal range of the detected first gastric-duodenal transition indicator; and
based on detecting the first and second gastric-duodenal transition indicators, determining that the first transition event has occurred and a timing thereof.

9. The apparatus of any of claims 6 to 8, wherein the second subset includes the antenna reflectance related readings, and determining the second transition event timing further comprises:
detecting an ileocecal junction transition indicator in the antenna reflectance related readings from the second subset; and
determining that the second transition event has occurred and a timing thereof based on the ileocecal junction transition indicator detected in the antenna reflectance related readings from the first subset.

10. The apparatus of claim 9, wherein
the ileocecal junction transition indicator comprises an increase in sensor side VOC gas sensor readings with a contemporaneous increase in H2 concentration, the H2 concentration being derived from TCD readings of the TCD gas sensor and/or heater side readings of the VOC gas sensor; or wherein
the ileocecal junction transition indicator comprises an increase in sensor side VOC gas sensor readings with a contemporaneous increase in CH4 concentration, the CH4 concentration being derived from TCD readings of the TCD gas sensor and/or heater side readings of the VOC gas sensor.

11. The apparatus of any of the preceding claims, wherein
the ingestible capsule (10) includes an environmental sensor (14), and the readings include readings of the environmental sensor (14), the environmental sensor (14) being an environmental temperature sensor, an environmental relative humidity sensor, or an environmental temperature sensor and an environmental humidity sensor;
the process further comprising determining (S107) an excretion event timing by detecting an excretion indicator, the excretion indicator being a change in the environmental sensor readings between an internal environmental condition of the subject mammal (40) and an external environmental condition at a location of the subject mammal, the excretion event timing being a timing of excretion of the ingestible capsule by the subject mammal.

12. The apparatus of any of the preceding claims, wherein
the ingestible capsule (10) includes an environmental sensor (14), and the readings include readings of the environmental sensor (14), the environmental sensor (14) being an environmental temperature sensor, an environmental relative humidity sensor, or an environmental temperature sensor and an environmental humidity sensor;
the process further comprising determining (S103) an ingestion event timing by detecting an ingestion indicator, the ingestion indicator being a change in the environmental sensor readings between an internal environmental condition of the subject mammal and an external environmental condition at a location of the subject mammal, the ingestion event timing being a timing of ingestion of the ingestible capsule by the subject mammal.

13. The apparatus of any of the preceding claims, wherein the ingestible capsule (10) further comprises a secondary transceiver (21), the secondary transceiver (21) being operable in a listening phase of the ingestible capsule during which the primary transceiver (19), sensors, and on-board processor of ingestible capsule are powered down, the secondary transceiver (21) being configured during the listening mode to receive an encoded activation control signal from an encoded activation control signal transmitting device, and to respond by ending the listening phase and initiating a live phase of the ingestible capsule during which the primary transceiver (19), sensors, and on-board processor and memory, are powered-on and the readings are being recorded and optionally transmitted to a receiver apparatus (30);
wherein the secondary transceiver (21) is an NFC transceiver and wherein the encoded activation control signal is an NFC signal;
and wherein the receiver apparatus (30) is the encoded activation control signal transmitting device, and wherein the receiver apparatus (30) is a smartphone or tablet computer running an application causing the smartphone or tablet computer to generate and transmit the encoded activation control signal and to receive, and optionally to process and transmit, the recorded readings transmitted away from the capsule by the primary transceiver (19).

14. A computer program for determining a location of an ingestible capsule (10) within a gastrointestinal, GI, tract of a subject mammal, the ingestible capsule (10) being ingestible by a subject mammal (40) and comprising a housing, a power source (16), a TCD gas sensor (131), and a VOC gas sensor (132);
the computer program being executable by a computing apparatus comprising a processor and a memory, and upon execution to cause the computing apparatus to perform a process comprising:
accessing recorded readings of the ingestible capsule (10) as a function of time including a period during which the ingestible capsule (10) is within the GI tract of the subject mammal (40), the readings including TCD gas sensor readings, and VOC gas sensor readings,
the computer program being **characterised by** the process further comprising processing the recorded readings, including:
determining (S104) a first transition event timing, the first transition event timing being a timing of a gastric-duodenal transition by the ingestible capsule (10), wherein determining the first transition event timing comprises detecting a gastric-duodenal transition indicator in a first subset of the recorded readings, the first subset including the recorded TCD gas sensor readings; and
determining (S106) a second transition event timing, the second transition event timing being a timing of a transition across an ileocecal junction by the ingestible capsule (10), wherein determining the second transition event timing comprises detecting an ileocecal junction indicator in a second subset of the recorded readings, the second subset including the VOC gas sensor readings.

## Patentansprüche

1. Vorrichtung zur Bestimmung einer Position einer einnehmbaren Kapsel innerhalb eines Magen-Darm-, GI-, Trakts eines betroffenen Säugetiers, umfassend:
eine einnehmbare Kapsel (10), die von einem betroffenen Säugetier (40) eingenommen werden kann, wobei die einnehmbare Kapsel (10) ein Gehäuse, eine Stromquelle (16), einen TCD-Gassensor (131) und einen VOC-Gassensor (132) umfasst;
Mittel zum Aufzeichnen der Messwerte der einnehmbaren Kapsel als Funktion von der Zeit, die einen Zeitraum beinhaltet, während dem sich die einnehmbare Kapsel (10) innerhalb des Magen-Darm-, GI-, Trakts des betroffenen Säugetiers (40) befindet, wobei die Messwerte TCD-Gassensor-Messwerte und VOC-Gassensor-Messwerte beinhalten, wobei die Mittel die einnehmbare Kapsel (10) oder eine Empfängervorrichtung (30), die dazu konfiguriert ist, an einer Position außerhalb des betroffenen Säugetiers (40) Messwerte von der einnehmbaren Kapsel (10) an einer Position innerhalb des betroffenen Säugetiers (40) zu empfangen, sind;
wobei die Vorrichtung durch eine Verarbeitungsvorrichtung gekennzeichnet ist, wobei die Verarbeitungsvorrichtung dazu konfiguriert ist, die aufgezeichneten Messwerte durch einen Vorgang zu verarbeiten, der Folgendes beinhaltet:
Bestimmen (S104) eines Zeitpunkts eines ersten Übergangsereignisses, wobei der Zeitpunkt des ersten Übergangsereignisses ein Zeitpunkt eines Übergangs vom Magen zum Zwölffingerdarm durch die einnehmbare Kapsel (10) ist, wobei das Bestimmen des Zeitpunkts des ersten Übergangsereignisses das Erkennen eines Indikators für den Übergang vom Magen zum Zwölffingerdarm in einer ersten Teilmenge der aufgezeichneten Messwerte umfasst, wobei die erste Teilmenge die aufgezeichneten TCD-Gassensor-Messwerte beinhaltet; und
Bestimmen (S106) eines Zeitpunkts eines zweiten Übergangsereignisses, wobei der Zeitpunkt des zweiten Übergangsereignisses ein Zeitpunkt eines Übergangs durch die Ileozökalklappe durch die einnehmbare Kapsel (10) ist, wobei das Bestimmen des Zeitpunkts des zweiten Übergangsereignisses das Erkennen eines Indikators für die Ileozökalklappe in einer zweiten Teilmenge der aufgezeichneten Messwerte umfasst, wobei die zweite Teilmenge die VOC-Gassensor-Messwerte beinhaltet.

2. Vorrichtung nach Anspruch 1, wobei die einnehmbare Kapsel (10) einen Umgebungstemperatursensor (14) beinhaltet und die Messwerte Umgebungstemperatursensor-Messwerte beinhalten;
und wobei entweder:
das Bestimmen (S104) des Zeitpunkts des ersten Übergangsereignisses ferner das Vergleichen eines oder mehrerer der Umgebungstemperatursensor-Messwerte zu oder in der Nähe eines Zeitpunkts eines erkannten Indikators für den Übergang vom Magen zum Zwölffingerdarm mit einem Ausgangswert der Umgebungstemperatur und basierend auf einem Ergebnis des Vergleichs das Bestimmen, ob der Zeitpunkt des erkannten Indikators für den Übergang vom Magen zum Zwölffingerdarm mit dem Zeitpunkt des ersten Übergangsereignisses in Verbindung steht oder nicht, umfasst; oder
das Erkennen des Indikators für den Übergang vom Magen zum Zwölffingerdarm das Bereinigen der TCD-Gassensor-Messwerte gemäß den jeweiligen zeitgleichen Umgebungstemperatursensor-Messwerten und das Erkennen des Indikators für den Übergang vom Magen zum Zwölffingerdarm in den bereinigten TCD-Gassensor-Messwerten beinhaltet.

3. Vorrichtung nach Anspruch 2, wobei
der Indikator für den Übergang vom Magen zum Zwölffingerdarm eine Spitze, eine sprunghafte Änderung oder ein Wendepunkt in den TCD-Gassensor-Messwerten aus den aufgezeichneten Messwerten ist und wobei bestimmt wird, dass der Zeitpunkt des Indikators für den Übergang vom Magen zum Zwölffingerdarm mit dem Zeitpunkt des ersten Übergangsereignisses in Verbindung steht, wenn der Umgebungstemperaturwert, für einen vorbestimmten Zeitraum vor dem Zeitpunkt des Indikators für den Übergang vom Magen zum Zwölffingerdarm, innerhalb eines vordefinierten Schwellenabstands von einem Ausgangswert der Umgebungstemperatur lag.

4. Vorrichtung nach einem der vorhergehenden Ansprüche, wobei
die einnehmbare Kapsel (10) ferner einen Primär-Sendeempfänger (18) umfasst, der eine Antenne (17), einen Richtkoppler (171) in Reihe mit der Antenne, um ein Reflektometer zu bilden, und einen Diodendetektor, der Teil des Reflektometers ist, umfasst;
wobei die einnehmbare Kapsel (10) dazu konfiguriert ist, die Messwerte der einnehmbaren Kapsel (10) durch die Antenne (17) an die Empfängervorrichtung (30) zu übertragen;
wobei der Diodendetektor dazu konfiguriert ist, das reflektierte Signal von der Antenne (17) über den Richtkoppler (171) zu empfangen und eine Amplitude des reflektierten Signals zu messen;
wobei die aufgezeichneten Messwerte der Gassensorkapsel einen oder mehrere Messwerte im Zusammenhang mit dem Antennenreflektonsgrad umfassen, und zwar aus:
Messwerten der Amplitude des reflektierten Signals, die von dem Diodendetektor gemessen wird;
Messwerten der Phaseninformationen des reflektierten Signals, die von dem Quadratur-Demodulator extrahiert werden; und
dem Antennenimpedanz-Steuersignal;
und wobei die erste Teilmenge der aufgezeichneten Messwerte, aus der der Zeitpunkt des ersten Übergangsereignisses bestimmt wird, die Messwerte im Zusammenhang mit dem Antennenreflexionsgrad umfasst, und/oder die zweite Teilmenge der aufgezeichneten Messwerte, aus der der Zeitpunkt des zweiten Übergangsereignisses bestimmt wird, die Messwerte im Zusammenhang mit dem Antennenreflexionsgrad umfasst.

5. Vorrichtung nach Anspruch 4,
wobei die erste Teilmenge die Messwerte im Zusammenhang mit dem Antennenreflektionsgrad beinhaltet und das Bestimmen (S104) des Zeitpunkts des ersten Übergangsereignisses ferner Folgendes umfasst:
Erkennen eines Indikators für den Übergang vom Magen zum Zwölffingerdarm in den Messwerten im Zusammenhang mit dem Antennenreflektionsgrad aus der ersten Teilmenge; und
Bestimmen, dass das erste Übergangsereignis stattgefunden hat, und eines Zeitpunkts davon basierend auf dem Indikator für den Übergang vom Magen zum Zwölffingerdarm, der in den Messwerten im Zusammenhang mit dem Antennenreflektionsgrad aus der ersten Teilmenge erkannt wurde;
und wobei der Vorgang ferner Folgendes umfasst:
Erkennen, als einen ersten Indikator für den Übergang vom Magen zum Zwölffingerdarm, eines Indikators für den Übergang vom Magen zum Zwölffingerdarm in den TCD-Gassensor-Messwerten aus der ersten Teilmenge; und
Erkennen, als einen zweiten Indikator für den Übergang vom Magen zum Zwölffingerdarm, eines Indikators für den Übergang vom Magen zum Zwölffingerdarm in den Messwerten im Zusammenhang mit dem Antennenreflektionsgrad aus der ersten Teilmenge innerhalb eines vordefinierten Zeitbereichs des erkannten ersten Indikators für den Übergang vom Magen zum Zwölffingerdarm; und
basierend auf dem Erkennen des ersten und des zweiten Indikators für den Übergang vom Magen zum Zwölffingerdarm Bestimmen, dass das erste Übergangsereignis stattgefunden hat, und eines Zeitpunkts davon.

6. Vorrichtung nach einem der vorhergehenden Ansprüche, wobei die einnehmbare Kapsel (10) ferner einen Beschleunigungsmesser (10) umfasst und die Messwerte der einnehmbaren Kapsel Beschleunigungsmesser-Messwerte beinhalten, wobei die erste Teilmenge Beschleunigungsmesser-Messwerte beinhaltet.

7. Vorrichtung nach Anspruch 6, wobei das Bestimmen (S104) des Zeitpunkts des ersten Übergangsereignisses ferner Folgendes umfasst:
Erkennen eines Indikators für den Übergang vom Magen zum Zwölffingerdarm in den Beschleunigungsmesser-Messwerten aus der ersten Teilmenge; und
Bestimmen (S104), dass das erste Übergangsereignis stattgefunden hat, und eines Zeitpunkts davon basierend auf dem Indikator für den Übergang vom Magen zum Zwölffingerdarm, der in den Beschleunigungsmesser-Messwerten aus der ersten Teilmenge erkannt wurde.

8. Vorrichtung nach Anspruch 7, wobei der Vorgang ferner Folgendes umfasst:
Erkennen, als einen ersten Indikator für die Magen-Zwölffingerdarm-Verbindung, eines Indikators für die Magen-Zwölffingerdarm-Verbindung in den TCD-Gassensor-Messwerten aus der ersten Teilmenge; und
Erkennen, als einen zweiten Indikator für den Übergang vom Magen zum Zwölffingerdarm, eines Indikators für die Magen-Zwölffingerdarm-Verbindung in den Beschleunigungsmesser-Messwerten aus der ersten Teilmenge innerhalb eines vordefinierten Zeitbereichs des erkannten ersten Indikators für den Übergang vom Magen zum Zwölffingerdarm; und
basierend auf dem Erkennen des ersten und des zweiten Indikators für den Übergang vom Magen zum Zwölffingerdarm Bestimmen, dass das erste Übergangsereignis stattgefunden hat, und eines Zeitpunkts davon.

9. Vorrichtung nach einem der Ansprüche 6 bis 8, wobei die zweite Teilmenge die Messwerte im Zusammenhang mit dem Antennenreflektionsgrad beinhaltet und das Bestimmen des Zeitpunkts des zweiten Übergangsereignisses ferner Folgendes umfasst:
Erkennen eines Indikators für den Übergang an der Ileozökalklappe in den Messwerten im Zusammenhang mit dem Antennenreflektionsgrad aus der zweiten Teilmenge; und
Bestimmen, dass das zweite Übergangsereignis stattgefunden hat, und eines Zeitpunkts davon basierend auf dem Indikator für den Übergang an der Ileozökalklappe, der in den Messwerten im Zusammenhang mit dem Antennenreflektionsgrad aus der ersten Teilmenge erkannt wurde.

10. Vorrichtung nach Anspruch 9, wobei
der Indikator für den Übergang an der Ileozökalklappe einen Anstieg der sensorseitigen VOC-Gassensor-Messwerte mit einem zeitgleichen Anstieg der H2-Konzentration umfasst, wobei die H2-Konzentration aus den TCD-Messwerten des TCD-Gassensors und/oder den heizseitigen Messwerten des VOC-Gassensors abgeleitet wird; oder wobei
der Indikator für den Übergang an der Ileozökalklappe einen Anstieg der sensorseitigen VOC-Gassensor-Messwerte mit einem zeitgleichen Anstieg der CH4-Konzentration umfasst, wobei die CH4-Konzentration aus den TCD-Messwerten des TCD-Gassensors und/oder den heizseitigen Messwerten des VOC-Gassensors abgeleitet wird.

11. Vorrichtung nach einem der vorhergehenden Ansprüche, wobei
die einnehmbare Kapsel (10) einen Umgebungssensor (14) beinhaltet und die Messwerte Messwerte des Umgebungssensors (14) beinhalten, wobei der Umgebungssensor (14) ein Umgebungstemperatursensor, ein Sensor für die relative Luftfeuchtigkeit der Umgebung oder ein Umgebungstemperatursensor und ein Sensor für die Luftfeuchtigkeit der Umgebung ist;
der Vorgang ferner das Bestimmen (S107) eines Zeitpunkts eines Ausscheidungsereignisses durch Erkennen eines Indikators für die Ausscheidung umfasst, wobei der Indikator für die Ausscheidung eine Änderung der Umgebungssensor-Messwerte zwischen einer inneren Umgebungsbedingung des betroffenen Säugetiers (40) und einer äußeren Umgebungsbedingung an einer Position des betroffenen Säugetiers ist, wobei der Zeitpunkt des Ausscheidungsereignisses ein Zeitpunkt der Ausscheidung der einnehmbaren Kapsel durch das betroffene Säugetier ist.

12. Vorrichtung nach einem der vorhergehenden Ansprüche, wobei
die einnehmbare Kapsel (10) einen Umgebungssensor (14) beinhaltet und die Messwerte Messwerte des Umgebungssensors (14) beinhalten, wobei der Umgebungssensor (14) ein Umgebungstemperatursensor, ein Sensor für die relative Luftfeuchtigkeit der Umgebung oder ein Umgebungstemperatursensor und ein Sensor für die Luftfeuchtigkeit der Umgebung ist;
der Vorgang ferner das Bestimmen (S103) eines Zeitpunkts eines Einnahmeereignisses durch das Erkennen eines Indikators für die Einnahme umfasst, wobei der Indikator für die Einnahme eine Änderung der Umgebungssensor-Messwerte zwischen einer inneren Umgebungsbedingung des betroffenen Säugetiers und einer äußeren Umgebungsbedingung an einer Position des betroffenen Säugetiers ist, wobei der Zeitpunkt des Einnahmeereignisses ein Zeitpunkt der Einnahme der einnehmbaren Kapsel durch das betroffene Säugetier ist.

13. Vorrichtung nach einem der vorhergehenden Ansprüche, wobei die einnehmbare Kapsel (10) ferner einen Sekundär-Sendeempfänger (21) umfasst, wobei der Sekundär-Sendeempfänger (21) in einer Abhörphase der einnehmbaren Kapsel betreibbar ist, während der der Primär-Sendeempfänger (19), die Sensoren und der interne Prozessor der einnehmbaren Kapsel ausgeschaltet sind, wobei der Sekundär-Sendeempfänger (21) während des Abhörmodus dazu konfiguriert ist, ein codiertes Aktivierungssteuersignal von einem Gerät zum Übertragen eines codierten Aktivierungssteuersignals zu empfangen und darauf zu reagieren, indem die Abhörphase beendet und eine Live-Phase der einnehmbaren Kapsel eingeleitet wird, während der der Primär-Sendeempfänger (19), die Sensoren und der interne Prozessor und Speicher eingeschaltet sind und die Messwerte aufgezeichnet und optional an eine Empfängervorrichtung (30) übertragen werden;
wobei der Sekundär-Sendeempfänger (21) ein NFC-Sendeempfänger ist und wobei das codierte Aktivierungssteuersignal ein NFC-Signal ist;
und wobei die Empfängervorrichtung (30) das Gerät zum Übertragen des codierten Aktivierungssteuersignals ist und wobei die Empfängervorrichtung (30) ein Smartphone oder ein Tablet-Computer ist, auf dem eine Anwendung läuft, die bewirkt, dass das Smartphone oder der Tablet-Computer das codierte Aktivierungssteuersignal erzeugt und überträgt und die von der Kapsel weg durch den Primär-Sendeempfänger (19) übertragenen aufgezeichneten Messwerte empfängt und optional verarbeitet und überträgt.

14. Computerprogramm zur Bestimmung einer Position einer einnehmbaren Kapsel (10) innerhalb eines Magen-Darm-, GI-, Trakts eines betroffenen Säugetiers, wobei die einnehmbare Kapsel (10) voneinem betroffenen Säugetier (40) eingenommen werden kann und ein Gehäuse, eine Stromquelle (16), einen TCD-Gassensor (131) und einen VOC-Gassensor (132) umfasst;
wobei das Computerprogramm von einer Rechenvorrichtung ausgeführt werden kann, die einen Prozessor und einen Speicher umfasst, und bei Ausführung bewirkt, dass die Rechenvorrichtung einen Vorgang durchführt, der Folgendes umfasst:
Zugeifen auf aufgezeichnete Messwerte der einnehmbaren Kapsel (10) als Funktion von der Zeit, die einen Zeitraum beinhaltet, während dem sich die einnehmbare Kapsel (10) innerhalb des GI-Trakts des betroffenen Säugetiers (40) befindet, wobei die Messwerte TCD-Gassensor-Messwerte und VOC-Gassensor-Messwerte beinhalten,
wobei das Computerprogramm durch den Vorgang gekennzeichnet ist, das ferner das Verarbeiten der aufgezeichneten Messwerte umfasst, einschließlich:
Bestimmen (S104) eines Zeitpunkts eines ersten Übergangsereignisses, wobei der Zeitpunkt des ersten Übergangsereignisses ein Zeitpunkt eines Übergangs vom Magen zum Zwölffingerdarm durch die einnehmbare Kapsel (10) ist, wobei das Bestimmen des Zeitpunkts des ersten Übergangsereignisses das Erkennen eines Indikators für den Übergang vom Magen zum Zwölffingerdarm in einer ersten Teilmenge der aufgezeichneten Messwerte umfasst, wobei die erste Teilmenge die aufgezeichneten TCD-Gassensor-Messwerte beinhaltet; und
Bestimmen (S106) eines Zeitpunkts eines zweiten Übergangsereignisses, wobei der Zeitpunkt des zweiten Übergangsereignisses ein Zeitpunkt eines Übergangs durch die Ileozökalklappe durch die einnehmbare Kapsel (10) ist, wobei das Bestimmen des Zeitpunkts des zweiten Übergangsereignisses das Erkennen eines Indikators für die Ileozökalklappe in einer zweiten Teilmenge der aufgezeichneten Messwerte umfasst, wobei die zweite Teilmenge die VOC-Gassensor-Messwerte beinhaltet.

## Revendications

1. Appareil pour déterminer un emplacement d'une capsule ingérable dans un tractus gastro-intestinal, GI, d'un mammifère sujet, comprenant :
une capsule ingérable (10), qui est ingérable par un mammifère sujet (40), la capsule ingérable (10) comprenant un boîtier, une source d'alimentation (16), un capteur de gaz TCD (131) et un capteur de gaz COV (132) ;
des moyens pour enregistrer les lectures de la capsule ingérable en fonction du temps, y compris une période pendant laquelle la capsule ingérable (10) se trouve dans le tractus gastro-intestinal, GI, du mammifère sujet (40), les lectures comprenant des lectures de capteur de gaz TCD et des lectures de capteur de gaz COV, lesdits moyens étant la capsule ingérable (10) ou un appareil récepteur (30) configuré pour recevoir, en un emplacement externe au mammifère sujet (40), des lectures de la capsule ingérable (10) en un emplacement interne au mammifère sujet (40) ;
l'appareil étant **caractérisé par** un appareil de traitement, l'appareil de traitement étant configuré pour traiter les lectures enregistrées par un procédé comprenant :
la détermination (S104) d'une première synchronisation d'événement de transition, la première synchronisation d'événement de transition étant une synchronisation d'une transition gastrique-duodénale par la capsule ingérable (10), ladite détermination de la première synchronisation d'événement de transition comprenant la détection d'un indicateur de transition gastrique-duodénale dans un premier sous-ensemble des lectures enregistrées, le premier sous-ensemble comprenant les lectures de capteur de gaz TCD enregistrées ; et
la détermination (S106) d'une seconde synchronisation d'événement de transition, la seconde synchronisation d'événement de transition étant une synchronisation d'une transition à travers une jonction iléocécale par la capsule ingérable (10), ladite détermination de la seconde synchronisation d'événement de transition comprenant la détection d'un indicateur de jonction iléocécale dans un second sous-ensemble des lectures enregistrées, le second sous-ensemble comprenant les lectures du capteur de gaz COV.

2. Appareil selon la revendication 1, ladite capsule ingérable (10) comprenant un capteur de température environnementale (14), et lesdites lectures comprenant des lectures de capteur de température environnementale ;
et soit :
ladite détermination (S104) de la première synchronisation d'événement de transition comprenant en outre la comparaison d'une ou plusieurs des lectures de capteur de température environnementale au niveau ou autour d'une synchronisation d'un indicateur de transition gastrique-duodénale détecté à une valeur de température environnementale de base, et sur la base d'un résultat de la comparaison, ladite détermination pour savoir si la synchronisation de l'indicateur de transition gastrique-duodénale détecté est associée ou non à la première synchronisation d'événement de transition ; soit
ladite détection de l'indicateur de transition gastrique-duodénale comprenant la modération des lectures du capteur de gaz TCD en fonction des lectures simultanées respectives du capteur de température environnementale, et la détection de l'indicateur de transition gastrique-duodénale dans les lectures modérées du capteur de gaz TCD.

3. Appareil selon la revendication 2,
ledit indicateur de transition gastrique-duodénale étant une pointe, un changement de pas ou une inflexion, dans les lectures de capteur de gaz TCD à partir des lectures enregistrées, et étant déterminé que la synchronisation de l'indicateur de transition gastrique-duodénale est associée à la première synchronisation d'événement de transition si la valeur de température environnementale se trouvait dans la limite d'une distance seuil prédéfinie d'une valeur de température environnementale de base pendant une période prédéfinie précédant la synchronisation de l'indicateur de transition gastrique-duodénale.

4. Appareil selon l'une quelconque des revendications précédentes,
ladite capsule ingérable (10) comprenant en outre un émetteur-récepteur primaire (18) comprenant une antenne (17), un coupleur directionnel (171) en série avec l'antenne pour former un réflectomètre, et un détecteur à diode faisant partie du réflectomètre ;
ladite capsule ingérable (10) étant configurée pour transmettre, au moyen de l'antenne (17), les lectures de la capsule ingérable (10) à l'appareil récepteur (30) ;
ledit détecteur à diode étant configuré pour recevoir le signal réfléchi de l'antenne (17) via le coupleur directionnel (171) et pour mesurer une amplitude du signal réfléchi ;
lesdites lectures enregistrées de la capsule de capteur de gaz comprenant une ou plusieurs lectures liées à la réflectance de l'antenne parmi :
des lectures de l'amplitude du signal réfléchi mesuré par le détecteur à diode ;
des lectures des informations de phase du signal réfléchi extrait par le démodulateur en quadrature ; et
le signal de commande d'impédance d'antenne ;
et ledit premier sous-ensemble de lectures enregistrées, à partir duquel la première synchronisation d'événement de transition est déterminée, comprenant les lectures liées à la réflectance de l'antenne, et/ou ledit second sous-ensemble de lectures enregistrées, à partir duquel la seconde synchronisation d'événement de transition est déterminée, comprenant les lectures liées à la réflectance de l'antenne.

5. Appareil selon la revendication 4,
ledit premier sous-ensemble comprenant les lectures liées à la réflectance de l'antenne, et ladite détermination (S104) de la première synchronisation d'événement de transition comprenant en outre :
la détection d'un indicateur de transition gastrique-duodénale dans les lectures liées à la réflectance de l'antenne à partir du premier sous-ensemble ; et
la détermination que le premier événement de transition s'est produit et une synchronisation de celui-ci sur la base de l'indicateur de transition gastrique-duodénale détecté dans les lectures liées à la réflectance de l'antenne à partir du premier sous-ensemble ;
et ledit procédé comprenant en outre :
la détection, en tant que premier indicateur de transition gastrique-duodénale, d'un indicateur de transition gastrique-duodénale dans les lectures de capteur de gaz TCD à partir du premier sous-ensemble ; et
la détection, en tant que second indicateur de transition gastrique-duodénale, d'un indicateur de transition gastrique-duodénale dans les lectures liées à la réflectance de l'antenne à partir du premier sous-ensemble dans une plage temporelle prédéfinie du premier indicateur de transition gastrique-duodénale détecté ; et
sur la base de la détection des premier et second indicateurs de transition gastrique-duodénale, la détermination que le premier événement de transition s'est produit et une synchronisation de celui-ci.

6. Appareil selon l'une quelconque des revendications précédentes, ladite capsule ingérable (10) comprenant en outre un accéléromètre (10), et lesdites lectures de la capsule ingérable comprenant des lectures d'accéléromètre, ledit premier sous-ensemble comprenant des lectures d'accéléromètre.

7. Appareil selon la revendication 6, ladite détermination (S104) de la première synchronisation d'événement de transition comprenant en outre :
la détection d'un indicateur de transition gastrique-duodénale dans les lectures d'accéléromètre à partir du premier sous-ensemble ; et
la détermination (S104) que le premier événement de transition s'est produit et une synchronisation de celui-ci sur la base de l'indicateur de transition gastrique-duodénale détecté dans les lectures d'accéléromètre à partir du premier sous-ensemble.

8. Appareil selon la revendication 7, le procédé comprenant en outre :
la détection, en tant que premier indicateur de jonction gastrique-duodénale, d'un indicateur de jonction gastrique-duodénale dans les lectures de capteur de gaz TCD à partir du premier sous-ensemble ; et
la détection, en tant que second indicateur de transition gastrique-duodénale, d'un indicateur de jonction gastrique-duodénale dans les lectures d'accéléromètre à partir du premier sous-ensemble dans une plage temporelle prédéfinie du premier indicateur de transition gastrique-duodénale détecté ; et
sur la base de la détection des premier et second indicateurs de transition gastrique-duodénale, la détermination que le premier événement de transition s'est produit et une synchronisation de celui-ci.

9. Appareil selon l'une quelconque des revendications 6 à 8, ledit second sous-ensemble comprenant les lectures liées à la réflectance de l'antenne, et ladite détermination de la seconde synchronisation d'événement de transition comprenant en outre :
la détection d'un indicateur de transition de jonction iléocécale dans les lectures liées à la réflectance de l'antenne à partir du second sous-ensemble ; et
la détermination que le second événement de transition s'est produit et une synchronisation de celui-ci sur la base de l'indicateur de transition de jonction iléocécale détecté dans les lectures liées à la réflectance de l'antenne à partir du premier sous-ensemble.

10. Appareil selon la revendication 9,
ledit indicateur de transition de jonction iléocécale comprenant une augmentation des lectures de capteur de gaz COV côté capteur avec une augmentation simultanée de la concentration en H2, ladite concentration en H2 étant dérivée des lectures TCD du capteur de gaz TCD et/ou des lectures côté réchauffeur du capteur de gaz COV ; ou
ledit indicateur de transition de jonction iléocécale comprenant une augmentation des lectures de capteur de gaz COV côté capteur avec une augmentation simultanée de la concentration en CH4, ladite concentration en CH4 étant dérivée des lectures TCD du capteur de gaz TCD et/ou des lectures côté réchauffeur du capteur de gaz COV.

11. Appareil selon l'une quelconque des revendications précédentes,
ladite capsule ingérable (10) comprenant un capteur environnemental (14), et lesdites lectures comprenant des lectures dudit capteur environnemental (14), ledit capteur environnemental (14) étant un capteur de température environnementale, un capteur d'humidité relative environnementale, ou un capteur de température environnementale et un capteur d'humidité environnementale ;
ledit procédé comprenant en outre la détermination (S107) d'une synchronisation d'événement d'excrétion par détection d'un indicateur d'excrétion, ledit indicateur d'excrétion étant un changement dans les lectures du capteur environnemental entre une condition environnementale interne du mammifère sujet (40) et une condition environnementale externe au niveau d'un emplacement du mammifère sujet, ladite synchronisation d'événement d'excrétion étant une synchronisation d'excrétion de la capsule ingérable par le mammifère sujet.

12. Appareil selon l'une quelconque des revendications précédentes,
ladite capsule ingérable (10) comprenant un capteur environnemental (14), et lesdites lectures comprenant des lectures dudit capteur environnemental (14), ledit capteur environnemental (14) étant un capteur de température environnementale, un capteur d'humidité relative environnementale, ou un capteur de température environnementale et un capteur d'humidité environnementale ;
ledit procédé comprenant en outre la détermination (S103) d'une synchronisation d'événement d'ingestion en détectant un indicateur d'ingestion, ledit indicateur d'ingestion étant un changement dans les lectures du capteur environnemental entre une condition environnementale interne du mammifère sujet et une condition environnementale externe au niveau d'un emplacement du mammifère sujet, ladite synchronisation d'événement d'ingestion étant une synchronisation d'ingestion de la capsule ingérable par le mammifère sujet.

13. Appareil selon l'une quelconque des revendications précédentes, ladite capsule ingérable (10) comprenant en outre un émetteur-récepteur secondaire (21), ledit émetteur-récepteur secondaire (21) pouvant fonctionner dans une phase d'écoute de la capsule ingérable pendant laquelle l'émetteur-récepteur primaire (19), les capteurs et le processeur embarqué de la capsule ingérable sont mis hors tension, ledit émetteur-récepteur secondaire (21) étant configuré pendant le mode d'écoute pour recevoir un signal de commande d'activation codé provenant d'un dispositif d'émission de signal de commande d'activation codé, et pour répondre en mettant fin à la phase d'écoute et en initiant une phase active de la capsule ingérable pendant laquelle l'émetteur-récepteur primaire (19), les capteurs et le processeur embarqué et la mémoire sont mis sous tension et les lectures sont enregistrées et éventuellement transmises à un appareil récepteur (30) ;
ledit émetteur-récepteur secondaire (21) étant un émetteur-récepteur NFC et ledit signal de commande d'activation codé étant un signal NFC ;
et ledit appareil récepteur (30) étant le dispositif de transmission de signal de commande d'activation codé, et ledit appareil récepteur (30) étant un smartphone ou une tablette informatique exécutant une application amenant le smartphone ou la tablette informatique à générer et à émettre le signal de commande d'activation codé et à recevoir, et éventuellement à traiter et à transmettre, les lectures enregistrées émises hors de la capsule par l'émetteur-récepteur primaire (19).

14. Programme informatique pour déterminer un emplacement d'une capsule ingérable (10) dans un tractus gastro-intestinal, GI, d'un mammifère sujet, ladite capsule ingérable (10) étant ingérable par un mammifère sujet (40) et comprenant un boîtier, une source d'alimentation (16), un capteur de gaz TCD (131) et un capteur de gaz COV (132) ;
ledit programme informatique étant exécutable par un appareil informatique comprenant un processeur et une mémoire, et lors de l'exécution pour amener l'appareil informatique à réaliser un procédé comprenant :
l'accès aux lectures enregistrées de la capsule ingérable (10) en fonction du temps, y compris une période pendant laquelle la capsule ingérable (10) se trouve dans le tractus GI du mammifère sujet (40), les lectures comprenant des lectures de capteur de gaz TCD et des lectures de capteur de gaz COV,
le programme informatique étant **caractérisé par** le procédé comprenant en outre le traitement des lectures enregistrées, y compris :
la détermination (S104) d'une première synchronisation d'événement de transition, la première synchronisation d'événement de transition étant une synchronisation d'une transition gastrique-duodénale par la capsule ingérable (10), ladite détermination de la première synchronisation d'événement de transition comprenant la détection d'un indicateur de transition gastrique-duodénale dans un premier sous-ensemble des lectures enregistrées, le premier sous-ensemble comprenant les lectures de capteur de gaz TCD enregistrées ; et
la détermination (S106) d'une seconde synchronisation d'événement de transition, la seconde synchronisation d'événement de transition étant une synchronisation d'une transition à travers une jonction iléocécale par la capsule ingérable (10), ladite détermination de la seconde synchronisation d'événement de transition comprenant la détection d'un indicateur de jonction iléocécale dans un second sous-ensemble des lectures enregistrées, le second sous-ensemble comprenant les lectures du capteur de gaz COV.
